# EUROPEAN PATENT SPECIFICATION

(11) **EP 0 461 079 B1**
(45) Date of publication and mention of the grant of the patent: **16.07.1997**
(21) Application number: 91810428.2
(22) Date of filing: 05.06.1991
(51) Int. Cl.: C07D 405/04, C07D 239/52, C07D 401/06, C07D 403/04, C07D 413/10, C07D 491/04

(54) **Substituted phthalides and heterocyclic phthalides**
Substituierte Phthalide und heterozyklische Phthalide
Phtalides substitués et phtalides hétérocycliques

(30) Priority: 07.06.1990 US 534794; 21.12.1990 US 633592
(43) Date of publication of application: 11.12.1991
(73) Proprietor: Novartis AG, 4058 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Anderson, Richard James, Palo Alto, CA 94303 (US); Cloudsdale, Ian Stuart, Boulder Creek, CA 95006 (US); Hokama, Takeo, Sunnyvale, CA 94087 (US)

(56) References cited:
- EP-A- 0 410 590
- WO-A-91/10653
- IUPAC, Nomenclature of Organic Chemistry , Sections A,B,C,D,E,F and H Pergamon Press, 1979 Edition, page 20
- Aldrich Catalogue, Seite 148, 1994/95

## Description

The present invention concerns substituted phthalides and heterocyclic phthalides and derivatives thereof, processes for their production, compositions containing them and their use in agriculture.

More particularly, the invention concerns compounds of formula I 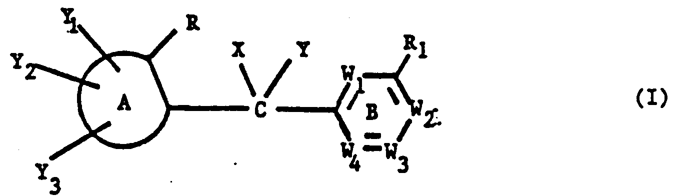 wherein ring system A is selected from
a) phenyl or naphthyl
b) pyridyl which may be fused by its (b) or (c) side to benzene
c) pyridyl-N-oxide or pyrazinyl-N-oxide
d) pyrimidinyl
e) pyrazinyl
f) 3- or 4- cinnolynyl or 2-quinoxalinyl, and
g) a five membered heteroaromatic ring comprising oxygen, sulphur or nitrogen as heteroatom(s) which ring may be fused to a benzene ring or may comprise nitrogen as an additional heteroatom.

- R is: cyano, formyl, CX₁X₂X₃, -C(O)R'' wherein R'' is C₁₋₈alkyl, C₁₋C₈haloalkyl, C₁₋₈alkoxyC₁₋₈alkyl,C₂₋₈alkenyl, C₂₋₈alkynyl, aryl or arylC₁₋₈alkyl; a carboxyl group which may be in the form of the free acid or in ester or salt form, a thiocarboxyl group which may be in the form of the free acid or in ester form, a carbamoyl group, or a group -CONR₇R₈, hydroxyC₁₋₈alkyl, hydroxybenzyl, -CH=NOH, -CH=NO-C₁₋₈alkyl, or a ring C 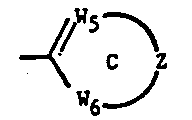
- Y₁, Y₂ and Y₃: are attached to carbon atoms and are independently hydrogen, halogen, hydroxy, C₁₋₈alkyl, C₂₋₈alkenyl, C₂₋₈alkynyl, C₁₋₈alkoxy, C₂₋₈alkenyloxy, C₂₋₈alkynyloxy, C₁₋₈alkylsulfonyloxy, di(C₁₋₈alkyl)sulfamoyloxy, C₁₋₈alkylsulfonyl, C₁₋₈alkysulfinyl, di(C₁₋₈alkyl)carbamoyloxy, C₁₋₈alkylthio, C₂₋₈alkenylthio or C₂₋₈alkynylthio each of which may in turn be substituted by 1 to 6 halogen atoms; di(C₁₋₈alkoxy)methyl, conjugated C₁₋₈alkoxy, hydroxyC₁₋₈alkyl, C₂₋₈acyl, C₂₋₈acyloxy, tri(C₁₋₈alkyl)silyloxy, tri(C₁₋₈alkyl)silyl, cyano, nitro, amino, aryl, arylC₁₋₈alkyl, aryloxy, arylC₁₋₈alkoxy, arylsulfonyl, arylsulfinyl, arylthio or arylC₁₋₈alkylthio, each of which may be substituted by one to three substituents selected from halogen, C₁₋₈alkyl, C₁₋₈haloalkyl, C₁₋₈alkoxy, C₁₋₈haloalkoxy, nitro, cyano, C₁₋₈alkylthio, C₂₋₈acyl, amino a group -C(O)-R' wherein R' is hydrogen, C₁₋₈alkyl, or C₁₋₈alkoxy; or
- Y₁ and R: taken together on adjacent carbon atoms form a bridge having the formula -C(S)-O-, -C(O)-O-E- or -C(O)-N(R₂)-E- wherein E is a direct bond or a 1 to 3 membered linking group with elements selected from methylene, -N(R₂)- and oxygen; or
- Y₁ and Y: ₂ taken together on adjacent carbon atoms form a 3- to 5-membered bridge comprised of elements selected from methylene, -CH=, -C(R₄)=, -NH-, oxygen and -S(O)ₙ- ;
- each of W₁, W₂, W₃, W₄ and W₅: is independently CH, CR₃ or nitrogen;
- W₆ is: NH, oxygen, sulfur, -CR₄=, -CH= or -C(O)- ;
- Z is: a 2- or 3-membered bridge comprised of elements selected from methylene, -CH=, -C(R₄)=, -C(O)-, -NH-, -N=, oxygen and S(O)ₙ- ;
- R₁ and R₃: each is independently hydrogen, halogen, C₁₋₈alkyl, C₂₋₈alkenyl, C₂₋₈alkynyl, C₁₋₈alkoxy, C₂₋₈alkenyloxy, C₂₋₈alkynyloxy, C₁₋₈alkylthio, C₂₋₈alkenylthio or C₂₋₈alkynylthio, each of which may in turn be substituted by 1 to 6 halogen atoms; C₃₋₆cycloalkyl, a 5- or 6-membered heterocycloC₁₋₈alkoxy, aryloxy, arylC₁₋₈alkoxy or arylC₁₋₈alkylthio each of which may be substituted by 1 to 3 substituents selected from halogen, C₁₋₈alkyl, C₁₋₈haloalkyl, C₁₋₈alkoxy, C₁₋₈haloalkoxy, nitro, cyano, C₁₋₈alkylthio, C₂₋₈acyl, amino ; aminoxy, iminoxy, amido, C₁₋₈alkylsulfonylmethyl, cyano, nitro; or -C(O)-Y₄, wherein Y₄ is hydrogen, C₁₋₈alkyl, C₁₋₈alkoxy, hydroxy or phenyl;
- R₂ is: hydrogen, C₁₋₈alkyl, C₁₋₈haloalkyl, C₁₋₈alkoxyalkyl, aryl, or arylC₁₋₈alkyl;
- R₄ is: as defined for Y₁ except for hydrogen;
- X and Y: each is independently hydrogen, hydroxy, halogen, cyano, C₁₋₈alkyl, C₁₋₈alkoxy, C₁₋₈alkoxycarbonyl, C₁₋₈alkoxycarbonyloxy, hydroxyC₁₋₈alkyl, C₁₋₈haloalkyl, C₂₋₈acyl, C₂₋₈acyloxy, carbamoyl, carbamoyloxy, C₁₋₈alkylthio, C₁₋₈alkylsulfinyl, C₁₋₈alkylsulfonyl or C₁₋₈alkylsulfonyloxy; aryl, aryloxy, arylS(O)ₚ, arylsulphonyloxy, each of which may in turn be substituted by 1 to 3 substituents selected from halogen, C₁₋₈alkyl. C₁₋₈haloalkyl, C₁₋₈alkoxy, C₁₋₈haloalkoxy, nitro, cyano, C₁₋₈alkylthio, C₂₋₈acyl; amino or together represent =O, =S, =NH, =NOR₁₂ or =CR₁₃R₁₄; or
- X and R: together may form a bridge having the formula -C(O)-O-, -C(O)-S or -C(O)-NR₂- wherein the carbonyl is attached to A;
- p is: 0, 1 or 2;
- X₁, X₂ and X₃: are independently hydrogen, hydroxy, C₁₋₈alkoxy, C₁₋₈alkylthio, hydroxyC₁₋₈alkyl or hydroxybenzyl whereby at least one of X₁, X₂ and X₃ is other than hydrogen; or
- X₃ is: hydrogen and X₁ and X₂ together form a 4- or 5-membered bridge comprising elements selected from -O(CH₂)ₙ,-O-, -OC(O)(CH₂)ₘO- and -S(CH₂)ₙ,S-;
- R₇ and R₈: are each independently (a) hydrogen, halogen; (b) C₁₋₂₄ alkyl, C₂₋₈alkenyl, C₂₋₈alkynyl, C₁₋₈alkoxy, C₁₋₈alkoxyC₁₋₈alkoxy, C₂₋₈-alkenyloxy, C₂₋₈alkynyloxy, C₁₋₈alkylthio, C₂₋₈alkenylthio or C₂₋₈alkynylthio, each of which may in turn be substituted by 1 to 6 halogen atoms; (c) C₃₋₆cycloalkyl, C₃₋₆cycloalkylC₁₋₈alkyl, heterocyclyl, heterocycloC₁₋₈alkyl, heterocycloC₁₋₈alkoxy, aryloxy, arylC₁₋₈alkoxy. or arylC₁₋₈alkylthio, each of which is unsubstituted or may be substituted by 1 to 3 substituents selected from (i) halogen; (ii) C₁₋₈alkyl, C₁₋₈alkoxy, C₁₋₈haloalkoxy, C₁₋₈haloalkyl, C₁₋₈alkylthio, C₁₋₈alkylsulfonyl, C₁₋₈alkylsulfonylmethyl; and (iii) nitro, cyano, acyl, amino; (d) amino, amido, aminosulfonyl, cyano, nitro, or -(CHR₄')ₙ'''-C(O)Y₄', wherein Y₄' is hydrogen, C₁₋₈alkyl, C₁₋₈ alkoxy or hydroxy and n''' is 0, 1, 2 or 3; R₄' is as defined for Y₁;
- R₁₂ is: hydrogen or C₁₋₈ alkyl;
- R₁₃ and R₁₄: are independently hydrogen, C₁₋₈ alkyl or halogen;
- m is: 1 or 2;
- n is: 0, 1 or 2; and
- n' is: 2 or 3;
with the proviso that when R is carboxyl in free ester or salt form and X and Y together are =O one of rings A and B contains a hetero atom.

Herbicidal benzylpyrimidine and benzyltriazine derivatives have been described in the European patent application EP-A-0410590, published on January 30, 1991.

When R is a carboxyl or thiocarboxyl group in ester form it is preferably of formula -COOR₅ or -COSR₅ wherein R₅ is alkyl, haloalkyl, alkoxyalkyl, alkenyl, haloalkenyl, alkynl, haloalkynyl, unsubstituted or substituted aryl, unsubstituted or substituted aralkyl, hydroxyalkyl, cycloalkyl, cyanoalkyl, aralkoxyalkyl; a group -N=C(R₁₅)(R₁₆); a group -(CH₂)_{n''}CH(R₁₇)(R₁₈);
a group 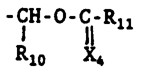
R₁₅ and R₁₆ are independently hydrogen or alkyl,
R₁₇ and R₁₈ are independently S(O)ₙalkyl, COOR₉, alkoxy, amino, substituted amino, benzyloxy, trimethylsilyl, cyano, -C(R₁₉)SR₂₀ or additionally one thereof may be hydrogen.
R₁₉ is hydrogen or alkyl,
R₂₀ is alkyl or aryl,
R₉, R₁₀ and R₁₁ are independently hydrogen, alkyl, haloalkyl, alkoxyalkyl, unsubstituted or substituted aryl or unsubstituted or substituted aralkyl,
n and n'' are independently zero, one or two, and
X₄ is oxygen or sulfur.

When R is carboxyl in salt form the salt is preferably formed with an alkali metal, alkali earth metal, optionally substituted ammonium cation, a trialkyl sulfonium cation, a trialkylsulfoxonium cation or a phosphonium cation, especially the cation of an alkali metal (e.g. the Li or Na cation) or of an earth alkali metal (e.g. the Ca or Mg cation); the ammonium cation; a substituted ammonium cation [such as a C₁₋₅alkylammonium cation, a di-C₁₋₅alkylammonium cation, a tri-C₁₋₅alkylammonium cation, a tetra-C₁₋₅ammonium cation, a (C₁₋₅alkoxy-alkyl)ammonium cation, a (hydroxy-C₁₋₅alkyl)ammonium cation]; a phosphonium cation; a tri(C₁₋₈akyl)sulfonium cation; or a tri(C₁₋₈alkyl)sulfoxonium cation.

When Y₁, Y₂ and/or Y₃ is a carboxyl group this may be in ester or salt form or in amide form (i.e. a carbamoyl) and as such is as described above for R in these forms. Where A has meaning g) it contains one to three heteroatoms and signifies for example thienyl, furanyl, pyrrolyl, oxazolyl, thiazolyl, isoxazolyl, isothiazolyl, imidazolyl, pyrazolyl, oxadiazolyl or thiadiozalyl.

Where A has one of the above defined heteroaromatic significances, b) through g), the substituted hetero ring is particularly selected from pyridyl, quinolyl, pyridyl-N-oxide, pyrimidinyl, pyrazinyl, thienyl or furyl, more particularly from pyridyl or thienyl.

Alkyl moieties unless otherwise specified contain 1 to 8 carbon atoms, preferably 1 to 5, especially 1 to 4, e.g. 1 or 2 carbon atoms. Lower alkyl moieties contain 1 to 4, e.g. 1 or 2 carbon atoms. Alkyl moieties as present in *R*₅, R₇ or R₈ contain preferably 1 to 12, especially 1 to 6 whereby one of R₇ and R₈ is preferably hydrogen when the other is alkyl.

Alkyl moieties as bridging groups may be straight chain or branched and preferably contain 1 to 4, e.g. 1 or 2 carbon atoms. They may be optionally substituted by aryl or substituted aryl and may optionally be interrupted by or attached via an oxygen or sulfur atom.

"Conjugated alkoxy" stands for an alkoxy group interrupted in its alkyl moiety by one or more oxygen atoms eg alkoxyalkoxy, alkoxyalkoxyalkoxy, etc.

Alkenyl and alkynyl moieties contain 2 to 8, preferably 2 to 4, especially 2 or 3 carbon atoms.

Halogen is preferably F, Cl or Br, especially for Cl.

Aryl moieties are preferably as defined for meanings a) of ring system A, especially phenyl.

Substituted amino, -amido, -aminoxy, -aminoalkyl, - iminoxy, -carbamyl (other than as R) is preferably substituted by one or two substituents selected from alkyl, alkoxy, haloalkyl, acyl, alkoxyalkyl, unsubstituted or substituted aryl or unsubstituted or substituted aralkyl.

Acyl as or as part of a substituent is conveniently 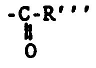 wherein R''' is as defined for Y₁ (for example alkyl, haloalkyl, cycloalkyl, alkoxyalkyl, unsubstituted or substituted aryl (especially phenyl). Examples of acyl include acetyl, propionyl, butyryl, unsubstituted or substituted benzoyl, pivaloyl or chloracetyl, especially acetyl or unsubstituted or substituted benzoyl.

Cycloalkyl is preferably of 3 to 6 carbon atoms especially cyclopropyl, cyclopentyl or cyclohexyl. heterocyclo is preferably 5 or 6 membered and as defined for A definitions b) to g) and preferences or saturated and containing O, S or N as heteroatom, eg tetrahydrofuryl, piperidinyl, morpholinyl.

For convenience bridging members such as 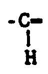 are so written but are to be understood as embracing

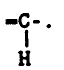

Carbamoyl or substituted carbamoyl moieties are attached to the molecule which they substitute via their carbonyl. Amido or substituted amido moieties are attached to the molecule which they substitute via their nitrogen atom.

A particular group of compounds of formula I (compounds Ia) comprises those wherein ring system A is selected from phenyl, pyridyl or pyridyl-N-oxide.

R is a carboxyl group which may be in the form of the free acid or in ester or salt form, a thiocarboxyl group which may be in the form of the free acid or in ester form, a carbamoyl group or a mono- or di- substituted carbamoyl group.
Y₁, Y₂ and Y₃ are attached to carbon atoms and are independently hydrogen, halogen, C₁₋₈alkyl, C₁₋₈alkoxy;
each of W₁, W₂, W₃ and W₄ is independently CH, CR₃ or nitrogen;
R₁ and R₃ each is independently hydrogen, halogen, C₁₋₈alkyl, C₁₋₈alkoxy, aryloxy or arylC₁₋₈alkoxy.
X and Y each is independently hydrogen, hydroxy, cyano. C₁₋₈alkoxy, C₂₋₈acyloxy or together represent =O; or
X and R together form a bridge having the formula 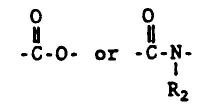 wherein the carbonyl is attached to A.

When R is carboxyl or thiocarboxyl in ester form it is preferably of formula -COOR₅ or COSR₅;
wherein each R₅ is independently alkyl, alkoxyalkyl, alkenyl, alkynyl, substituted aryl or unsubstituted or substituted aralkyl.

When R is carboxy or thiocarboxyl in salt form the salt is preferably formed with an alkali metal, alkali earth metal, optionally substituted ammonium cation especially the cation of an alkali metal (e.g. the Li or Na cation) or of an earth alkali metal (e.g. the Ca or Mg cation); the ammonium cation; a substituted ammonium cation [such as a C₁₋₅alkylammonium cation, a di-C₁₋₅alkylammonium cation, a tri-C₁₋₅alkylammonium cation, a tetra-C₁₋₅ammonium cation.

When R is carbamoyl or mono- or di- substituted carbamoyl it is preferably of formula CONR₇R₈ wherein R₇ is hydrogen, alkyl, haloalkyl, alkoxyalkyl, unsubstituted oil substituted aryl or unsubstituted or substituted aralkyl and R₈ is hydrogen, alkyl, NH₂, NHR₆ or OR₆ wherein R₆ is as defined for R₇.

A particular compound group (compounds Ib) comprises those compounds of formula I wherein ring system A represents phenyl, pyridyl or thienyl; B represents pyrimidinyl or triazinyl; R represents a ring C especially oxazole, oxazolone, oxazolidine or oxazolidinone; carboxyl in the form of the free acid or in ester or salt form; -CONR₇R₈, cyano or together with X represent 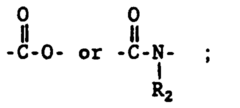 Y₁, Y₂ and Y₃ each represent independently hydrogen, halogen, C₁₋₈ alkyl, C₁₋₈alkoxy, C₁₋₈alkylthio or arylthio.

X, Y each represent independently hydrogen, hydroxy, C₁₋₈alkoxy, C₁₋₈acyloxy, a ring B, halogen, C₁₋₈alkylthio or arylthio or together =O or =NH
and R₁ and R₃ each represent independently halogen, C₁₋₈alkoxy, C₁₋₈alkyl, C₁₋₈haloalkoxy, aryloxy, arylC₁₋₈alkoxy, C₂₋₈alkylnyloxy, C₂₋₈alkenyloxy.

A further compound group comprises compounds Ib wherein Y₁, Y₂ and Y₃ additionally may each represent independently aryl-C₁₋₈ alkoxy, C₂₋₈alkenyloxy or C₂₋₈alkynyloxy.

B is especially pyrimidinyl, particularly 4,6-dimethoxy-2-pyrimidinyl.

A is especially phenyl or pyridyl substituted as defined above.

X and Y are preferably hydrogen, halogen, cyano, hydroxy, alkoxy or together =O, especially hydrogen, hydroxy or together =O.

A further group of compounds according to the invention (Compounds Ic) comprises those of formula I wherein ring system A is pyridyl,
R is CONR₇'R₈'
wherein R₇' and R₈' represent independently hydrogen, alkoxy, alkyl; or aryl or aralkyl each of which may be unsubstituted or substituted,
X is hydrogen,
Y is OR₃', SR₃' on OCOR₃'
wherein R₃' is alkyl; or aryl; or aralkyl each of which may be unsubstituted or substituted,
or X and Y together represent =O or =S and ring system B is m-CF₃ phenyl.

Within this group Ic, compounds are preferred wherein X is OH and Y is H or X and Y together represent =O, A is 2- or 3-pyridyl, R₇ is hydrogen or alkyl especially methyl, R₈ is phenyl or benzoyl which may be unsubstituted or substituted eg 1-3 times by halogen, alkyl and/or alkoxy. The following meanings are preferred independently for each substituent.
- A: a) meanings a) and b)
b) phenyl
c) pyridyl
- R: a) carboxyl in the form of the free acid or in salt or ester form or carbamoyl or mono- or di-substituted carbamoyl
b) COOR₅ wherein R₅ is hydrogen alkyl, COO⁺Ma⁻ wherein Ma is an alkali metal cation or CONR₇R₈ wherein R₇ is hydrogen or alkyl and R₈ is alkyl, aryl or substituted aryl
c) COO⁻Na⁺, COOCH₃, CONHC₆H₁₃, CONH(CH₃) phenyl
- Y₁: a) hydrogen, halogen, alkyl or alkoxy
b) halogen, especially fluorine or chlorine
- Y₂, Y₃: a) hydrogen or halogen, alkyl or alkoxy
b) hydrogen or halogen
c) hydrogen
- W₁: N
- W₂: a) CH or N
b) CH
- W₃: CR₃
- W₄: N
- W₅: a) CH or N
b) N
- W₆: a) O
b) NH
- Z: a) elements selected from methylene, substituted methylene, 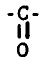 b) 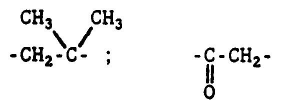
- X₁, X₂: a) alkoxy, especially methoxy
b) hydroxy
- X₃: a) hydrogen
b) alkoxy especially methoxy
- R₁, R₃: a) alkoxy, especially methoxy
- R₄: a) halogen, especially chlorine
b) alkyl, especially methyl
- R₂: a) alkyl, especially methyl
b) hydrogen
- R₅: a) alkyl, alkenyl or alkynyl
b) C₁₋₄alkyl, especially methyl or ethyl
c) C₂₋₄alkenyl
d) C₂₋₄alkynyl, especially propargyl
- R₆, R₇: a) alkyl
b) methyl, ethyl
- R₈: a) hydrogen
b) alkyl, especially methyl or ethyl
c) an aryl, especially a phenyl
- R₉, R₁₀,: a) hydrogen or alkyl
- R₁₂, R₁₅, R₁₉: b) hydrogen or methyl
- R₁₁: a) alkyl
b) propyl (n- or iso-)
- Y₄: a) alkyl or alkoxy
b) CH₃ or CH₃O
- R₁₃, R₁₄: a) hydrogen or halogen
b) hydrogen or fluorine
- R₁₆: a) alkyl
b) C₁₋₄alkyl, especially methyl or ethyl
- R₁₇: a) S(O)ₙalkyl or COOR₉
b) SO₂CH₃ or COOCH₃
- R₁₈: a) hydrogen
- R₂₀: a) alkyl or phenyl
b) methyl or phenyl
- n: a) 2
b) 0
- n': a) 2
b) 3
- n'': a) 1
b) 0
- m: a) 1
b) 2
- X: a) hydroxyl
b) hydrogen
c) taken with Y, =O
d) acyloxy
e) alkoxycarbonyloxy
f) carbamoyloxy
g) sulphonyloxy
- Y: a) taken with X, =O
b) hydrogen
- X + R: 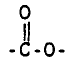
- R': a) alkyl
b) alkoxy
- R'': a) alkyl
b) methyl
- R''': a) alkyl
b) aryl, especially phenyl
- Ring A, Ring B: a) at least one contains a heteroatom
b) ring A = a phenyl or a pyridine
ring B = a pyrimidine especially 3,5 dimethoxy pyrimidine

Combinations of the above listed preferred meanings are especially preferred. One such combination comprises compounds of formula (I) in which
A is phenyl or pyridyl;
R is a carboxyl group in the form of a free acid or salt; carbamoyl; COOR₅'' wherein R₅'' is C₁₋₅alkyl or C₂₋₅alkenyl or CONR₇''R₈'' wherein
R₇'' is C₁₋₁₂alkyl, amino, C₁₋₄alkylamino, anilino, haloanilino, benzyl, halobenzyl, C₁₋₄alkylbenzyl, C₁₋₄alkoxybenzyl, phenyl, halophenyl, C₁₋₄alkylphenyl or C₁₋₄alkoxyphenyl;
R₈'' is hydrogen or C₁₋₄alkyl;
Y₁, Y₂ and Y₃ are independently hydrogen or halogen;
W₁ and W₄ are N;
W₂ is CH;
W₃ is CR₃ wherein R₃ is C₁₋₅alkoxy:
R₁ is C₁₋₅alkoxy;
X is hydroxyl or C₁₋₄alkoxycarbonyloxy or taken with Y is =O;
Y is hydrogen or taken with Y is =O; or
X and R together form a bridge having the formula -C(O)O- wherein the carbonyl is attached to A, and Y is hydrogen or C₂₋₈acyloxy.

Examples of preferred compounds according to the invention are compound nos. 13, 40, 53, 55, 58, 64, 77, 78, 82, 91, 111, 124, 125, 130, 143, 149, 163, 170, 175, 199, 204, 205, 247, 249, 258, 262, 263, 265, 266, 267 and 277.

Compounds having the formula 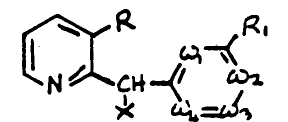 especially those wherein X is CN may exist in the alternate tautomeric form

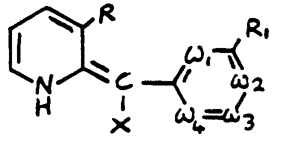

The compounds of formula I according to the invention may be prepared as follows.
a) when X and R combine to form a bridging group as defined above and Y is hydrogen, cyano, arylthio, arylsulfinyl or arylsulfonyl, reacting a compound of formula II 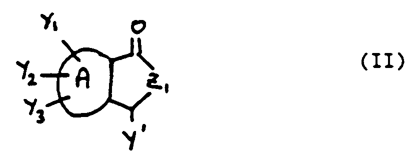 wherein A is as defined above, Y' represents hydrogen, cyano, arylthio, arylsulfinyl or arylfulfonyl and Z₁ represents oxygen, sulfur or NR₂ wherein R₂ is as defined above except for hydrogen.
   with a compound of formula III 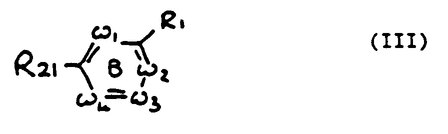 wherein W₁, W₂, W₃, W₄ are as defined above and R₂₁ represents methylsulfonyl, or halogen to obtain the corresponding compound of formula Ip 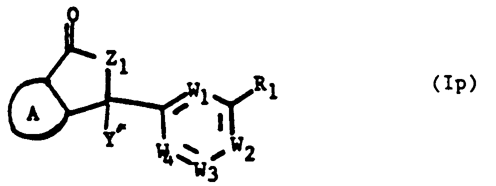
b) treating a compound of formula Ip wherein Y' represents cyano or arylsulfonyl and Z₁ represents oxygen and the other symbols are as defined above.
   (i) by hydrolysis to give a corresponding compound of formula I wherein R and X form a bridge and Y is hydroxy or a compound of formula I wherein X and Y together form =O
   (ii) with an amine to give a corresponding compound of formula I wherein R is an optionally substituted carbamoyl group and X and Y together form =O
   (iii) with a group

      MOR₂₂

      wherein M is an alkali metal and R₂₂ is hydrogen or alkyl, to give a corresponding compound wherein R and X form a bridge and Y is hydroxy or alkoxy
c) hydrolyzing a compound of formula Ip wherein Y' represents hydrogen and Z₁ represents oxygen to give a compound of formula I wherein R is a carboxyl group optionally in salt form, X is hydrogen and Y is hydroxy
d) ring opening a compound of formula Ip wherein Y' represents hydroxy and Z₁ represents oxygen to give a compound of formula I wherein R is a carboxyl group optionally in salt form and X and Y together are =O
e) esterifying a compound of formula I wherein R is a carboxyl group optionally in salt form and X and Y are =O to give the corresponding compound wherein R is a carboxyl group in ester form
f) halogenating a compound of formula Ip wherein Y' represent hydroxy to give a compound of formula I wherein X and R together form a bridging group and Y' is halogen
g) reacting a compound of formula Ip wherein Z₁ is oxygen and Y' is halogen with a group R₂NH₂ and a group HOR₂₃ wherein R₂₃ represents alkyl, acyl or aryl and R₂ is as defined above to give the corresponding compound wherein Z₁ is NR₂ and Y' is alkoxy, aryloxy or acyloxy
h) oxidizing a compound of formula Ip wherein Y' represents hydrogen to give the corresponding compound wherein Y' represents hydroxy
i) reacting a compound of formula IV 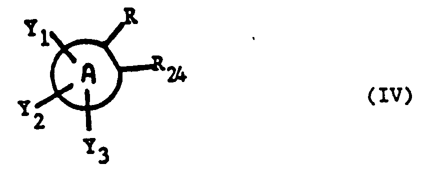 with a compound of formula V 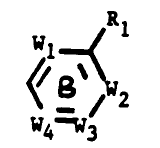 to produce a compound of formula Iq 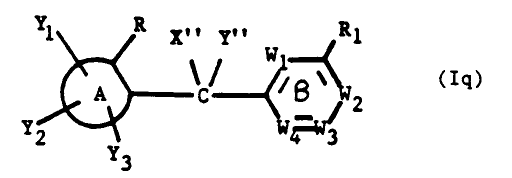 wherein A, R, R₁, W₁, W₂, W₃, W₄, Y₁, Y₂ and Y₃ are as defined above and X'' and Y'' are hydrogen and R₂₄ is alkyl, especially methyl
j) mono-or di-halogenating a compound of formula Iq wherein X'' and Y'' are hydrogen to produce the corresponding compound of formula Iq wherein one or both of X'' and Y'' are halogen
k) oxidizing a compound of formula Iq wherein X'' and Y'' are both hydrogen or X'' is halogen and Y'' is hydrogen to produce the corresponding compound wherein X'' and Y'' together represent =O or one represents hydrogen and the other represents hydroxy
l) alkylating a compound of formula Iq wherein X'' represents hydrogen and Y'' represents hydrogen to produce the corresponding compound wherein X'' represents alkyl and Y'' represents hydrogen
m) introducing an alkoxy or alkylthio group into a compound of formula Iq wherein X'' represents halogen and Y'' represents hydrogen to produce the corresponding compound wherein X'' represents alkoxy or alkylthio and Y'' represents hydrogen
n) acylating a compound of formula Iq wherein X'' represents hydroxy and Y represents hydrogen to produce the corresponding compound wherein X'' represents acyloxy and Y'' represents hydrogen
o) reacting a compound of formula Ip wherein Z₁ is oxygen and Y' is hydrogen with a group R₇NH₂ wherein R₇ is as defined above to give a compound of formula I wherein R is monosubstituted carbamoyl, X is hydrogen and Y is hydroxy
p) sulfonylating, carbamoylating, acylating or carbalkoxylating a compound of formula Ip wherein Z₁ is oxygen and Y' is hydroxy to produce the corresponding compound of formula I wherein R and X form a 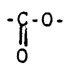 bridge and Y represents sulfonyloxy, carbamoyloxy, acyloxy or alkoxycarbonoyloxy
q) reacting a compound of formula Ip wherein Z₁ is oxygen and Y' is halogen with a group R₇R₈NH wherein R₇ and R₈ are as defined above (R₇ and R₈ ≠ H) to give a compound of formula I wherein R is disubstituted carbamoyl, and X and Y together represent =O.
   and recovering any compound wherein R is a carboxyl or thiocarboxyl group in free form or in ester form and any compound wherein R is carboxyl in free form or in salt form.

The following table is illustrative of suitable reaction conditions.

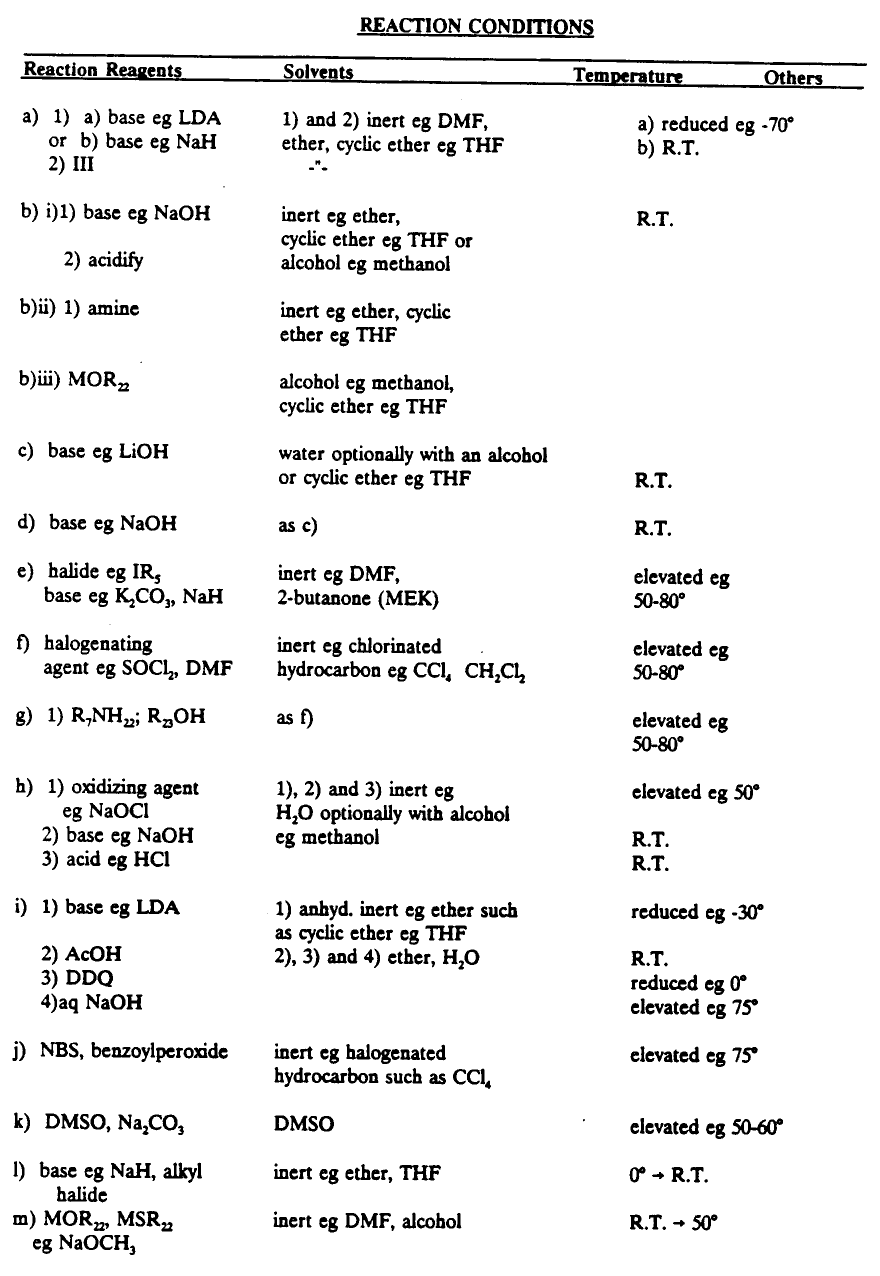

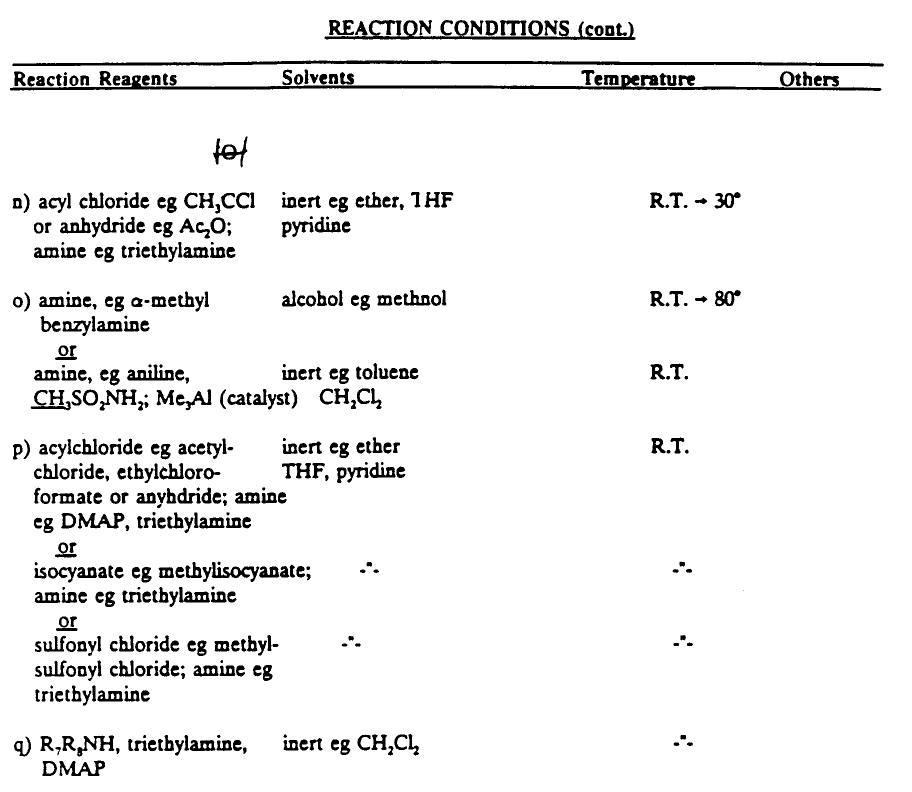

Process a) through p) also form part of the invention.

The starting materials of formula II or III are either known or may be prepared analogously to known methods.

The compounds of formula I have herbicidal activity as observed after their pre-emergent or post-emergent application to weeds or a weed locus.

The term "herbicide" (or "herbicidal") refers to an active ingredient (or an effect) which modifies the growth of plants because of plant growth regulating or phytotoxic properties so as to retard the growth of the plant or damage the plant sufficiently to kill it.

Application of a compound of formula I is made according to conventional procedure to the weeds or their locus using a herbicidally effective amount of the compound, usually from 10 g to 10 kg/ha.

Compounds according to the invention may be used in the control of both broad-leaf and grassy weeds on both pre- and post-emergent application. Compounds may also exhibit selectivity in various crops and are thus suited for use in weed control in crops such as corn, cotton, wheat and soybean.

The optimum usage of a compound of formula I is readily determined by one of ordinary skill in the art using routine testing such as greenhouse testing and small plot testing. It will depend on the compound employed, the desired effect (a phytotoxic effect requiring a higher rate than a plant growth regulating effect), the conditions of treatment and the like. In general satisfactory phytotoxic effects are obtained when the compound of formula I is applied at a rate in the range of from 0.01 to 5.0 kg, more preferably of from 0.05 to 2.5 kg per hectare, eg 0.05 to 5.0 kg per hectare, especially 0.1 to 2.5 kg kper hectare.

The compounds of formula I may be advantageously combined with other herbicides for broadspectrum weed control. Examples of herbicides which can be combined with a compound of the present invention include those selected from the carbamates, thiocarbamates, chloroacetamides, dinitroanilines, benzoic acids, glycerol ethers, pyridazinones, semicarbazones, uracils and ureas for controlling a broad spectrum of weeds.

The compounds of formula I are conveniently employed as herbicidal compositions in association with agriculturally acceptable diluents. Such compositions also form part of the present invention. They may contain, aside from a compound of formula I as active agent, other active agents, such as herbicides or compounds having antidotal, fungicidal, insecticidal or insect attractant activity. They may be employed in either solid or liquid forms eg in the form of a wettable powder or an emulsifiable concentrate incorporating conventional diluents. Such compositions may be produced in conventional manner, eg by mixing the active ingredient with a diluent and optionally other formulating ingredients such as surfactants.

Agriculturally acceptable additives may be employed in herbicidal compositions to improve the performance of the active ingredient and to reduce foaming, caking and corrosion, for example.

The term "diluent" as used herein means any liquid or solid agriculturally acceptable material which may be added to the active constituent to bring it in an easier or improved applicable form, respectively, to a usable or desirable strength of activity. It can for example be talc, kaolin, diatomaceous earth, xylene or water.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

Particularly formulations to be applied in spraying forms such as water dispersible concentrates or wettable powders may contain surfactants such as wetting and dispersing agents, for example the condensation product of formaldehyde with naphthylene sulphonate, an ethoxylated alkylphenol and an ethoxylated fatty alcohol.

In general, the formulations include from 0.01 to 90% by weight of active agent and from 0 to 20% by weight of agriculturally acceptable surfactant, the active agent consisting either of at least one compound of formula I or mixtures thereof with other active agents. Concentrate forms of compositions generally contain between about 2 and 90%, preferably between about 5 and 70% by weight of active agent. Application forms of formulation may for example contain from 0.01 to 20% by weight of active agent.

Typical herbicidal compositions, according to this invention, are illustrated by the following Examples A, B and C in which the quantities are in parts by weight.

### EXAMPLE A

### Preparation of a Dust

10 Parts of a compound according to this invention and 90 parts of powdered talc are mixed in a mechanical grinder-blender and are ground until a homogeneous, free-flowing dust of the desired particle size is obtained. This dust is suitable for direct application to the site of the weed infestation.

### EXAMPLE B

### Preparation of a Wettable Powder

25 Parts of a compound according to this invention are mixed and milled with 25 parts of synthetic fine silica, 2 parts of sodium lauryl sulphate, 3 parts of sodium ligninsulphonate and 45 parts of finely divided kaolin until the mean particle size is about 5 micron. The resulting wettable powder is diluted with water before use to a spray liquor with the desired concentration.

### EXAMPLE C

### Preparation of Emulsifiable Concentrate (EC)

13.37 Parts of a compound according to this invention are mixed in a beaker with 1.43 parts of Toximul 360A (a mixture of anionic and nonionic surfactants containing largely anionic surfactants), 5.61 parts of Toximul 360A (a mixture of anionic and non-ionic surfactants containing largely non-ionic surfactants), 23.79 parts of dimethylformamide and 55.8 parts of Tenneco 500-100 (predominantly a mixture of alkylated aromatics such as xylene and ethylbenzene) until solution is effected. The resulting EC is diluted with water for use.

The following examples are provided to illustrate the practice of the present invention. Temperature is given in degrees Celsius.
Abbreviations used in this specification.
THF = tetrahydrofuran
LDA = lithiumdiisopropylamide
RT = room temperature
DMF = dimethylformamide
DDQ = 2,3-dichloro-5,6-dicyanobenzoquinone
NBS = N-bromosuccinimide
DMSO = Dimethylsulfoxide
MEK = Methylethylketone
DMAP = Dimethylaminopyridine

Individual alkyl substituents listed in the following tables from A to F are in the "n" isomeric form unless otherwise indicated.

### EXAMPLE 1

### 7-chloro-3-(4,6-dimethoxy-2-pyrimidinyl)phthalide (Table A, cpd. no. 6)

1.68 g (0.01 mol) of 7-chlorophthalide is added to 100 ml of dry THF and the mixture cooled to -70°C. 6.8 ml (0.01 mol) of 1.5 M LDA is then added over 3 minutes and the reaction mixture stirred at -70°C for 15 minutes. 2.18 g (0.01 mol) of 2-methylsulfonyl-4,6-dimethoxypyrimidine in 50 ml of THF is then added and the mixture stirred for 4 hrs with temperature being maintained at -75 to -70°C. The reaction mixture is neutralized with 1.5 g of NH₄Cl in 5 ml of water, warmed and concentrated on a rotovaporator. The concentrate is partitioned between CH₂Cl₂/H₂O (50 ml each) and the aqueous phase separated and treated with further 30 ml of CH₂Cl₂. The combined CH₂Cl₂ phases are washed with 30 ml of water, separated and concentrated. The concentrate was flash chromatographed on silica gel using 80/20 hexane/ethyl acetate (500 ml), 50/50 hexane/ethyl acetate (500 ml) and 80/20 acetone/methanol (500 ml) (30 fractions X 50 ml). The title compound (fractions 9-23) was obtained after recrystalization from hexane/CH₂Cl₂ as a white solid, m.p. 148-149°C.

### EXAMPLE 2

### 5-(4,6-dimethoxy-2-pyrimidinyl)-furo[3,4,b] pyridine-7(5H)-one (Table B, cpd. no. 40)

A solution of 1.3 g (0.0096 mols) of furo [3,4-b]pyridine-7(5H)-one in 50 ml of dry THF is cooled to -75° C and 8 ml (0.0192 mols) of 2.5 M LDA added dropwise over 5 minutes. The mixture is allowed to react for 1 hr at -75° C and 2.1 g (0.0096 mol) of 2-methylsulfonyl-4,6-dimethoxypyrimidine in 30 ml of dry THF added dropwise over 10 minutes. The mixture is allowed to warm to RT, 1.6 ml of HCl added and the THF evaporated off. The residue is dissolved in 75 ml of CH₂Cl₂, washed with water (2 x 50 ml) and the organic phase concentrated to give a yellowish white gummy solid. This is chromatographed on a silica gel column using 50/50 hexane/ethylacetate (500 ml), ethyl acetate (500 ml) and 80/20 acetone/methanol (1000 ml) (30 fractions). The crystalline residue (fractions 18-21) of the title product has m.p. of 167-168°C.

### EXAMPLE 3

### 7-chloro-3-methoxy-3-(4,6-dimethoxy-2-pyrimidinyl)-2-methylisoindol-1(3H)-one (Table C, cpd. no. 54)

A mixture of 0.5 g of 7-chloro-3-hydroxy-3-(4,6-dimethoxy-2-pyrimidinyl)phthalide, 30 ml of CCl₄, 2 ml of SOCl₂ and 4 drops of DMF is heated at 65°C for 1½ hrs, cooled and excess SOCl₂ and CCl₄ removed on a rotovaporator. The residue is diluted with 20 ml of CH₂Cl₂ and added to a mixture of 5 ml of 40% aq methylamine and 10 ml of methanol with stirring over ½ hr. The mixture is placed on a rotovaporator and the residue partitioned between 50 ml each of CH₂Cl₂ and water. The organic phase is concentrated and flash chromatographed on silica gel using 50/50 hexane/ethyl acetate (800 ml), ethyl acetate (500 ml) and 80/20 acetone/methanol (200 ml) (30 fractions X 50 ml). The product (fractions 19-21) was obtained as a yellow gum.

### EXAMPLE 4

### 7-chloro-3-hydroxy-3-(4,6-dimethoxy-2-pyrimidinyl)phthalide (Table A, cpd. no. 13)

A mixture of 1.8 g of 7-chloro-3-cyano-3-(4,6-dimethoxy-2-pyrimidinyl)phthalide, 50 ml of 1% NaOH and 50 ml of THF are stirred at room temperature for 3 hrs. The THF is removed by evaporation and the mixture is diluted with water and extracted twice with ethyl acetate. The aqueous solution is acidified with 2N-H₂SO₄. The resulting acid solution is extracted with 3 x 100 ml ethyl acetate and the organic phases combined, dried over Na₂SO₄ and concentrated to give a pale yellow solid. This residue is taken up in ethyl acetate and treated with activated charcoal until the yellow base line material is removed to give the title product as a white solid m.p. 188-190°C.

### EXAMPLE 5

### 7-chloro-3-methoxy-3-(4,6-dimethoxy-2-pyrimidinyl)phthalide (Table A, cpd. no. 30)

1.0 g of 7-chloro-3-cyano-3-(4,6-dimethoxy-2-pyrimidinyl)phthalide is slurried in 20 ml of methanol and the solution cooled with ice and 0.6 ml of sodium methoxide added dropwise. After stirring for 10 min a further 1 ml of sodium methoxide is added and stirring continued for 10 min and the mixture is then quenched with 2N H₂SO₄. Methanol is removed on a rotovaporator and the residue partitioned between water and ethyl acetate. The organic phase is dried over Na₂SO₄ and concentrated. Flash chromatography of the residue over silica gel using 25% ethyl acetate/hexane yields a white solid m.p. 180-183°C.

### EXAMPLE 6

### a) Methyl 2-chloro-6-(4,6-dimethoxy-2-pyrimidinylcarbonyl)benzoate (Table C, cpd. no. 55), and

### b) 7-chloro-3-chloro-(4,6-dimethoxy-2-pyrimidinyl)phthalide (Table A, cpd. no. 21)

A mixture of 0.7 g of 7-chloro-3-hydroxy-3-(4,6-dimethoxy-2-pyrimidinyl)phthalide, 30 ml of CCl₄, 2 ml of SOCl₂ and 4 drops of DMF are refluxed at 60° for 1½ hrs. The mixture is then cooled, excess SOCl₂ and CCl₄ removed on a rotovaporator. The residue is diluted with 20 ml of CH₂Cl₂ and added to a stirred mixture of 10 ml of methanol and 2 ml of diethylamine. After 2½ hrs the mixture is stripped on a rotovaporator to remove excess CH₂Cl₂ and methanol and the residue partitioned between CH₂Cl₂ (50 ml) and water (50 ml). The organic phase is separated, concentrated and the gummy residue flash chromatographed over silica gel using 80/20 hexane/ethyl acetate (500 ml), 60/40 hexane/ethyl acetate (500 ml) (28 fractions X 50 ml). Fractions 18 to 20 yielded title compound a) and fractions 11 to 16 the compound b).

### EXAMPLE 7

### 7-chloro-3-cyano-3-(4,6-dimethoxy-2-pyrimidinyl)phthalide (Table A, cpd. no. 27)

600 mg of 7-chloro-3-cyanophthalide are added to an ice-cold suspension of hexane washed 60% NaH (160 mg) in DMF (20 ml). After 15 min, 710 mg of 2-methylsulfonyl-4,6-dimethoxypyrimidine are added. After stirring at RT for 1½ hr the mixture is poured onto 200 ml of ice/water acidified with 2N H₂SO₄ and stirred. The precipitate is filtered and dried in a vacuum oven to yield the title product, m.p. 159-161°C.

### EXAMPLE 8

### 7-chloro-3,3-bis(4,6-dimethoxy-1,3,5-triazin-2-yl)phthalide (Table A, cpd. no. 36)

1.48 g of 7-chlorophthalide are dissolved in 80 ml of THF. The solution is cooled to -70°C and 1.5 M LDA in THF (6 ml) is syringed in at -70°C over 3 min. Stirring is continued for 15 min at -70°, 1.54 g of 2-chloro-4,6-dimethoxy-1,3,5-triazine in 50 ml of THF added dropwise and the mixture is then allowed to warm to -20°. The mixture is again cooled to -70° and 1 ml of conc. HCl in 10 ml of water is added. The mixture is stirred for 25 min and allowed to warm to RT and the THF is removed by evaporation. The residue is partitioned between CH₂Cl₂ and water (50 ml each) and the aqueous phase extracted with an additional 30 ml of CH₂Cl₂. The combined organic phases are washed with 30 ml of water and concentrated to give a yellow gum. This is flash chromatographed on silica gel using 60/40 hexane/ethyl acetate (1000 ml), ethyl acetate (400 ml), 80/20 acetone/ methanol (500 ml) (30 fractions X 50 ml, 1 X 200 ml). Fractions 21 and 22 yielded a yellow gum which upon recrystalization from hexane yielded title product m.p. 126-127° as a yellow solid.

### EXAMPLE 9

### Lithium 2-chloro-6-(4,6-dimethoxy-α-hydroxy-2-pyrimidinylmethyl)benzoate (Table C, cpd. no. 53)

A mixture of 1.0 g of 7-chloro-3-(4,6-dimethoxy-2-pyrimidinyl)phthalide, 0.136 g of LiOH.H₂O, 2 ml of water and 10 ml of methanol is stirred overnight at RT. The mixture is evaporated to dryness on a rotovaporator. Further drying in a drying pistol yield the title compound as a white solid, m.p. 153-157°C.

### EXAMPLE 10

### Lithium 3-[(4,6-dimethoxy-α-hydroxy-2-pyrimidinyl)methyl]pyridine-2-carboxylate (Table D, cpd. no. 64)

A mixture of 0.490 g of 5-(4,6-dimethoxy-2-pyrimidinyl)furo [3,4,b]pyridine-7(5H)-one, 0.0768 gm of LiOH.H₂O, 10 ml of methanol and 2 ml of water is stirred for 24 hrs under nitrogen at RT and the solvent stripped off. The yellowish solid is dried for a further 2 hrs to yield the title product, m.p. >250°C (decomp.).

### EXAMPLE 11

### Sodium 2-chloro-6-[(4,6-dimethoxy-2-pyrimidinyl)carbonyl]benzoate (Table C, cpd. no. 58)

1.24 g of 7-chloro-3-hydroxy-3-(4,6-dimethoxy-2-pyrimidinyl)phthalide, 154 mg NaOH, 25 ml THF and 25 ml water are mixed until a yellow homogenous solution is achieved. The solvents are stripped on a rotovaporator and then on a Kugelrohr at 100°C to produce the title compound as a yellow solid, m.p. 276-278°C.

### EXAMPLE 12

### 3-[(4,6-dimethoxy-2-pyrimidinyl)carbonyl]-pyridine-2-carboxylic acid (Table D, cpd. no. 63)

490 mg of 5-(4,6-dimethoxy-2-pyrimidinyl)-furo[3,4-b]pyridine-7(5H)-one is dissolved in 50 ml of methanol and the mixture heated with stirring at 50°C until a homogenous solution is formed (ca ½ hr). 2.6 g of NaOCl is added dropwise and the solution heated for a further ½ hr at 55°C. 0.208 g of 50% NaOH is added at 55° and the mixture heated for a further ½ hr at this temperature and then cooled in an ice-bath and acidified with 1 ml conc. HCl. The solvent is evaporated and the residue partitioned between 50 ml of CH₂Cl₂ and 50 ml of water. The organic phase is concentrated to give a white solid, m.p. 71-73°.

### EXAMPLE 13

### 2-[(4,6-dimethoxy-2-pyrimidinyl)-α-iminomethyl]benzoic acid (Table C, cpd. no. 51)

2.67 g of isopropyl 2-bromobenzoate are dissolved in 100 ml of dry diethylether, the solution cooled to -100° C and 6.6 ml of 1.6 M n-butyllithium solution added. Stirring is continued for 10 min and 12 g of 2-cyano-4,6-dimethoxypyrimidine in 60 ml of diethylether is added over 2 min at -100°C. The mixture is stirred for ½ hr at -80° and then allowed to warm to RT. 3 g of NH₄Cl in 30 ml of water is added to the reaction mixture, cooled in an ice-bath. The ether layer is separated off, washed with water (2 x 30 ml) and concentrated. The gummy residue is dissolved in 20 ml of 85/15 hexane/ethyl acetate, and CH₂Cl₂, and flash chromatographed on silica gel using 800 ml 85/15 hexane/ethyl acetate, 500 ml 1% methanol in ethyl acetate, 500 ml 5% methanol in ethyl acetate and 500 ml of 80/20 acetone/methanol (40 fractions at 50 ml; 1 at 200 ml). Fractions 7 to 10 yielded title compound which on recrystallization from CH₂Cl₂ melted at 225-235°C.

### EXAMPLE 14

### 5-Chloro-5-(4,6-dimethoxy-2-pyrimidinyl)furo[3,4,b]pyridine-7(5H)-one (Table B, cpd. no. 68)

A mixture of 490 mg of 5-(4,6-dimethoxy-2-pyrimidinyl)furo[3,4,b]pyridine-7(5H)one and 50 ml of methanol is heated at 55° for 1/2 hour or until a homogenous solution is formed. 2.6 g of NaOCl (common house bleach) is added dropwise. The mixture is taken up in dichloromethane an the organic phase separated and evaporated to dryness to yield the title compound.

### EXAMPLE 15

### 3-[(4,6-dimethoxy-2-pyrimidinyl)carbonyl]-pyridine-2-carboxylic acid (Table E, cpd. no. 63)

0.208 g of 50% NaOH is added at 55° to a solution of 0.551 g of 5-chloro-5-(4,6-dimethoxy-2-pyrimidinyl)-furo[3,4,b]pyridine (Table B, cpd. no. 68) in 50 ml methanol. The mixture stirred for a further 1/2 hr at 55°, cooled in an ice-bath, acidified with 1 ml of concentrated HCl and the solvent evaporated. The residue is partitioned between 50 ml of CH₂Cl₂ and 50 ml H₂O and the CH₂Cl₂ layer concentrated to give 0.39 g of the title product as a white solid, m.p. 71-73°C.

### EXAMPLE 16

### 2-(2-(4,4-dimethyl-oxazolin-2-yl)-benzyl)-4,6-dichloropyrimidine (Table C, cpd. no. 61)

To a mixture of 1.25 g of 2-o-tolyl-4,4-dimethyl-oxazoline in 20 ml of ether under N₂ atmosphere at -30°C is added by syringe 4.2 ml of 1.6 M n-butyllithium in hexane with stirring which is continued for 1 hr at -10°C. 0.98 g of 4,6-dichloropyrimidine in 20 ml of ether are added slowly to the reaction mixture which is then stirred at -45 to -30°C for 30 min and at 0°C for a further 30 min. The reaction mixture is quenched with acetic acid (0.4 ml) and water (0.1 ml) in THF (1.3 ml) and then treated with 1.5 g of 2,3-dichloro-5,6-dicyanobenzoquinone (DDQ) in 6 ml of THF. The temperature is brought to RT and the mixture stirred for 5 min after cooling to 0°C. 7.6 ml of 1N NaOH (cooled) are added and the mixture stirred for 5 min. The organic phase is separated and dried over Na₂SO₄ filtered and the solvent removed. Following chromatography (10/90 ether/hexane) the title product is obtained.

### EXAMPLE 17

### 2-(2-(4,4-dimethyl-oxazolin-2-yl)-benzyl)-4,6-dimethoxypyrimidine (Table C, cpd. no. 48)

To a solution of 1.7 g of 2(2-(4,4-dimethyl-oxazolin-2-yl)-benzyl)-4,6-dichloropyrimidine in 100 ml of methanol are added 2.18 g of 25% methanolic NaOCH₃ and the mixture heated for 10 hrs at 65°C with stirring. The temperature is lowered to 60° and stirring continued overnight. The solvent is stripped and the residue taken up in 80 ml of toluene and 50 ml of water. The toluene layer was separated and washed with 50 ml of water, separated and concentrated to give the title compound as a yellow oil.

### EXAMPLE 18

### 2-(2-(4,4-dimethyl-oxazolin-2-yl)-α-bromobenzyl)-4,6-dimethoxy pyrimidine (Table C, cpd. no. 62)

0.55 g of 2-(2-(4,4-dimethyl-oxazolin-2-yl)-benzyl)-4,6-dimethoxypyrimidine, 0.30 g of a N-bromosuccinimide, 0.03 g of benzoyl peroxide are dissolved in 60 ml of CCl₄ and heated under reflux overnight at 75°C. The reaction mixture is filtered and the filtrate washed with 5% NaHCO₃ solution (50 ml), 50 ml of water and the organic phase separated and concentrated to give the title compound.

### EXAMPLE 19

### 2-(2-(4,4-dimethyl-oxazolin-2-yl)-benzoyl)4,6-dimethoxypyrimidine (Table C, cpd. no. 49)

A mixture of 1.2 g of 2-(2-(4,4-dimethyl-oxazolin-2-yl)-α-bromobenzyl)-4,6-dimethoxy-pyrimidine and 2 g of Na₂CO₃ in 30 ml of DMSO is heated with stirring at 50-60°C for 3 hrs. The mixture is poured into 150 ml of water and extracted with toluene. The toluene extract is washed twice with water (2 x 50 ml) separated and concentrated. The thus obtained gum is chromatographed with 800 ml of 80/20 hexane/ethyl acetate, 500 ml 70/30 hexane/ethyl acetate, 60/40 ml hexane/ethyl acetate (50 ml fractions) fractions 29 to 34 yielded the title compound.

### EXAMPLE 20

### 2-chloro-6-(4,6-dimethoxy-2-pyrimidinylcarbonyl)-benzoic acid dimethylamide (Table C, cpd. no. 57)

1.0 g of 7-chloro-3-cyano-3-(4,6-dimethoxy-2-pyrimidinyl) phthalide is dissolved in 15 ml of THF. 0.7 ml of a 40% aqueous dimethylamine solution is then added via syringe whereupon the solution darkens. Stirring is continued at R.T. for 15 minutes and the mixture diluted with water and partitioned between ethyl acetate and water. The organic phase is separated, washed with 2N H₂SO₄, then brine, dried and concentrated. The residue is purified on silica gel, eluant 200 ml of 50% ethyl acetate/hexane then 100% ethyl acetate. Fractions 12 to 15 yielded the title compound, m.p. 141-142°C.

### EXAMPLE 21

### 3-acetoxy-7-chloro-3-(4,6-dimethyloxy-2-pyrimidinyl)phthalide (Table A, cpd. no. 125)

1.1gof7-chloro-3-(4,6-dimethoxy-2-pyrimidinyl)-3-hydroxy-phthalide is dissolved in 20 ml of pyridine and 0.3 ml of acetic anhydride added with stirring. After stirring for 20 min the mixture is poured into 2N HCl and extracted with two portions of ethylacetate. The combined ethyl acetate extracts are washed once with 2N HCl, once with H₂O and once with brine and dried over magnesium sulfate. Filtration and evaporation produced the title compound as a white solid, m.p. 213-215°.

### EXAMPLE 22

### 3-[(4,6-dimethoxy-α-hydroxy-2-pyrimidinyl)methyl]pyridine-2-carboxamide (Table E, cpd. no. 82)

To a solution of 0.9 g of ammonia, in 15 ml of methanol, is added 0.5 g of 3[(4,6-dimethoxy-2-pyrimidinyl)-7-azaphthalide. After stirring for 2 hrs at RT, the methanol is removed under reduced pressure and the concentrate recrystallized from toluene to give the title compound as a white solid, m.p. 135-137°C.

### EXAMPLE 23

### 3[(4,6-dimethoxy-2-hydroxy-2-pyrimidinyl)methyl]pyridine-2-[carboxy(4-isopropyl)anilide] (Table E, cpd. no. 183)

To a solution of 3 ml of 4-isopropylaniline in 50 ml of toluene is syringed in 4 ml of 15.6% trimethylaluminum in hexane at RT. The mixture is stirred for 0.5 hr at RT and 0.5 g of 3-[(4,6-dimethoxy-2-pyrimidinyl)-7-azaphthalide is added. The mixture is stirred for 2 hrs at RT and acidified with 30 ml of 10% hydrochloric acid at 5-10°C. The toluene solution is separated, washed with 20 ml of 10% hydrochloric acid, 20 ml of 5% sodium carbonate and 20 ml of water, dried and concentrated. The concentrate is recrystallized from hexane to yield the title compound as a white solid, m.p. 113-114°C.

### TABLE 24

### 3-[(4,6-dimethoxy-α-(ethoxycarbonyloxy)-2-pyrimidinyl)methyl]pyridine-2-carbaxamide (Table E, cpd. no. 129)

To a solution of 0.5 g of 3-[(4,6-dimethoxy-α-hydroxy-2-pyrimidinyl)methyl]pyridine-2-carboxamide, 0.05 g of 4-(dimethylamino)pyridine, and 1 ml of triethylamine, in 20 ml of toluene and 10 ml of dichloromethane is added 1 ml of ethyl chloroformate at RT. After stirring for 1 hr at ambient temperature, the mixture is washed with water (2x30 ml), dried and concentrated on a rotoevaporator. The concentrate is digested with v/v mixture of hexane-toluene, 10 ml, at 50°C, cooled to RT and filtered to isolate 0.45 g of the title compound as a yellow solid, m.p. 112-114°C.

### EXAMPLE 25

### 3-[(4,6-dimethoxy-α-benzoyloxy-2-pyrimidinyl)methyl]pyridine-2-(N,N-dibenzoyl)carboxamide (Table E, cpd. no. 159)

To a solution of 0.05 g of 3-[(4,6-dimethoxy-α-hydroxy-2-pyrimidinyl)methyl]-2-carboxamide, 0.5 g, 4-(dimethylamino)pyridine and 4 ml of triethylamine in 30 ml of dichloromethane is added 1.4 g of benzoyl chloride at RT in two portions. The reaction mixture is stirred at RT for 17 hrs and washed with 30 ml of water, 30 ml of 5% hydrochloric acid and 30 ml of water. The dichloromethane solution is concentrated and the concentrate flash chromatographed through 300 ml silica gel, 230-400 mesh, using 1 L 70/30 hexane-ethyl acetic and 500 ml 50/50 hexane-ethyl acetate as eluting solvent mixtures. Fractions 18-21 gave after recrystallization from 70/30 hexane ethyl acetate the title compound as a white solid, m.p. 168-170°C.

### EXAMPLE 26

### 3-[(4,6-dimethoxy-α-(N-methylcarbamoyloxy)-2-pyrimidinyl)methyl]-2-pyridine carbox(N-allyl)amide (Table E, cpd. no. 133)

To a solution of 0.5 g of 3-[(4,6-dimethoxy-α-hydroxy-2-pyrimidinyl)methyl]-2-pyridine carbox(N-allyl)amide and 3 drops of triethylamine, in 20 ml of dichloromethane is added 3 ml of methyl isocyante, in three 1 ml portion/day while stirring at RT for 3 days. The reaction mixture is washed with water (2x50ml), dried and concentrated. The concentrate is flash chromoatographed through 300 ml silica gel, 230-400 mesh, using 1 L 50/50 hexane-ethyl acetate, 500 ml ethyl acetate, 500 ml 80/20 ethyl acetate methanol taking 34 fractions (50 m/m). Fractions 21-25 give 0.4 g of the title product as a yellow gum.

The following compounds may be prepared analogously to the preceding examples or as otherwise described herein.

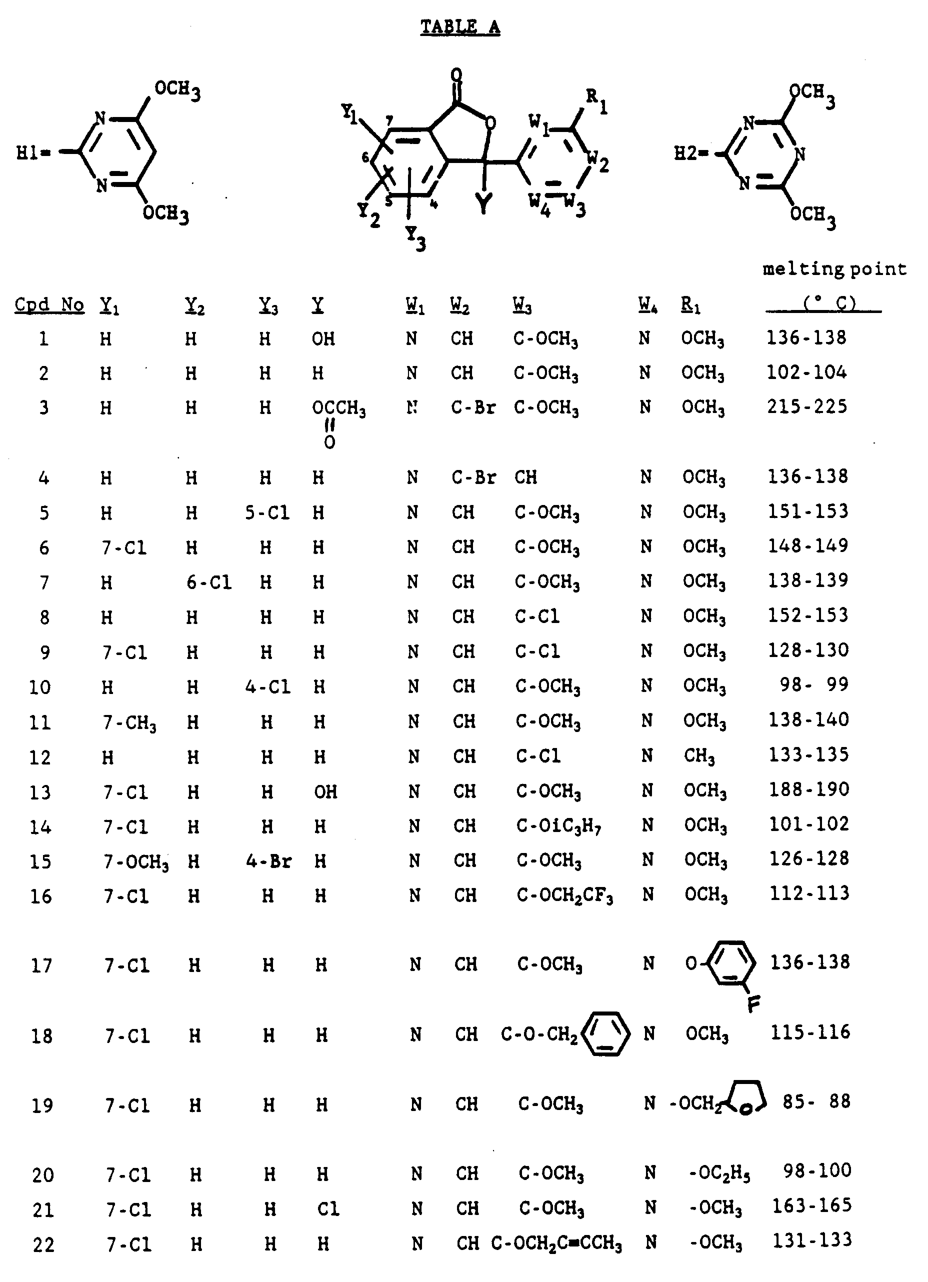

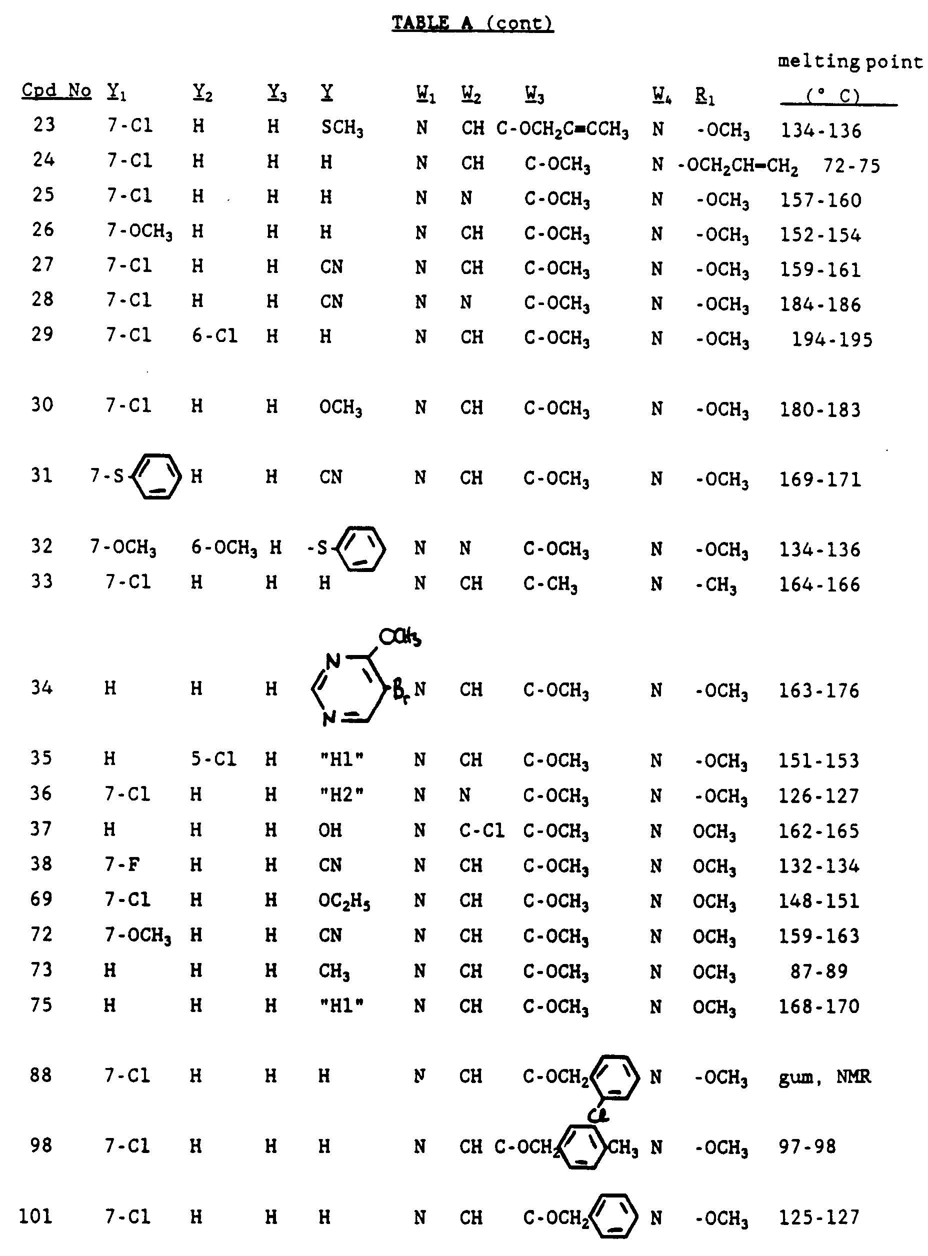

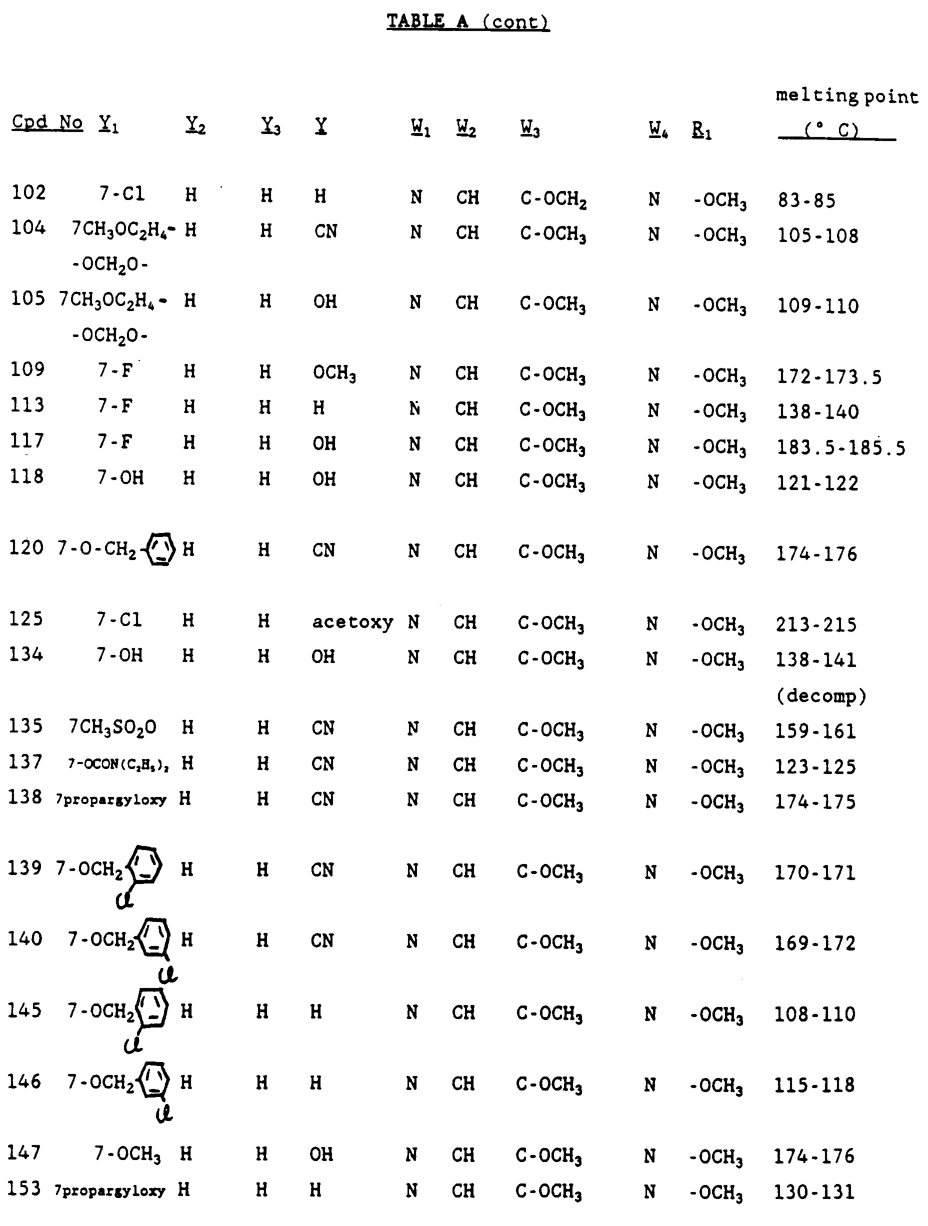

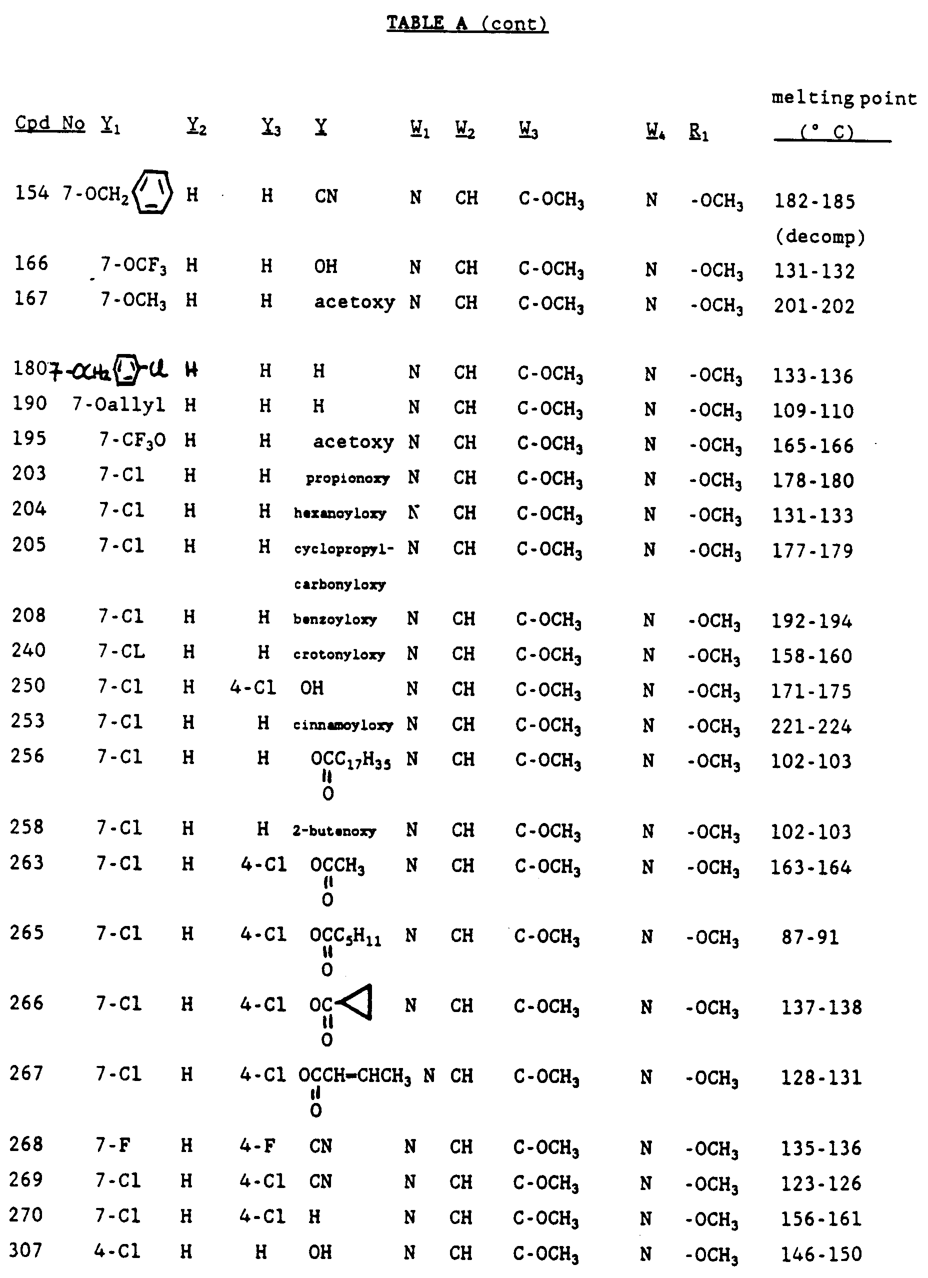

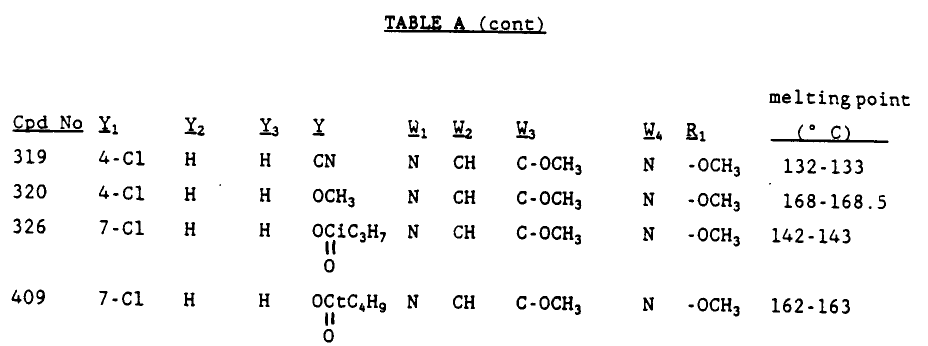

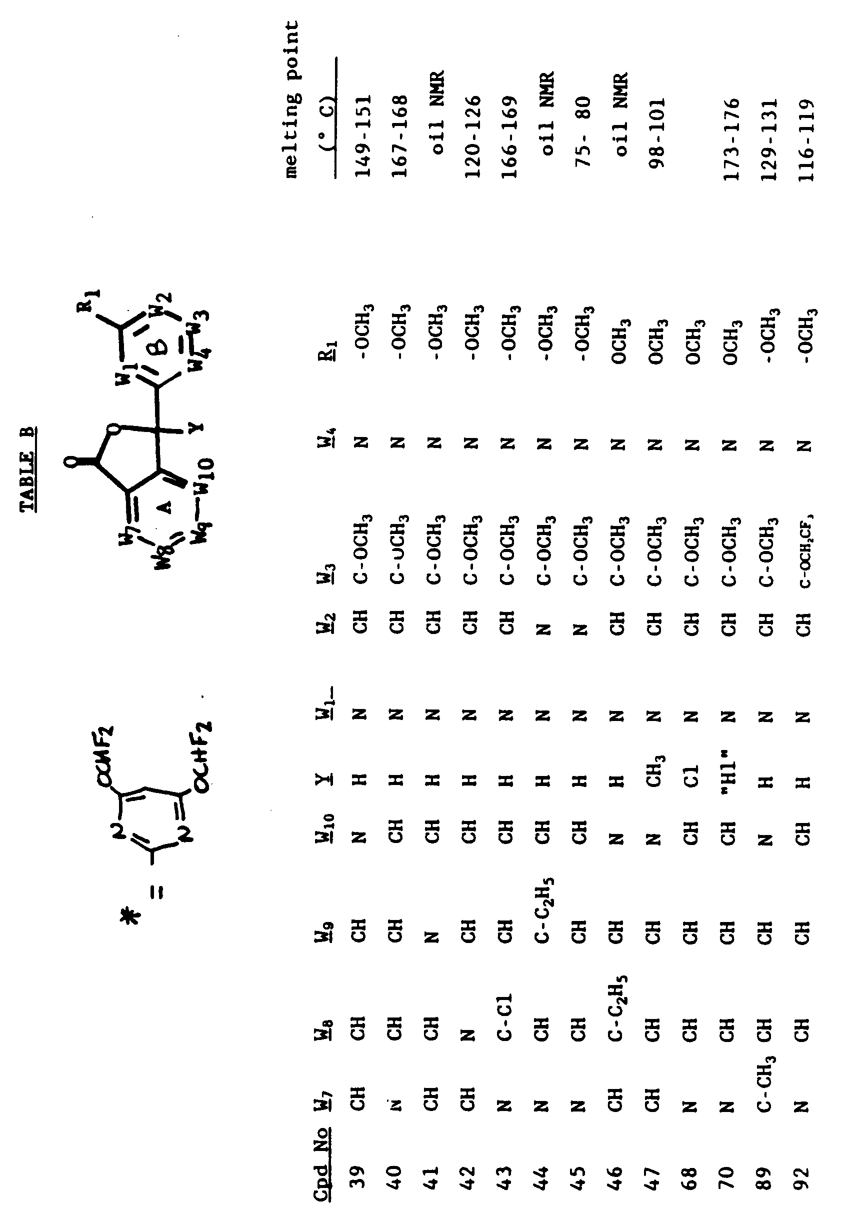

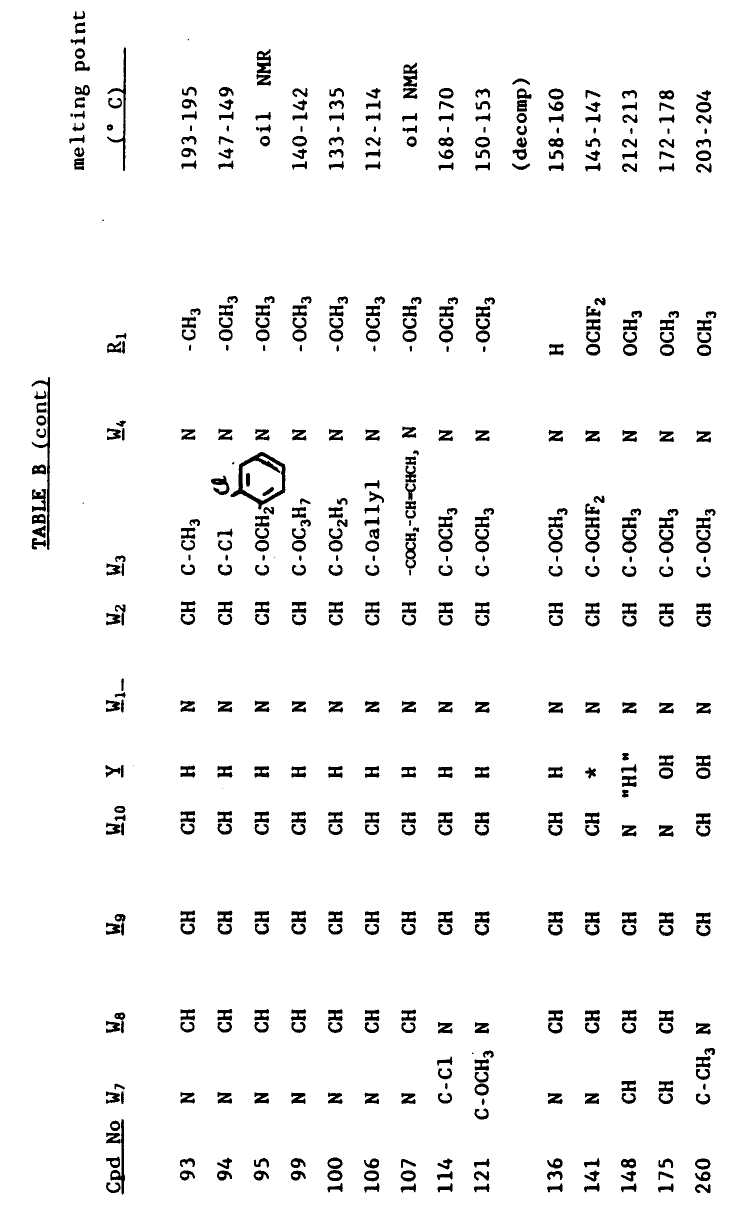

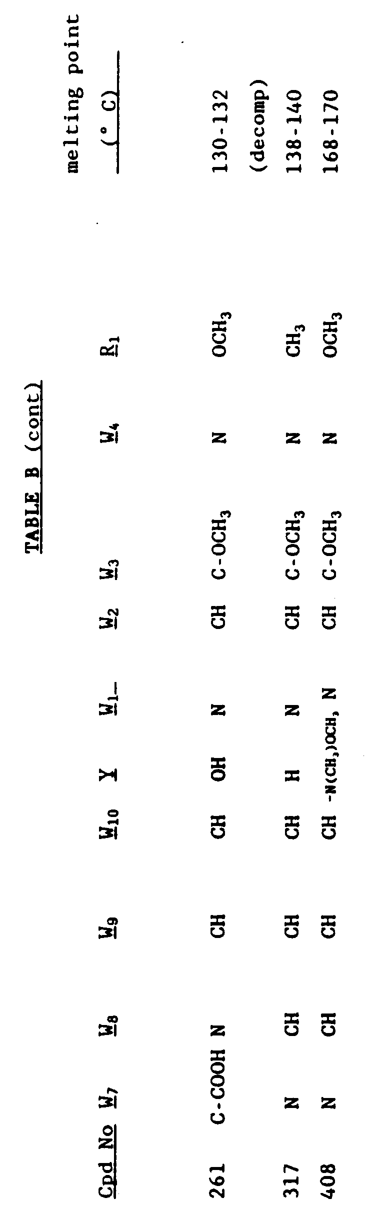

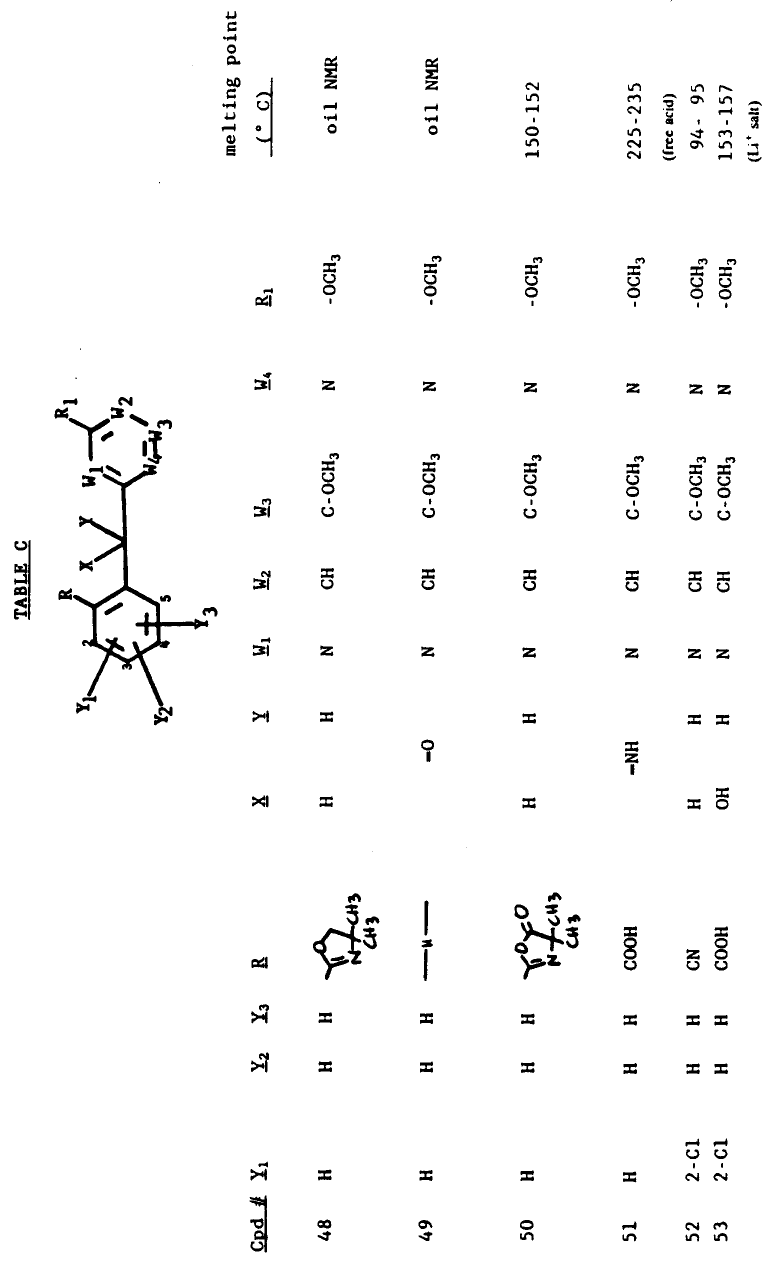

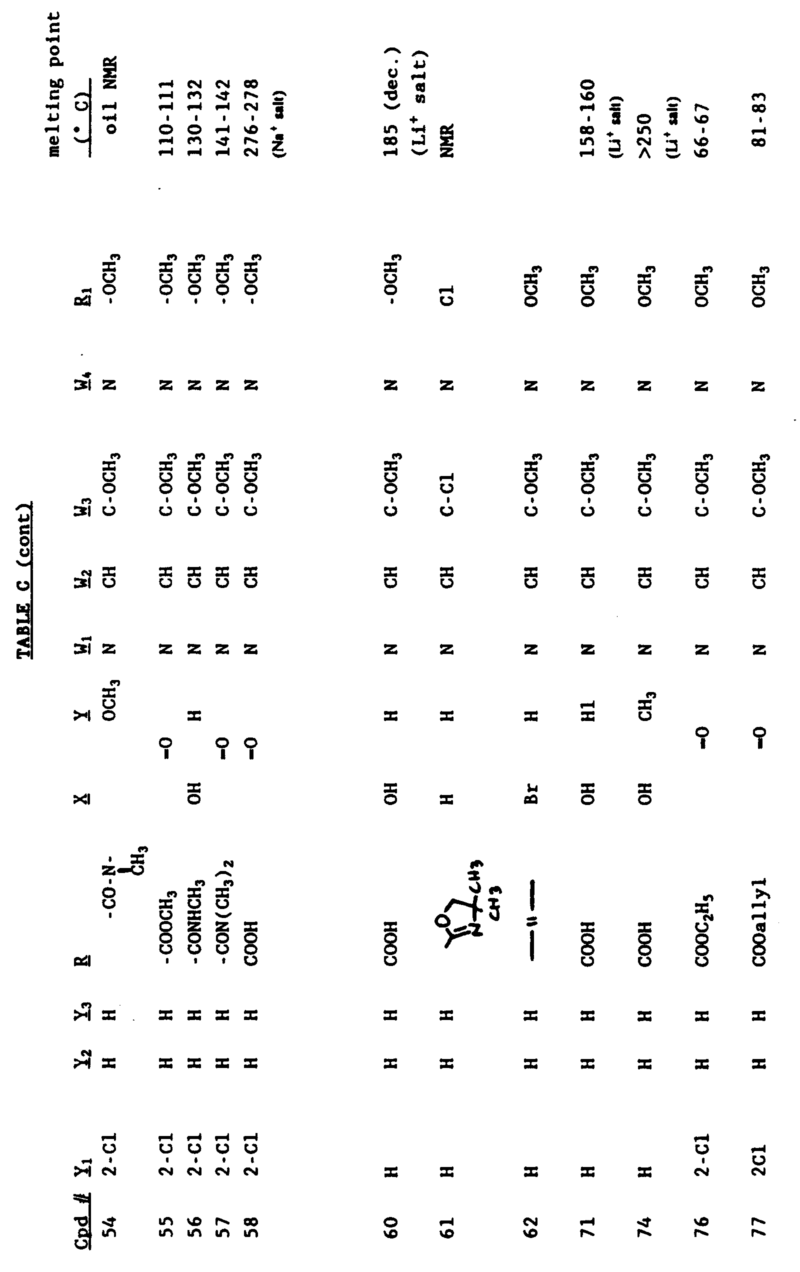

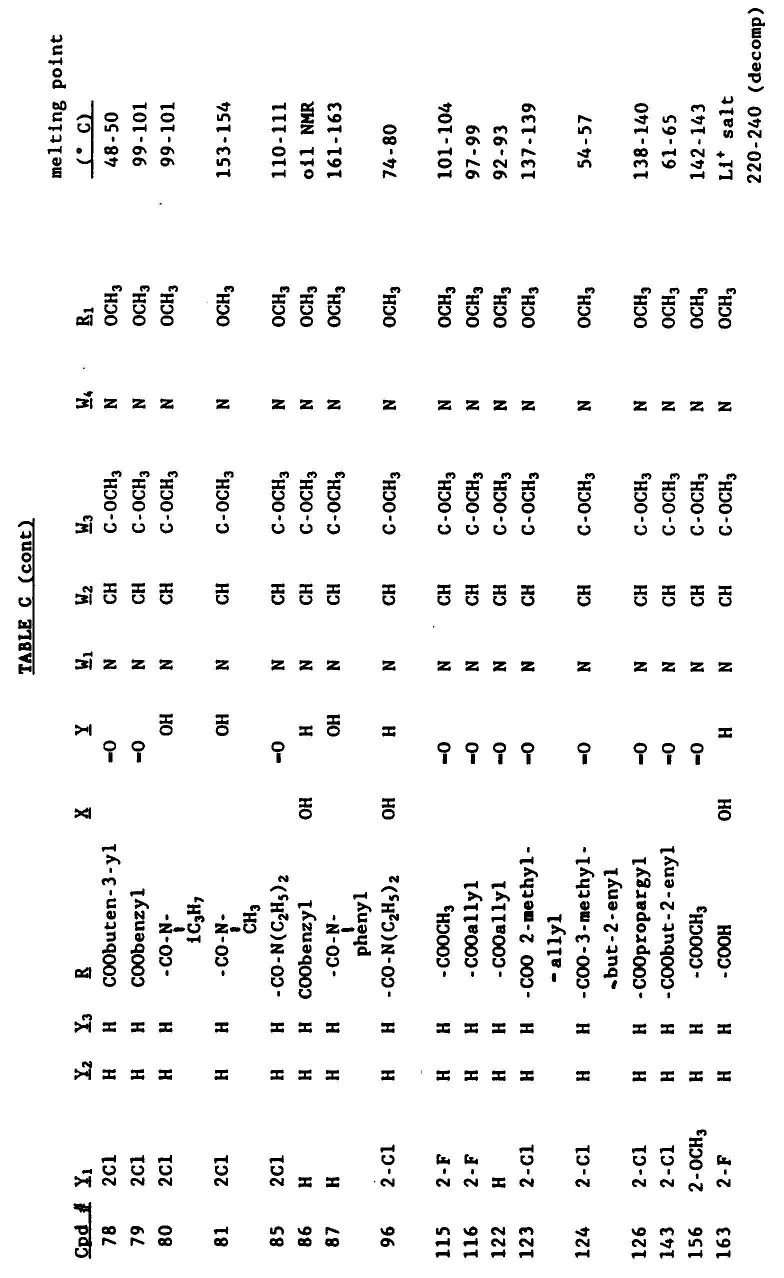

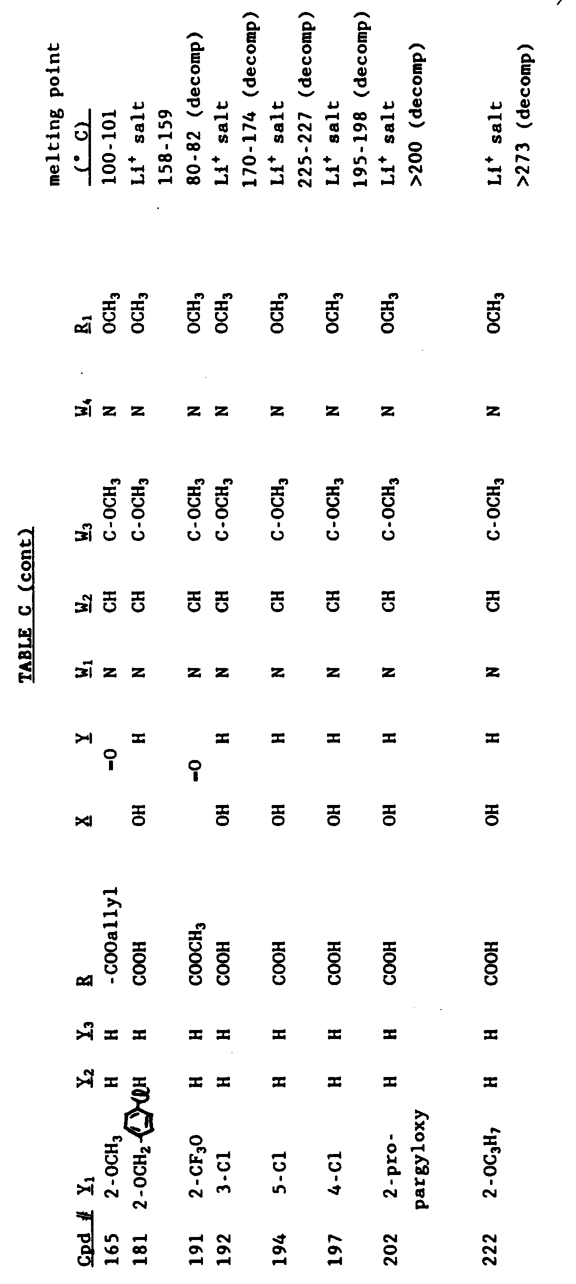

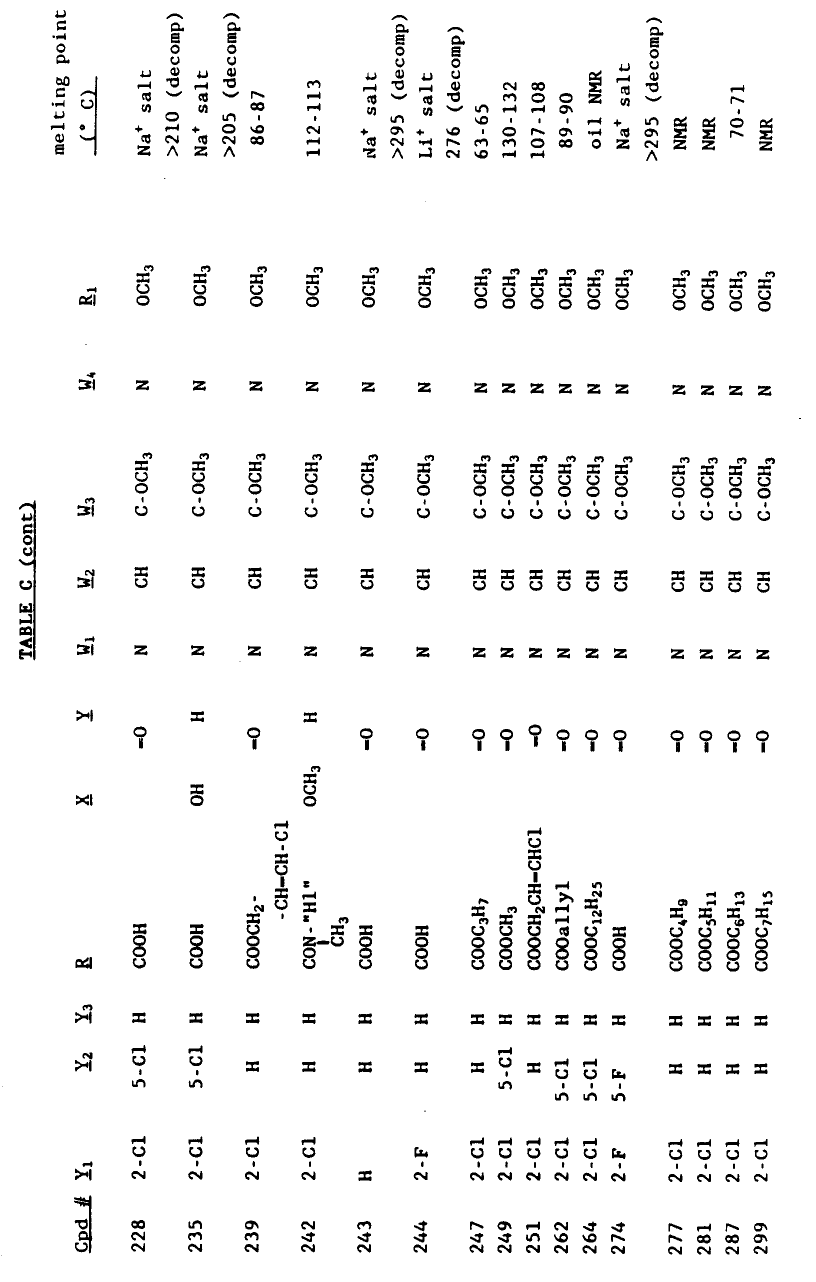

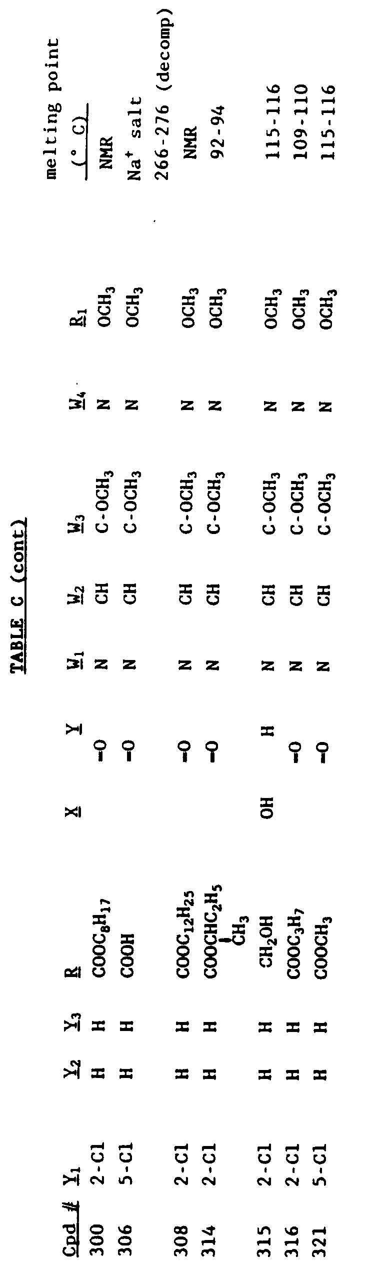

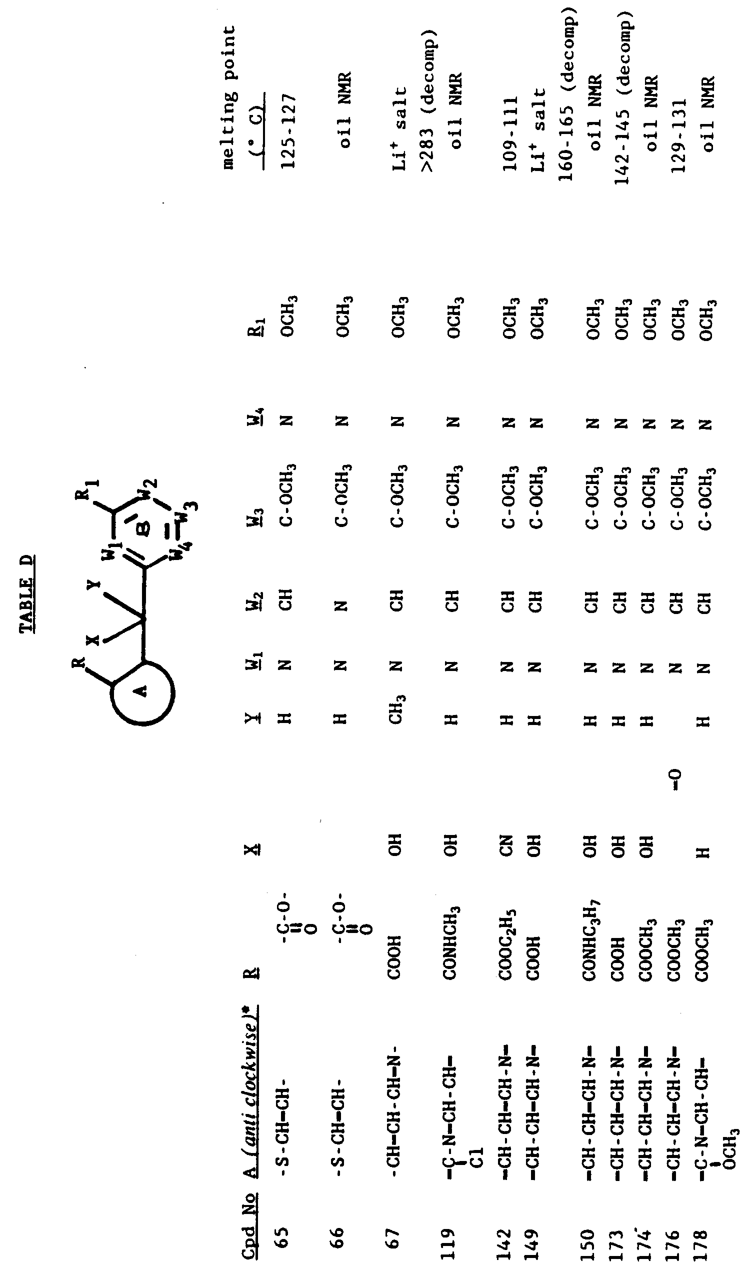

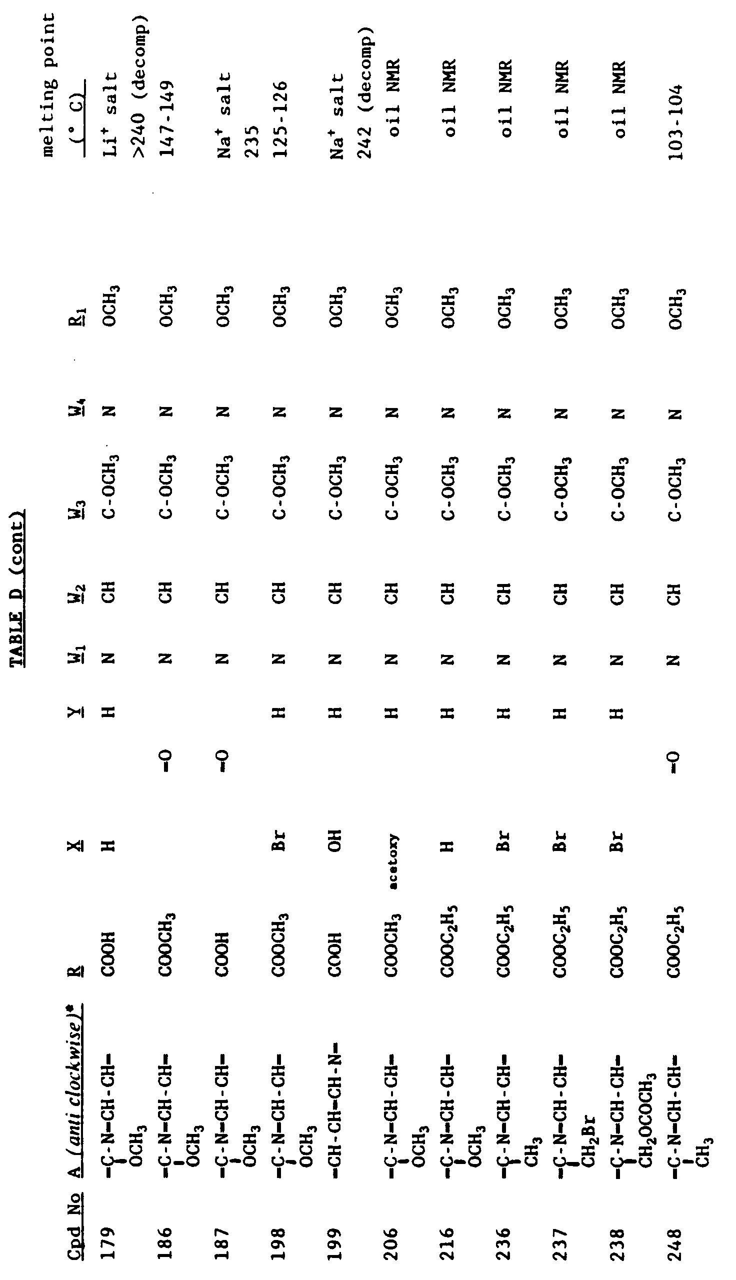

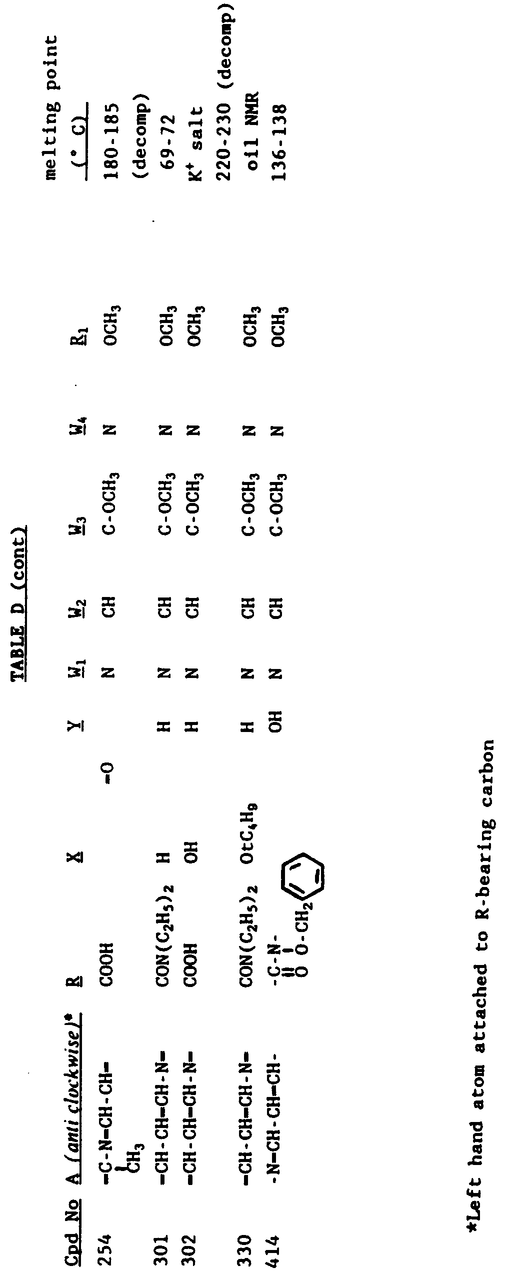

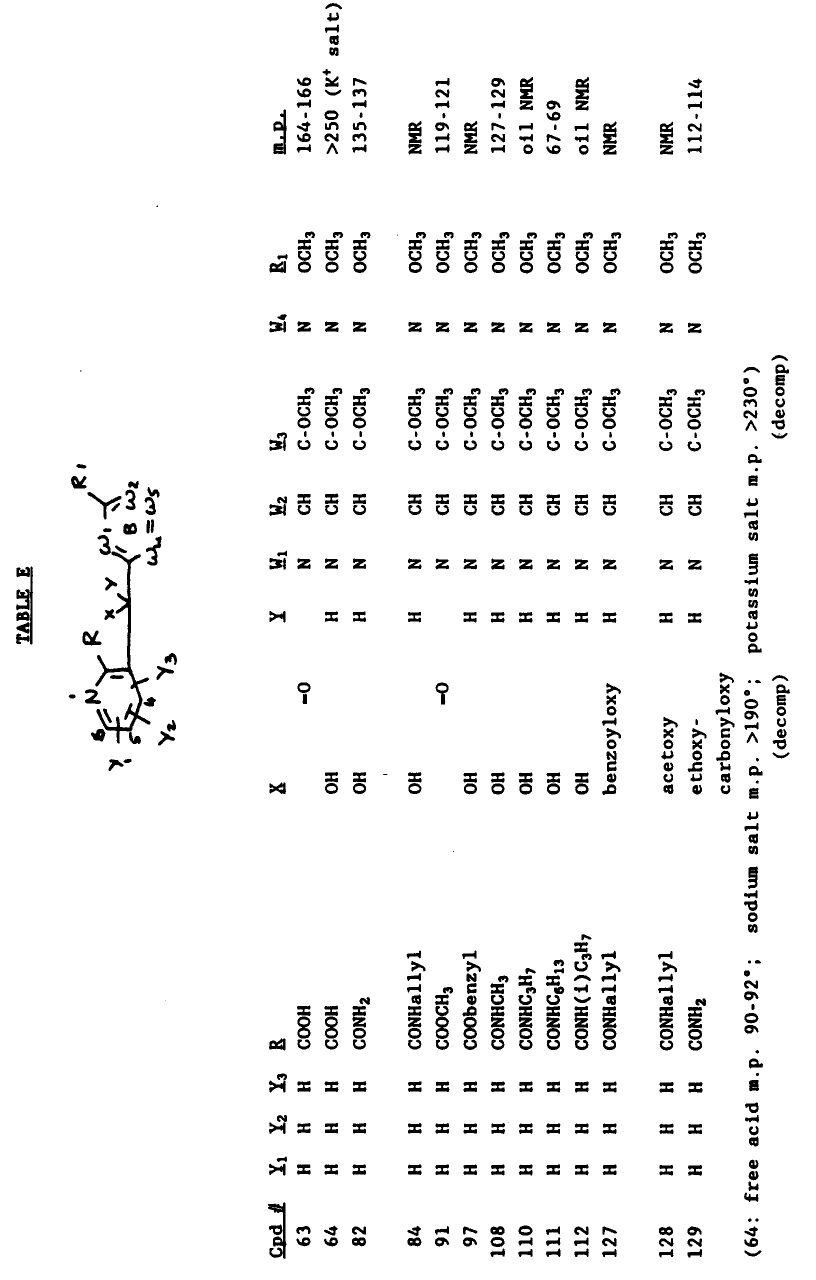

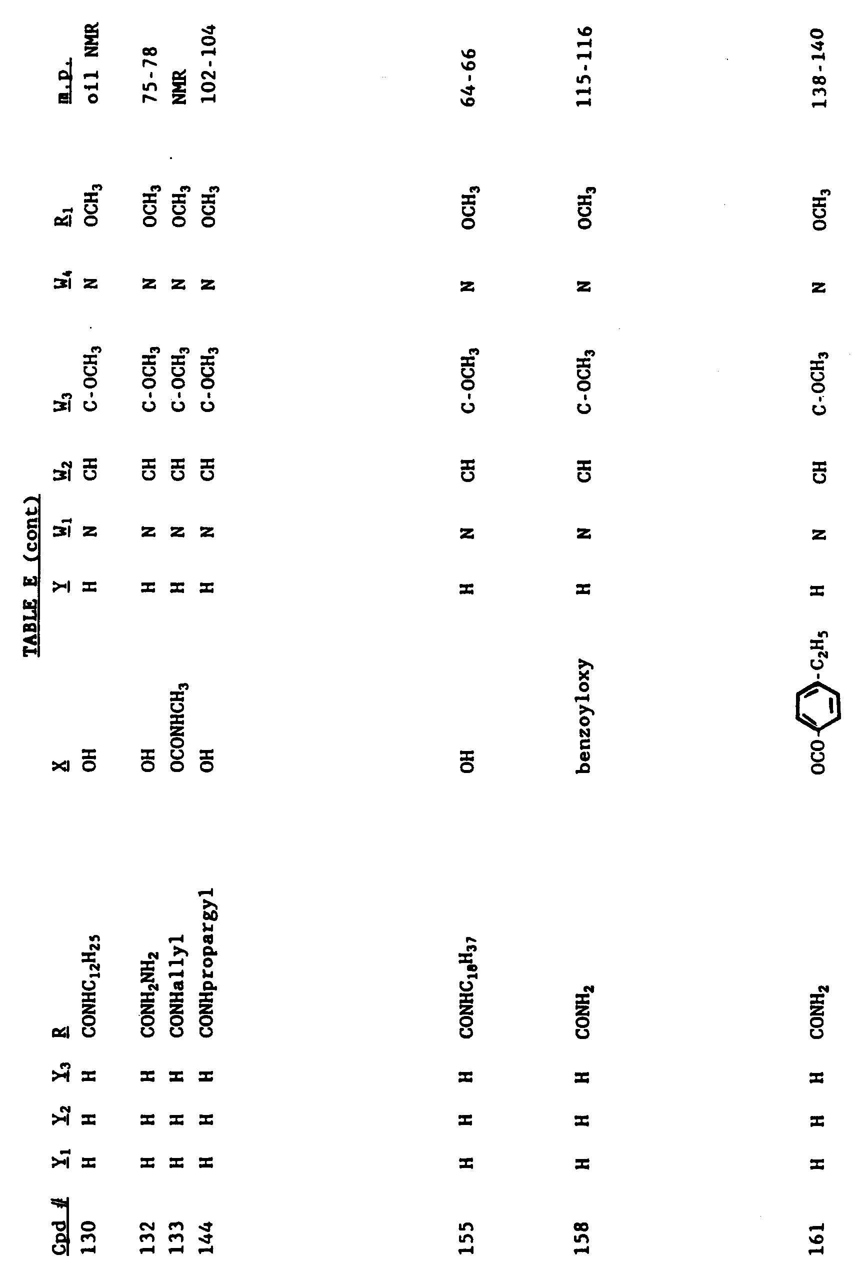

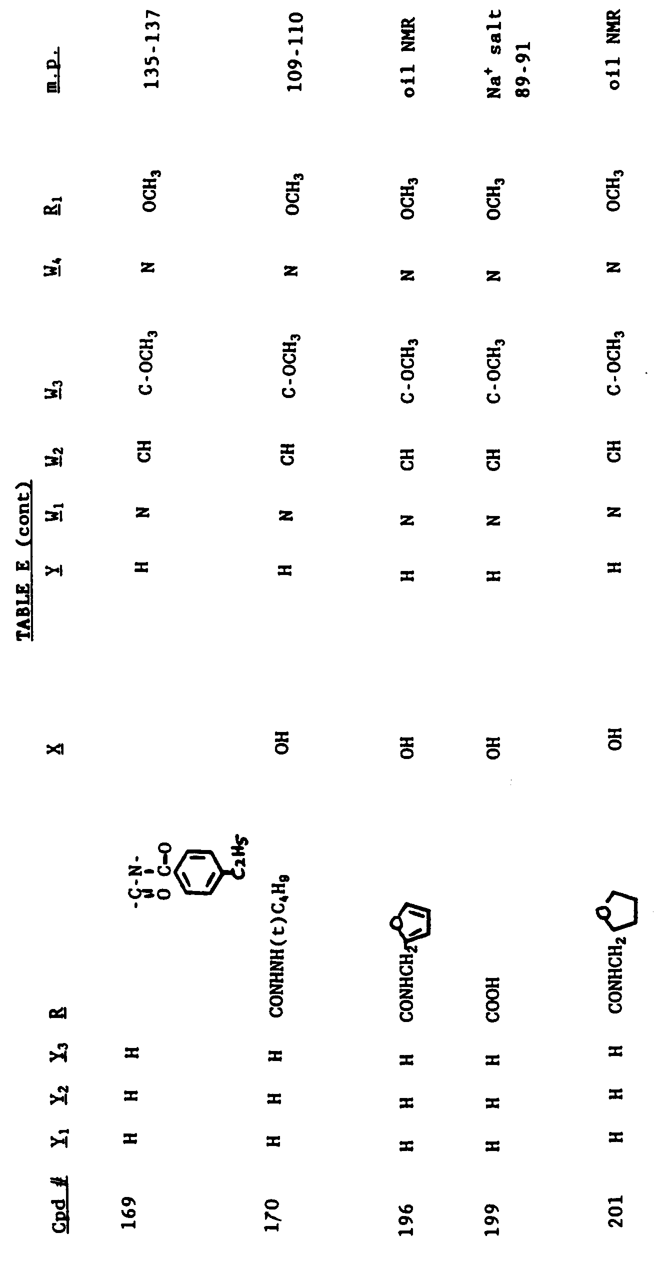

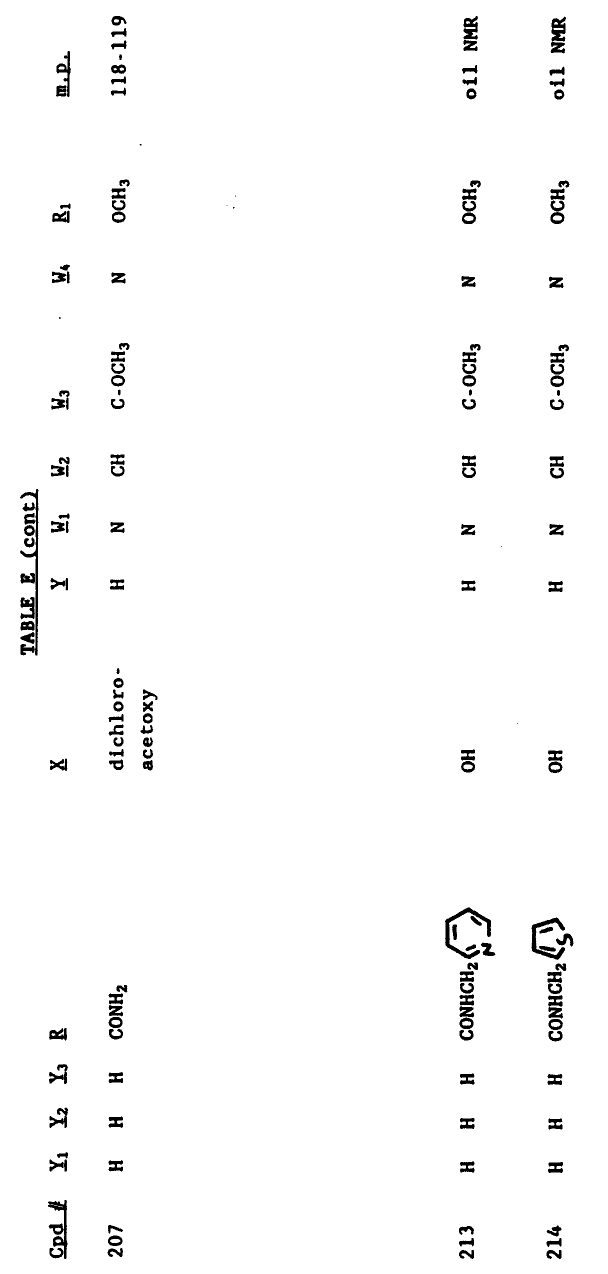

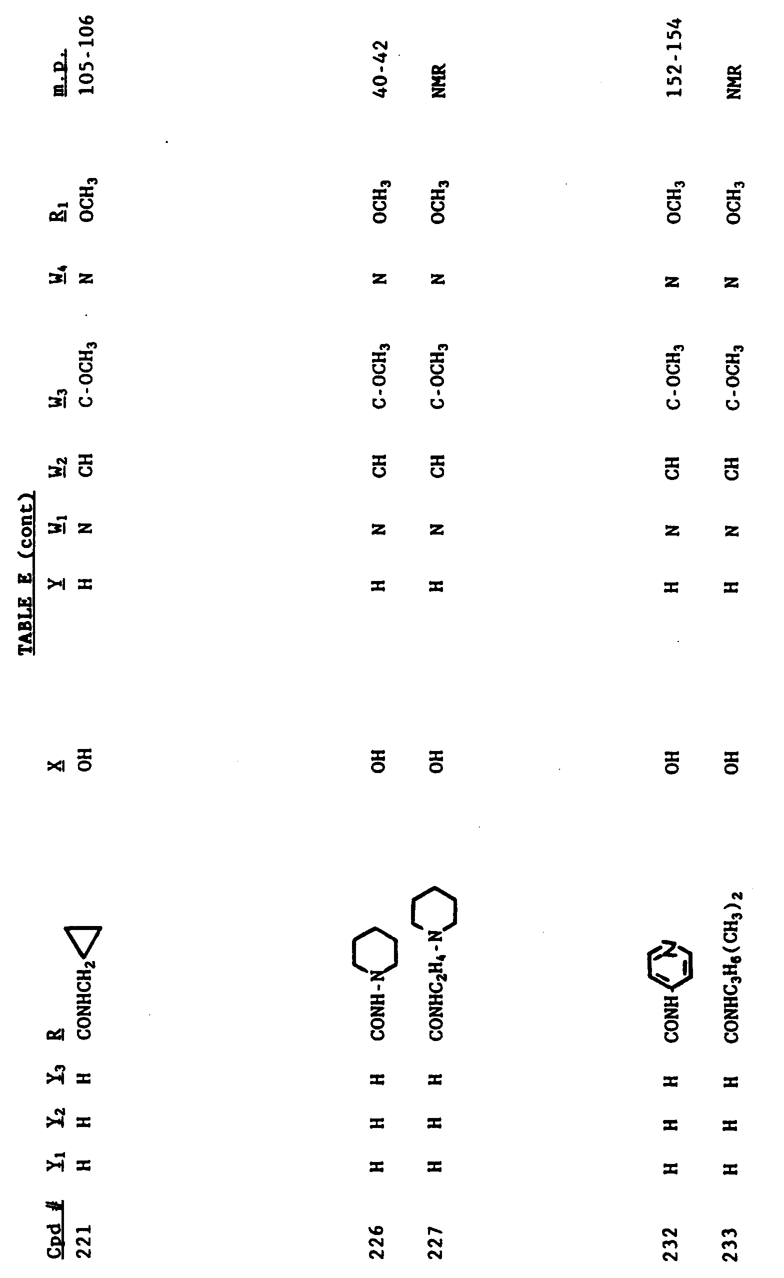

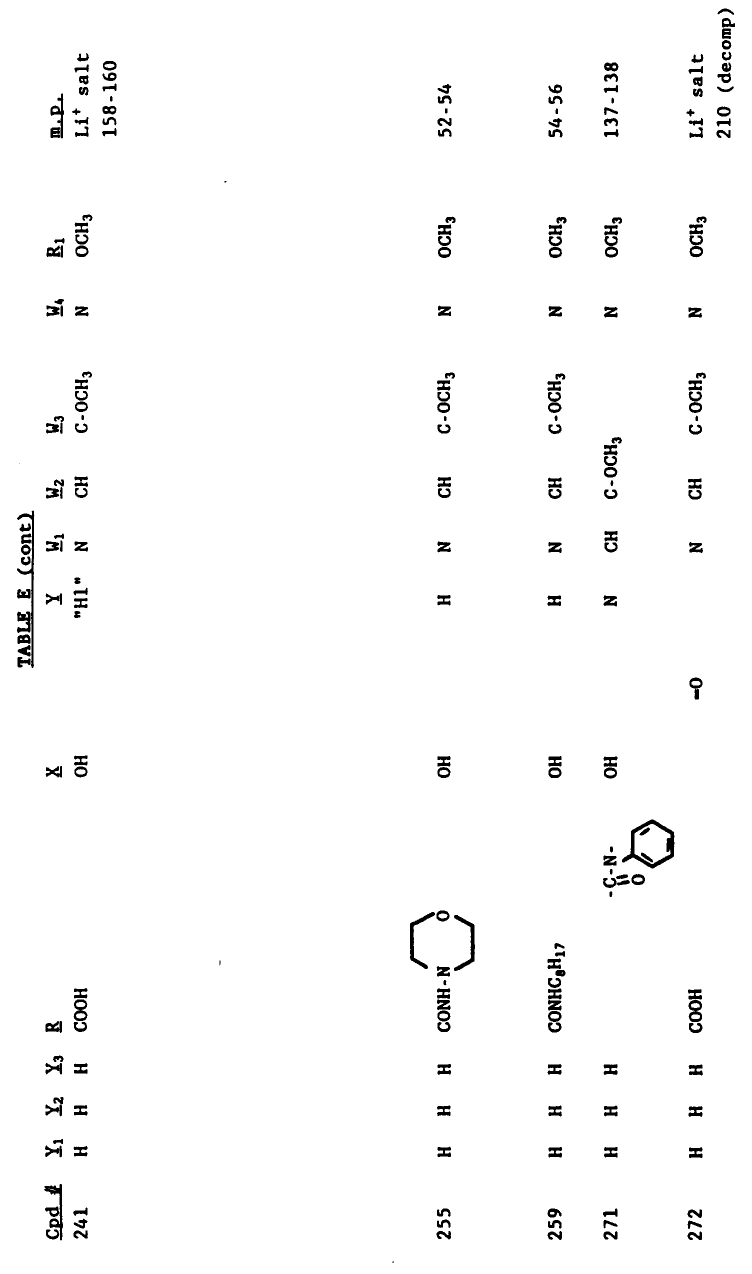

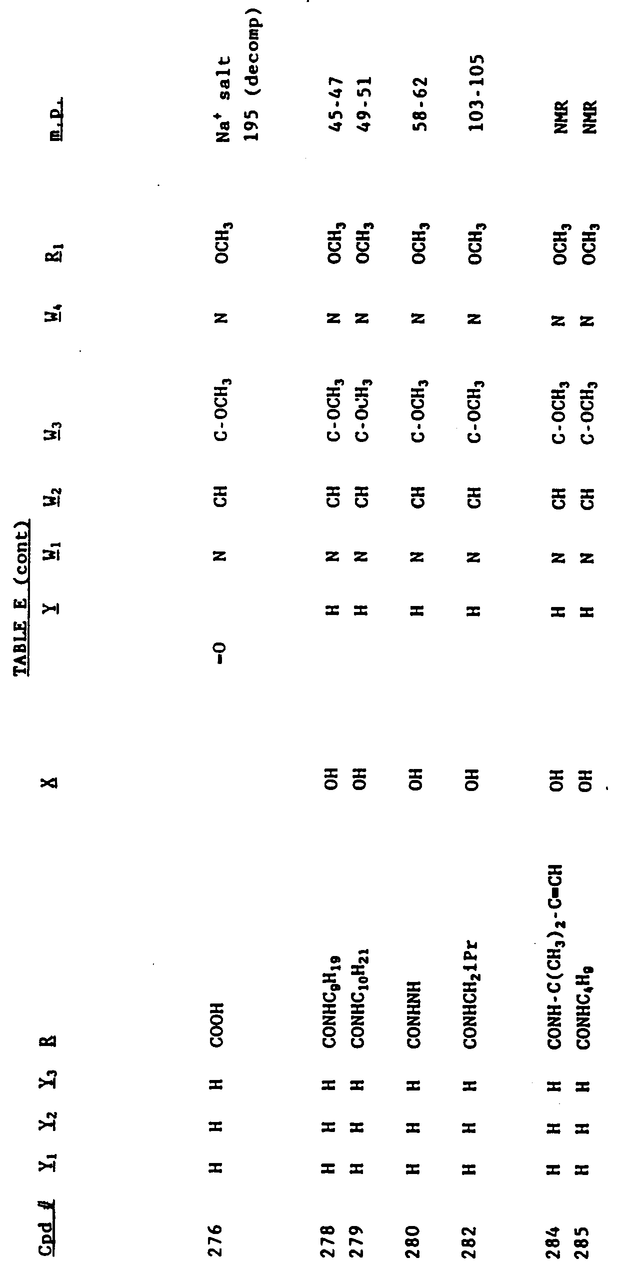

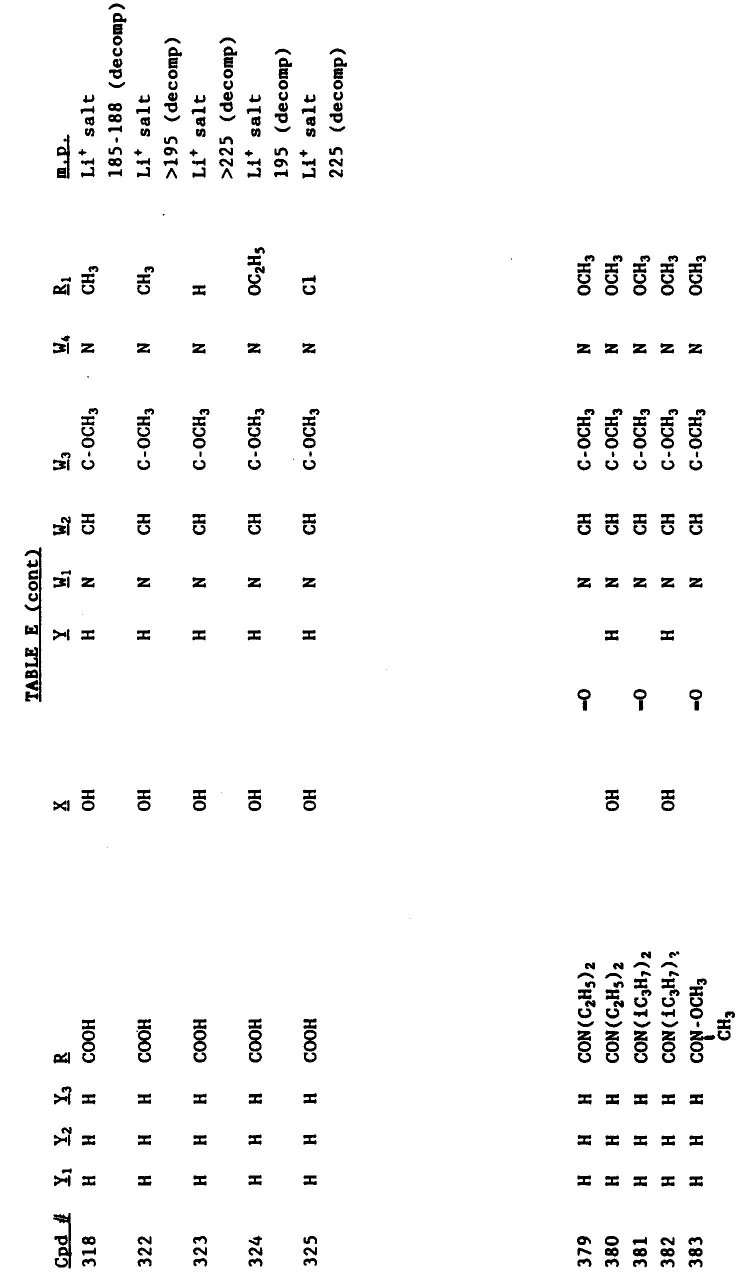

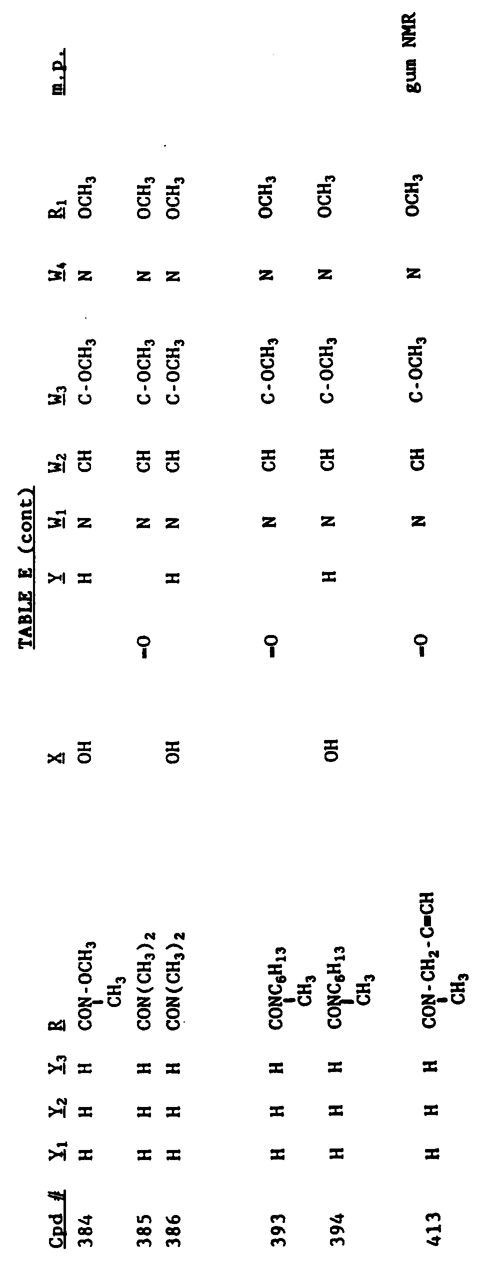

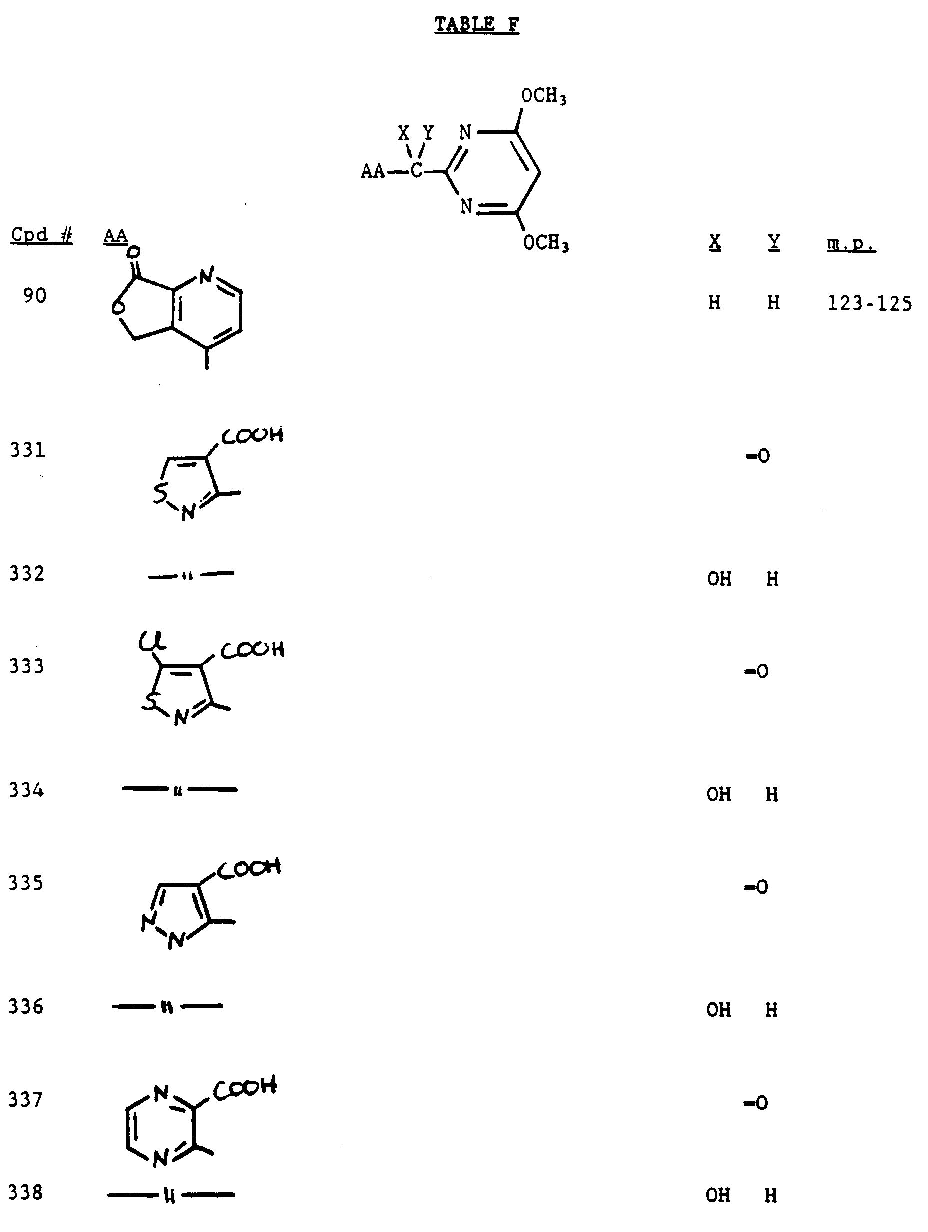

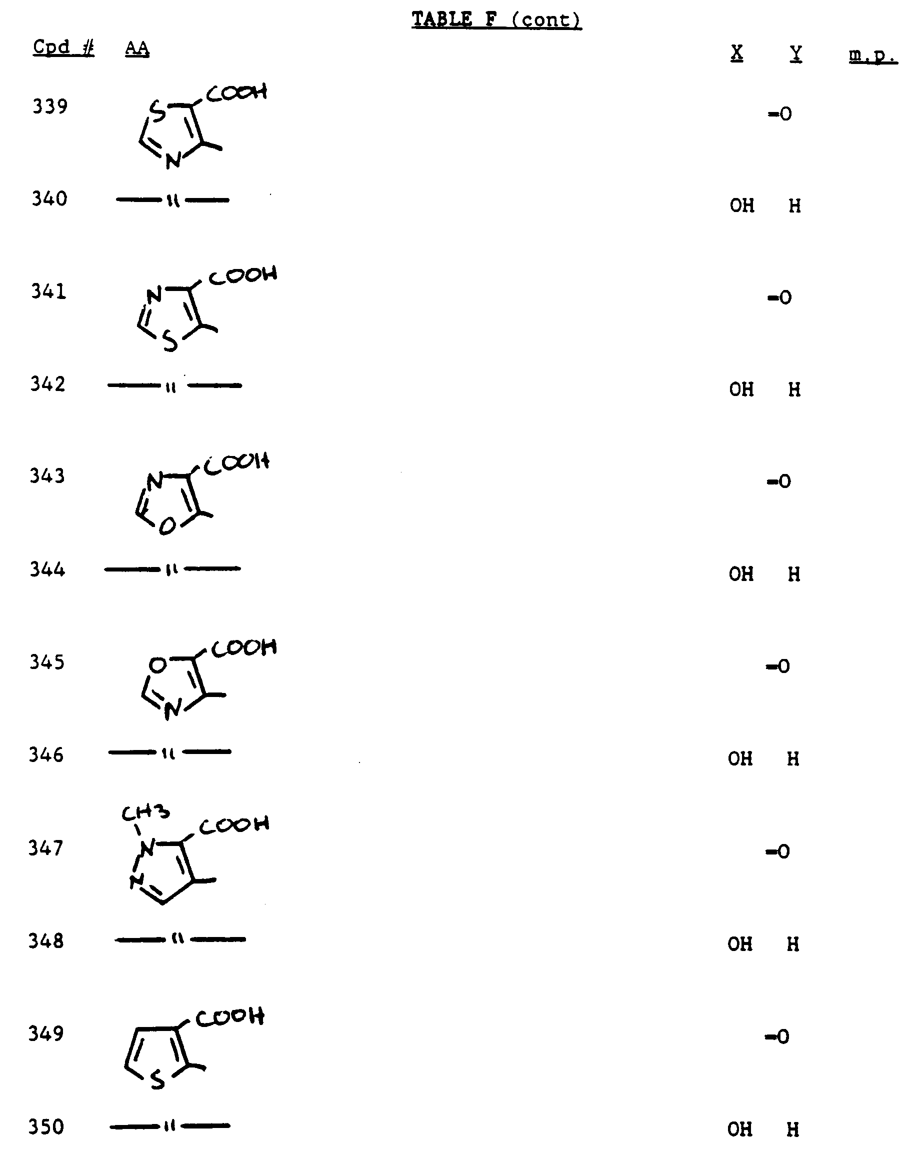

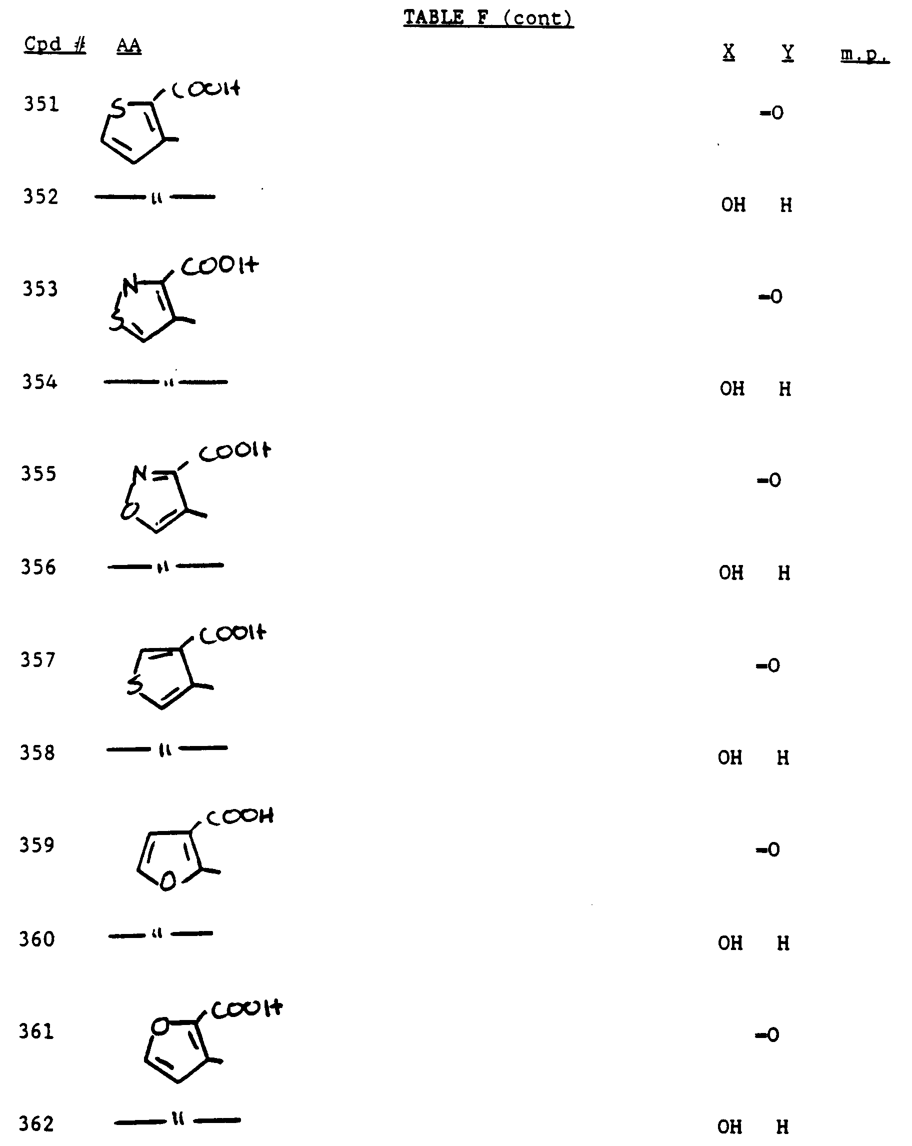

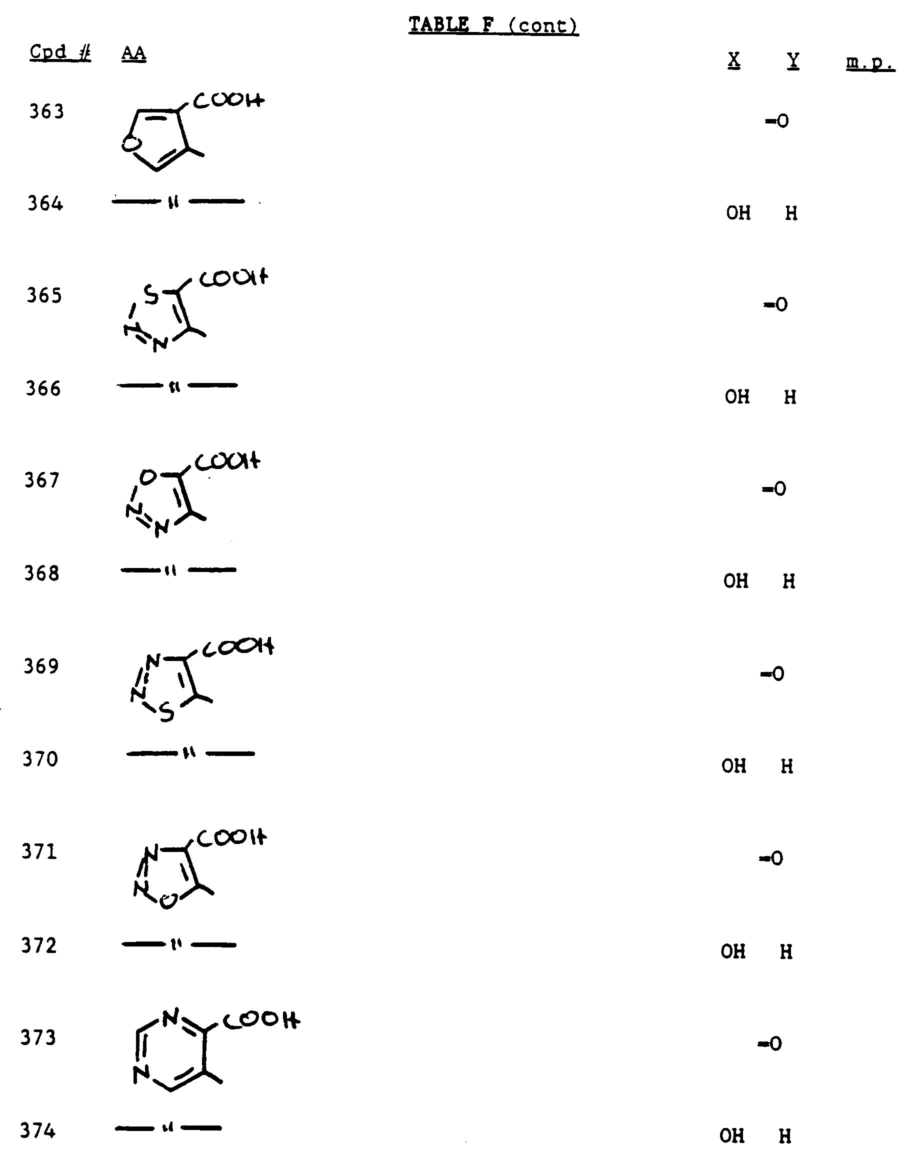

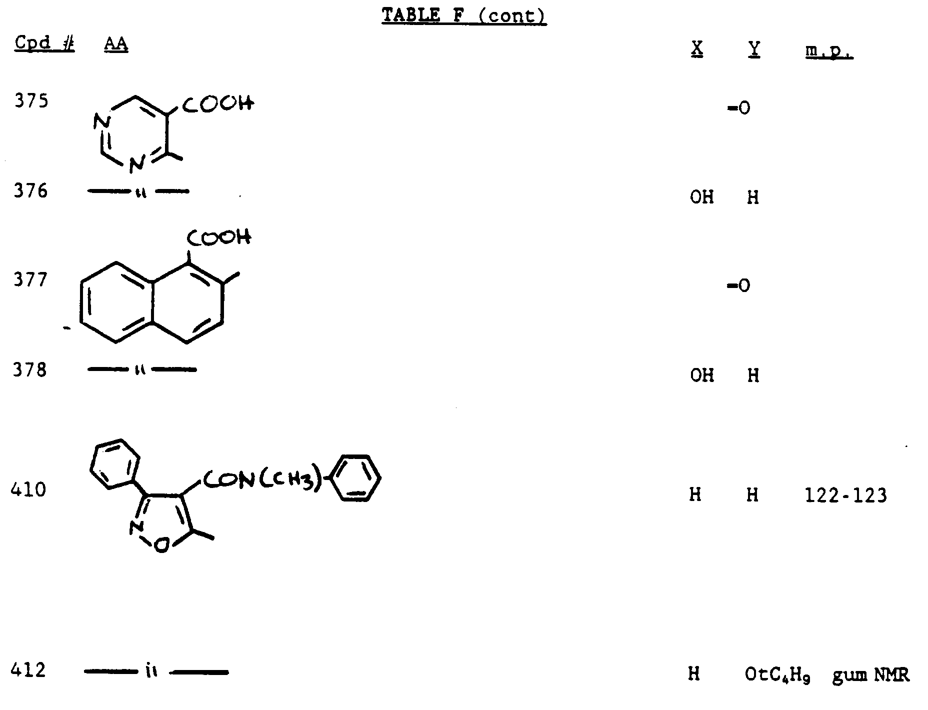

Compounds of Table F wherein COOH is replaced by other meanings of R as listed in Tables C, D and E above for R may be prepared analogously.

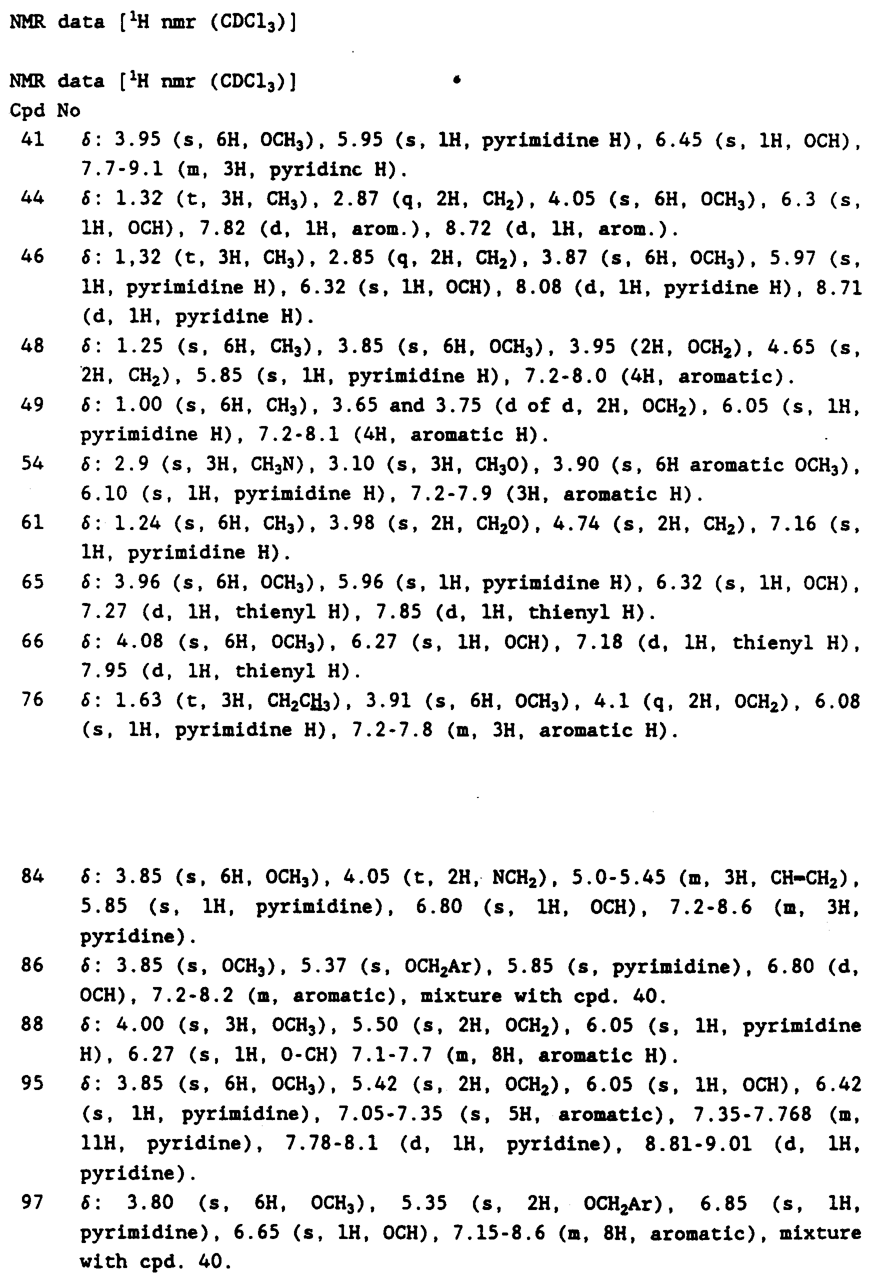

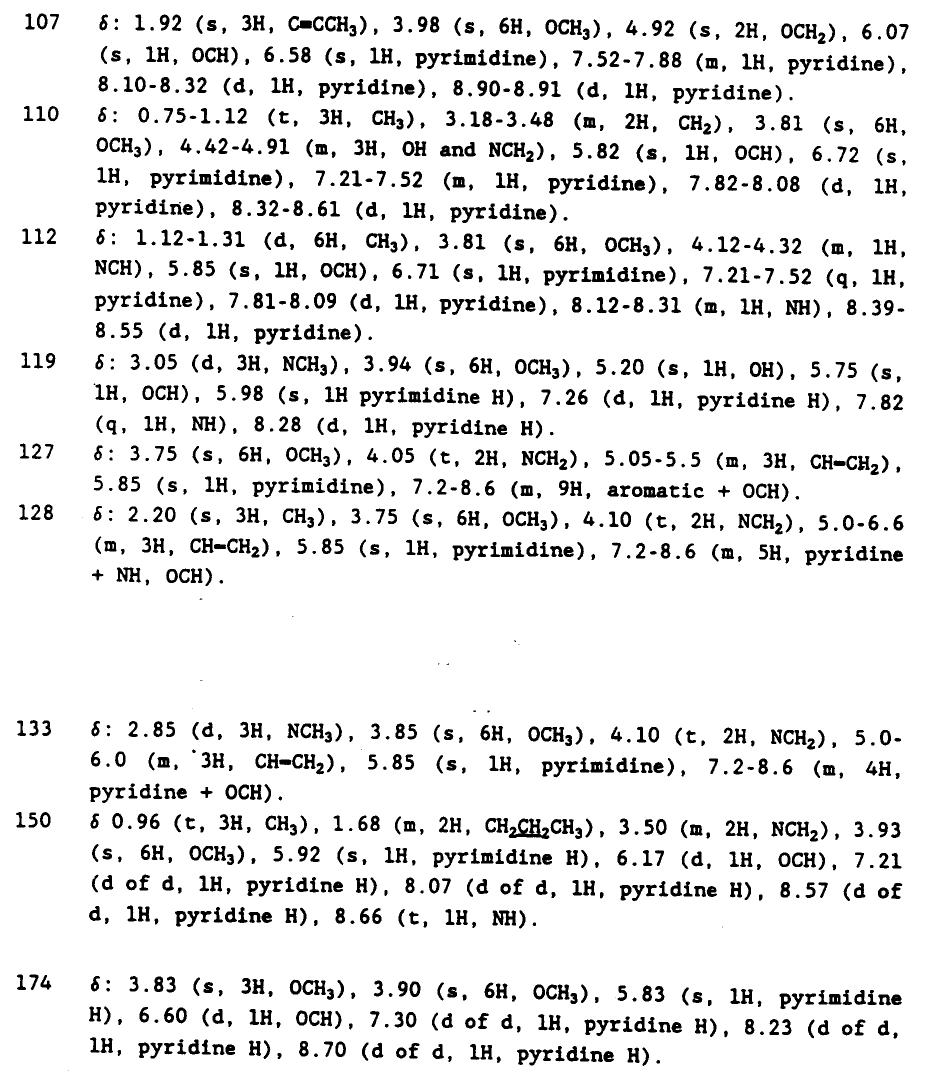

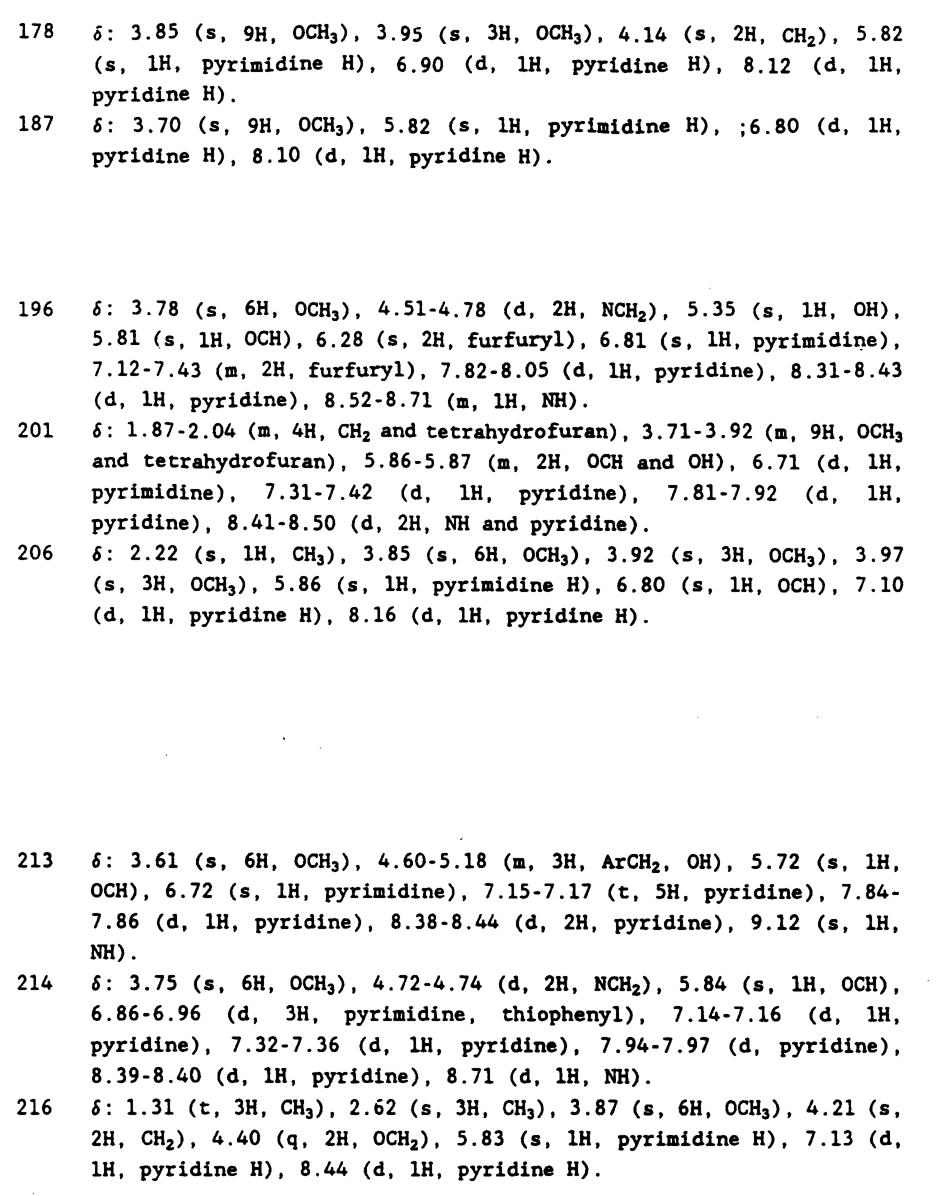

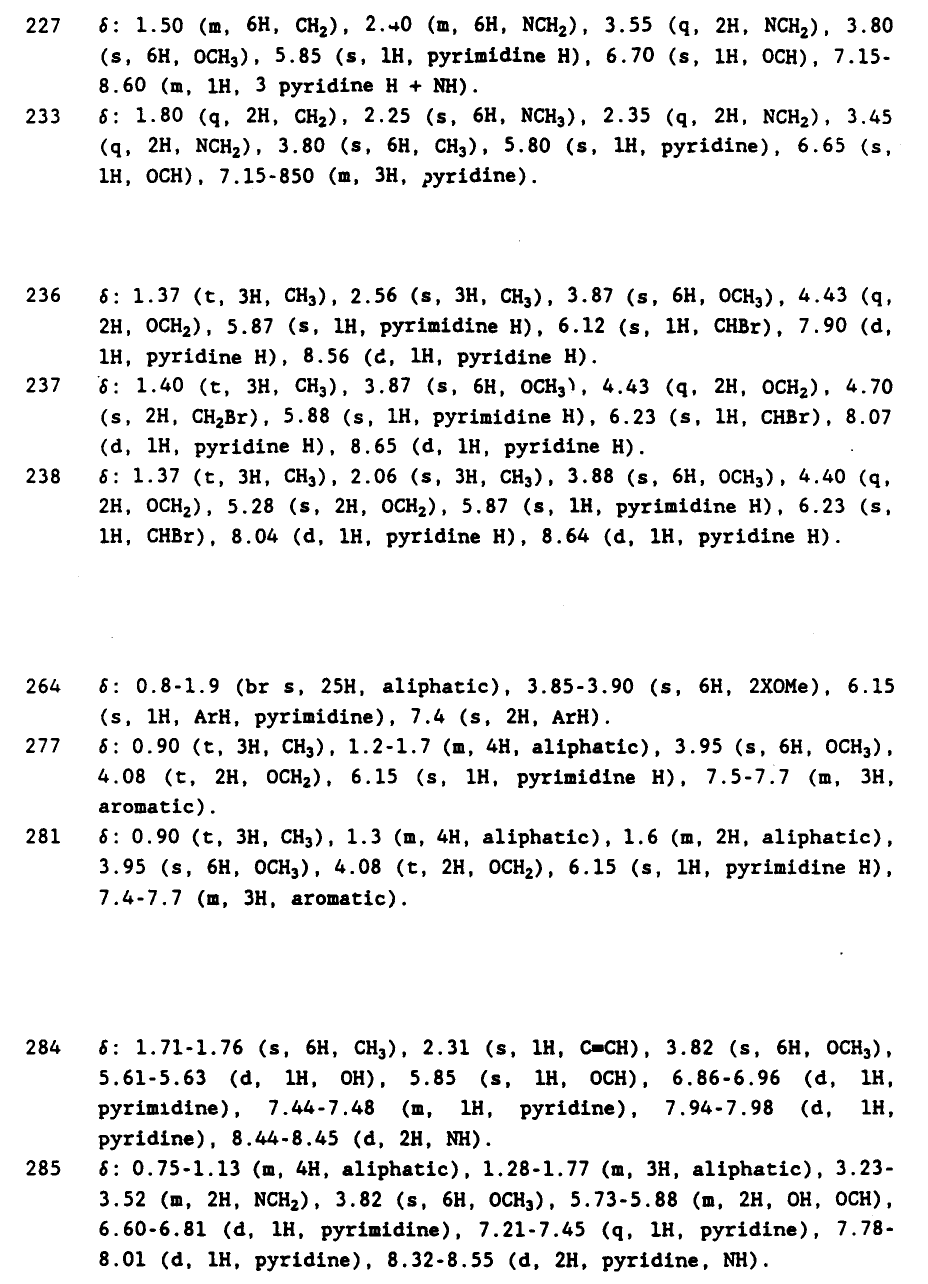

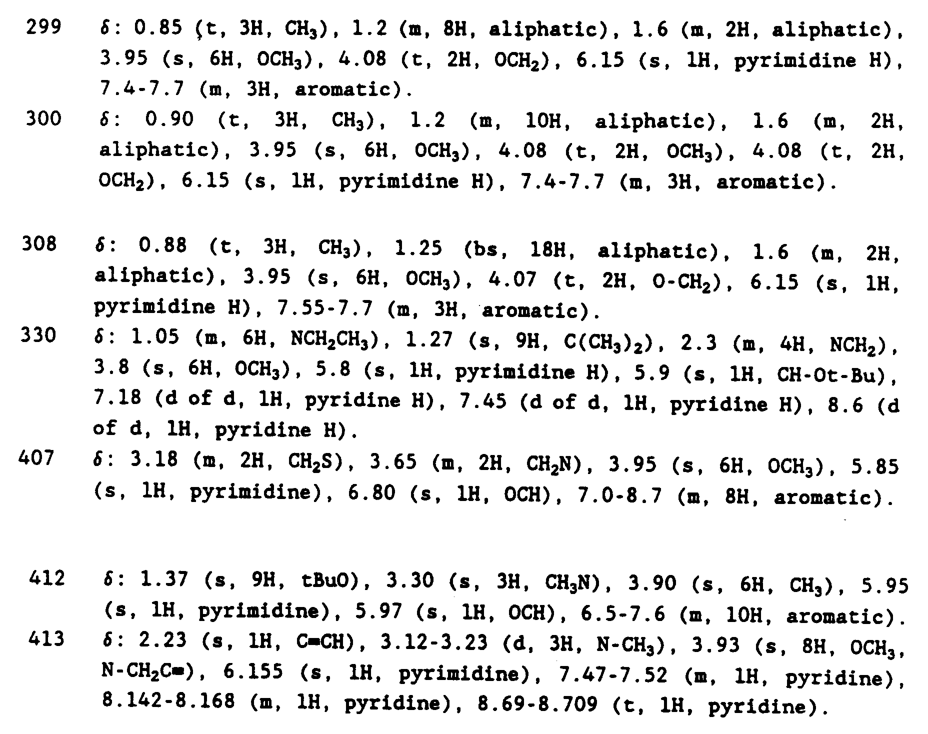

## Claims

1. A compound of formula I 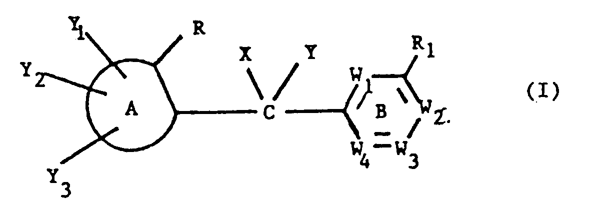 wherein
ring A is selected from
a) phenyl or naphthyl
b) pyridyl which may be fused by its (b) or (c) side to benzene
c) pyridyl-N-oxide or pyrazinyl-N-oxide
d) pyrimidinyl
e) pyrazinyl
f) 3- or 4-cinnolynyl or 2-quinoxalinyl, and
g) a five membered heteroaromatic ring comprising oxygen, sulphur or nitrogen as heteroatom which ring may be fused to a benzene ring or may comprise nitrogen as an additional heteroatom;
R is cyano, formyl, CX₁X₂X₃, -C(O)R'' wherein R'' is C₁₋₈alkyl, C₁₋C₈haloalkyl, C₁₋₈alkoxyC₁₋₈alkyl,C₂₋₈alkenyl, C₂₋₈alkynyl, aryl or arylC₁₋₈alkyl; a carboxyl group which may be in the form of the free acid or in ester or salt form, a thiocarboxyl group which may be in the form of the free acid or in ester form, a carbamoyl group, or a group -CONR₇R₈, hydroxyC₁₋₈alkyl, hydroxybenzyl, -CH=NOH, -CH=NO-C₁₋₈alkyl, or a ring C 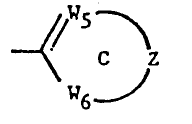
Y₁, Y₂ and Y₃ are attached to carbon atoms and are independently hydrogen, halogen, hydroxy, C₁₋₈alkyl, C₂₋₈alkenyl, C₂₋₈alkynyl, C₁₋₈alkoxy, C₂₋₈alkenyloxy, C₂₋₈alkynyloxy, C₁₋₈alkylsulfonyloxy,di(C₁₋₈alkyl)sulfamoyloxy,C₁₋₈alkylsulf onyl, C₁₋₈alkylsulfinyl, di(C₁₋₈alkyl)carbamoyloxy, C₁₋₈alkylthio, C₂₋₈alkenylthio or C₂₋₈alkynylthio each of which may in turn be substituted by 1 to 6 halogen atoms; di(C₁₋₈alkoxy)methyl, conjugated C₁₋₈alkoxy,hydroxyC₁₋₈alkyl, C₂₋₈acyl,C₂₋₈acyloxy,tri(C₁₋₈alkyl)silyloxy, tri(C₁₋₈alkyl)silyl, cyano, nitro, amino, aryl, arylC₁₋₈alkyl, aryloxy, arylC₁₋₈alkoxy, arylsulfonyl, arylsulfinyl, arylthio or arylC₁₋₈alkylthio, each of which may be substituted by one to three substituents selected from halogen, C₁₋₈alkyl, C₁₋₈haloalkyl, C₁₋₈alkoxy, C₁₋₈haloalkoxy, nitro, cyano, C₁₋₈alkylthio, C₂₋₈acyl, amino a group -C(O)-R' wherein R' is hydrogen, C₁₋₈alkyl, or C₁₋₈alkoxy; or
Y₁ and R taken together on adjacent carbon atoms form a bridge having the formula -C(S)-O-, -C(O)-O-E- or -C(O)-N(R₂)-E- wherein E is a direct bond or a 1 to 3 membered linking group with elements selected from methylene, -N(R₂)- and oxygen; or
Y₁ and Y₂ taken together on adjacent carbon atoms form a 3- to 5-membered bridge comprised of elements selected from methylene, -CH=, -C(R₄)=, -NH-, oxygen and S(O)ₙ- ;
each of W₁, W₂, W₃, W₄ and W₅ is independently CH, CR₃ or nitrogen;
W₆ is NH, oxygen, sulfur, -CR₄ =, -CH= or -C(O)- ;
Z is a 2- or 3-membered bridge comprised of elements selected from methylene, -CH=, -C(R₄)=, -C(O)-, -NH-, -N=, oxygen and -S(O)ₙ- ;
R₁ and R₃ each is independently hydrogen, halogen, C₁₋₈alkyl, C₂₋₈alkenyl, C₂₋₈alkynyl, C₁₋₈alkoxy, C₂₋₈alkenyloxy, C₂₋₈alkynyloxy, C₁₋₈alkylthio, C₂₋₈alkenylthio or C₂₋₈alkynylthio, each of which may in turn be substituted by 1 to 6 halogen atoms; C₃₋₆cycloalkyl, a 5- or 6-membered heterocycloC₁₋₈alkoxy, aryloxy, arylC₁₋₈alkoxy or arylC₁₋₈alkylthio each of which may be substituted by 1 to 3 substituents selected from halogen, C₁₋₈alkyl, C₁₋₈haloalkyl, C₁₋₈alkoxy, C₁₋₈haloalkoxy, nitro, cyano, C₁₋₈alkylthio, C₂₋₈acyl, amino; aminoxy, iminoxy, amido, C₁₋₈alkylsulfonylmethyl, cyano, nitro; or -C(O)-Y₄, wherein Y₄ is hydrogen, C₁₋₈alkyl, C₁₋₈alkoxy, hydroxy or phenyl;
R₂ is hydrogen, C₁₋₈alkyl, C₁₋₈haloalkyl, C₁₋₈alkoxyalkyl, aryl, or arylC₁₋₈alkyl;
R₄ is as defined for Y₁ except for hydrogen;
X and Y each is independently hydrogen, hydroxy, halogen, cyano, C₁₋₈alkyl, C₁₋₈alkoxy, C₁₋₈alkoxycarbonyl, C₁₋₈alkoxycarbonyloxy, hydroxyC₁₋₈alkyl, C₁₋₈haloalkyl, C₂₋₈acyl, C₂₋₈acyloxy, carbamoyl, carbamoyloxy, C₁₋₈alkylthio, C₁₋₈alkylsulfinyl, C₁₋₈alkylsulfonyl or C₁₋₈alkylsulfonyloxy; aryl, aryloxy, arylS(O)ₚ, arylsulphonyloxy, each of which may in turn be substituted by 1 to 3 substituents selected from halogen, C₁₋₈alkyl, C₁₋₈haloalkyl, C₁₋₈alkoxy, C₁₋₈haloalkoxy, nitro, cyano, C₁₋₈alkylthio, C₂₋₈acyl; amino or together represent =O, =S, =NH, =NOR₁₂ or =CR₁₃R₁₄; or
X and R together may form a bridge having the formula -C(O)-O-, -C(O)-S or -C(O)-NR₂- wherein the carbonyl is attached to A;
p is 0, 1 or 2;
X₁, X₂ and X₃ are independently hydrogen, hydroxy, C₁₋₈alkoxy, C₁₋₈alkylthio, hydroxyC₁₋₈alkyl or hydroxybenzyl whereby at least one of X₁, X₂ and X₃ is other than hydrogen; or
X₃ is hydrogen and X₁ and X₂ together form a 4- or 5-membered bridge comprising elements selected from -O(CH₂)_{n'}-O-, -OC(O)(CH₂)ₘO- and -S(CH₂)_{n'}S-;
R₇ and R₈ are each independently (a) hydrogen, halogen; (b) C₁₋₂₄ alkyl, C₂₋₈alkenyl, C₂₋₈alkynyl, C₁₋₈alkoxy, C₁₋₈alkoxyC₁₋₈alkoxy, C₂₋₈alkenyloxy, C₂₋₈alkynyloxy, C₁₋₈alkylthio, C₂₋₈alkenylthio or C₂₋₈alkynylthio, each of which may in turn be substituted by 1 to 6 halogen atoms; (c) C₃₋₆cycloalkyl, C₃₋₆cycloalkylC₁₋₈alkyl, heterocyclyl, heterocycloC₁₋₈alkyl, heterocycloC₁₋₈alkoxy, aryloxy, arylC₁₋₈alkoxy, or arylC₁₋₈alkylthio, each of which is unsubstituted or may be substituted by 1 to 3 substituents selected from (i) halogen; (ii) C₁₋₈alkyl, C₁₋₈alkoxy, C₁₋₈haloalkoxy, C₁₋₈haloalkyl, C₁₋₈alkylthio, C₁₋₈alkylsulfonyl, C₁₋₈alkylsulfonylmethyl; and (iii) nitro, cyano, acyl, amino; (d) amino, amido, aminosulfonyl, cyano, nitro, or -(CHR₄')ₙ'''-C(O)Y₄',
wherein Y₄' is hydrogen, C₁₋₈alkyl, C₁₋₈ alkoxy or hydroxy and n''' is 0, 1, 2 or 3; R₄' is as defined for Y₁;
R₁₂ is hydrogen or C₁₋₈alkyl;
R₁₃ and R₁₄ are independently hydrogen, C₁₋₈alkyl or halogen;
m is 1 or 2;
n is 0, 1 or 2; and
n' is 2 or 3;
with the proviso that when R is carboxyl in free ester or salt form and X and Y together are =O one of rings A and B contains a hetero atom.

2. A compound of formula (I) according to Claim 1 wherein A is pyridyl, quinolyl, pyridyl-N-oxide, pyrimidinyl, pyrazinyl, thienyl or furyl.

3. A compound of formula I according to claim 1 wherein the ring system A is selected from phenyl, pyridyl or pyridyl-N-oxide,
R is a carboxyl group which may be in the form of the free acid or in ester or salt form, a thiocarboxyl group which may be in the form of the free acid or in ester form, a carbamoyl group, or a group -CONR₇R₈,
Y₁, Y₂ and Y are attached to carbon atoms and are independently hydrogen, halogen, C₁₋₈alkyl, C₁₋₈alkoxy;
each of W₁, W₂, W₃ and W₄ is independently CH, CR₃ or nitrogen;
R₁ and R₃ each is independently hydrogen, halogen, C₁₋₈alkyl, C₁₋₈alkoxy, aryloxy or arylC₁₋₈alkoxy,
X and Y each is independently hydrogen, hydroxy, cyano, C₁₋₈alkoxy, C₂₋₈acyloxy or together represent =O; or
X and R together form a bridge having the formula -C(O)-O- or -C(O)NR₂- whrein the carbonyl is attached to A.

4. A compound of formula I according to claim 1 wherein ring system A represents phenyl, pyridyl or thienyl; B represents pyrimidinyl or triazinyl; R represents a ring C especially oxazole, oxazolone, oxazolidine or oxazolidinone; carboxyl in the form of the free acid or in ester or salt form; -CONR₇R₈, cyano or together with X represent -C(O)-O- or -C(O)NH₂-,
Y₁, Y₂ and Y₃ each represent independently hydrogen, halogen, C₁₋₈alkyl, C₁₋₈alkoxy, C₁₋₈alkylthio or arylthio,
X, Y each represent independently halogen, hydroxy, C₁₋₈alkoxy, C₁₋₈acyloxy, a ring B, halogen, C₁₋₈alkylthio or arylthio or together =O or =NH, and
and R₁ and R₃ each represent independently halogen, C₁₋₈alkoxy, C₁₋₈alkyl, C₁₋₈halogalkoxy, aryloxy, arylC₁₋₈alkoxy, C₂₋₈alkynyloxy, C₂₋₈alkenyloxy.

5. A compound according to claim 4, wherein Y₁, Y₂ and Y₃ additionally may each represent independently arylC₁₋₈alkoxy, C₂₋₈alkenyloxy, or C₂₋₈alkynyloxy,
B is especially pyrimidinyl, particularly 4,6-dimethoxy-2-pyrimidyl,
A is especially phenyl or pyridyl substituted as defined above,
X and Y are preferably hydrogen, halogen, cyano, hydroxy, alkoxy or together =O, especially hydrogen, hydroxy or together =O.

6. A compound according to claim 1 wherein X and Y are together =O.

7. The compound sodium 3,6-dichloro-2-[(4,6-dimethoxypyrimidin-2-yl) carbonyl]benzoate according to claim 1.

8. A herbicidal composition comprising an herbicidally effective amount of a compound of formula (I) according to Claims 1-7.

9. A method for combatting weeds which comprises applying thereto or to a locus thereof an herbicidally effective amount of a compound of formula (I) according to Claims 1-7.

10. A process for preparing a compound of formula (I) according to Claim 1 comprising
a) when X and R combine to form a bridging group as defined in claim 1 and Y is hydrogen, cyano, arylthio, arylsulfinyl or arylsulfonyl, reacting a compound of formula II 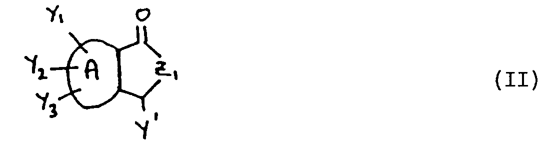 wherein ring A, Y₁, Y₂ and Y₃ are as defined in claim 1, Y' represents hydrogen, cyano, arylthio, arylsulfinyl or arylsulfonyl and Z₁ represents oxygen, sulfur or NR₂ wherein R₂ is as defined in claim 1
with a compound of formula III 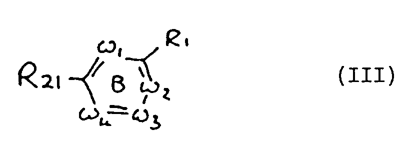 wherein W₁, W₂, W₃, W₄ and R₁ are as defined in claim 1 and R₂₁ represents methylsulfonyl or halogen to obtain the corresponding compound of formula Ip 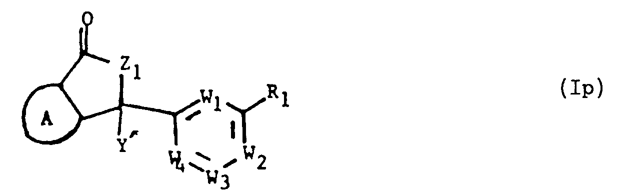
b) treating a compound of formula Ip wherein Y' represents cyano or arylsulfonyl and Z₁ represents oxygen and the other symbols are as defined in claim 1;
(i) by hydrolysis to give a corresponding compound of formula I wherein R and X form a bridge and Y is hydroxy or a compound of formula I wherein X and Y together form =O;
(ii) with an amino to give a corresponding compound of formula I wherein R is an optionally substituted carbamoyl group and X and Y together form =O;
(iii) with a group
MOR₂₂
wherein M is an alkali metal and R₂₂ is hydrogen or C₁₋₈alkyl, to give a corresponding compound wherein R and X form a bridge and Y is hydroxy or C₁₋₈alkoxy;
c) hydrolyzing a compound of formula Ip wherein Y' represents hydrogen, Z₁ represents oxygen and the other symbols are as defined in claim 1 to give a compound of formula I wherein R is a carboxyl group optionally in salt form, X is hydrogen and Y is hydroxy;
d) ring opening a compound of formula Ip wherein Y' represents hydroxy, Z₁ represents oxygen and the other symbols are as defined in claim 1 to give a compound of formula I wherein R is a carboxyl group optionally in salt form and X and Y together are =O;
e) esterifying a compound of formula I wherein R is a carboxyl group optionally in salt form and X and Y are =O and the other symbols are as defined in claim 1 to give the corresponding compound wherein R is a carboxyl group in ester form;
f) halogenating a compound of formula Ip wherein Y' represents hydroxy, Z₁ is as defined in part a) and the other symbols are as defined in claim 1 to give a compound of formula I wherein X and R together form a bridging group and Y' is halogen;
g) reacting a compound of formula Ip wherein Z₁ is oxygen, Y' is halogen and the other symbols are as defined in claim 1 with a group R₂NH₂ and a group HOR₂₃ wherein R₂₃ represents C₁₋₈alkyl, C₂₋₈acyl or aryl and R₂ is as defined in claim 1 to give the corresponding compound wherein Z₁ is NR₂ and Y' is C₁₋₈alkoxy, aryloxy or C₂₋₈acyloxy;
h) oxidizing a compound of formula Ip wherein Y' represents hydrogen, Z₁ is as defined in part a) and the other symbols are as defined in claim 1 to give the corresponding compound wherein Y' represents hydroxy;
i) reacting a compound of formula IV 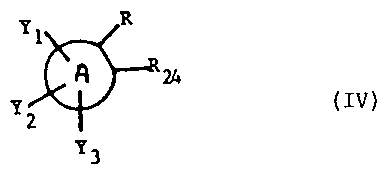 with a compound of formula V 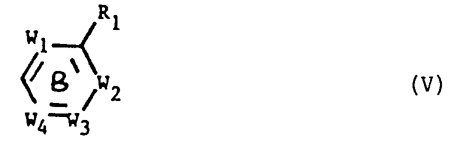 to produce a compound of formula Iq 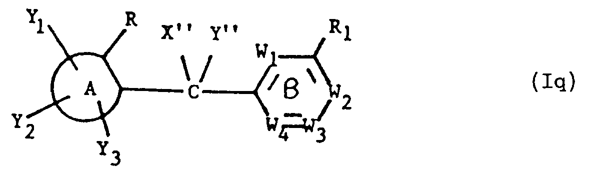 wherein ring A, R, R₁, W₁, W₂, W₃, W₄, Y₁, Y₂ and Y₃ are as defined in claim 1 and X'' and Y'' are hydrogen and R₂₄ is C₁₋₈alkyl,
j) mono- or di-halogenating a compound of formula Iq wherein X'' and Y'' are hydrogen and the other symbols are as defined in part i) to produce the corresponding compound of formula Iq wherein one or both of X'' and Y'' are halogen;
k) oxidizing a compound of formula Iq wherein X'' and Y'' are both hydrogen or X'' is halogen and Y'' is hydrogen and the other symbols are as defined in claim 1 to produce the corresponding compound wherein X'' and Y'' together represent =O or one represents hydrogen and the other represents hydroxy;
l) alkylating a compound of formula Iq wherein X'' represents hydrogen and Y'' represents hydrogen and the other symbols are as defined in claim 1 to produce the corresponding compound wherein X'' represents C₁₋₈alkyl and Y'', represents hydrogen;
m) introducing a C₁₋₈alkoxy or C₁₋₈alkylthio group into a compound of formula Iq wherein X'' represents halogen, Y'' represents hydrogen and the other symbols are as defined in claim 1 to produce the corresponding compound wherein X'' represents C₁₋₈alkoxy or C₁₋₈alkylthio and Y'' represents hydrogen;
n) acylating a compound of formula Iq wherein X'' represents hydroxy, Y represents hydrogen and the other symbols are as defined in claim 1 to produce the corresponding compound wherein X'' represents acyloxy and Y'' represents hydrogen;
o) reacting a compound of formula Ip wherein Z₁ is oxygen, Y' is hydrogen and the other symbols are as defined in claim 1 with a group R₇NH₂ wherein R₇ is (a) hydrogen, halogen; (b) C₁₋₂₄ alkyl, C₂₋₈alkenyl, C₂₋₈alkynyl, C₁₋₈alkoxy, C₁₋₈alkoxyC₁₋₈alkoxy, C₂₋₈ alkenyloxy, C₂₋₈alkynyloxy, C₁₋₈alkylthio, C₂₋₈alkenylthio or C₂₋₈alkynylthio, each of which may in turn be substituted by 1 to 6 halogen atoms; (c) C₃₋₆cycloalkyl, C₃₋₆cycloalkylC₁₋₈alkyl, heterocyclyl, heterocycloC₁₋₈alkyl, heterocycloC₁₋₈alkoxy, aryloxy, arylC₁₋₈alkoxy, or arylC₁₋₈alkylthio, each of which is unsubstituted or may be substituted by 1 to 3 substituents selected from (i) halogen; (ii) C₁₋₈alkyl, C₁₋₈alkoxy, C₁₋₈haloalkoxy, C₁₋₈haloalkyl, C₁₋₈alkylthio, C₁₋₈alkylsulfonyl, C₁₋₈alkylsulfonylmethyl; and (iii) nitro, cyano, acyl, amino; (d) amino, amido, aminosulfonyl, cyano, nitro, or -(CHR₄')ₙ'''-C(O)Y₄',
wherein Y₄' is hydrogen, C₁₋₈alkyl, C₁₋₈ alkoxy or hydroxy and n''' is 0, 1, 2 or 3, R₄' is as defined for Y₁; to give a compound of formula I wherein R is monosubstituted carbamoyl, X is hydrogen and Y is hydroxy;
p) sulfonylating, carbamoylating, acylating or carbalkoxylating a compound of formula Ip wherein Z₁ is oxygen, Y' is hydroxy and the other symbols are as defined in claim 1 to produce the corresponding compound of formula I wherein R and X form a -C(O)-O- bridge and Y represents sulfonyloxy, carbamoyloxy, C₂₋₈ acyloxy or C₁₋₈alkoxycarbonyloxy;
q) reacting a compound of formula Ip wherein Z₁ is oxygen, Y' is halogen and the other symbols are as defined in claim 1 with a group R₇R₈NH wherein R₇ is as defined in part o) and R₈ is as defined for R₇ to give a compound of formula I wherein R is disubstituted carbamoyl, and X and Z together represent =O;
and recovering any compound wherein R is a carboxyl or thiocarboxyl group in free form or in ester form and any compound wherein R is carboxyl in free form or in salt form.

## Patentansprüche

1. Verbindungen der Formel I 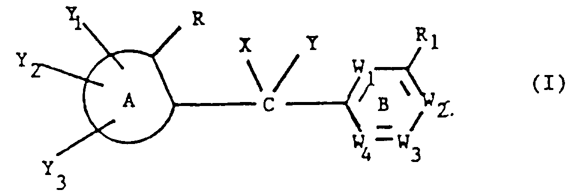 worin das Ringsystem A ausgewählt ist aus
a) Phenyl oder Naphthyl;
b) Pyridyl, das an seiner Seite (b) oder (c) mit Benzol verschmolzen sein kann,
c) Pyridyl-N-oxid oder Pyrazinyl-N-oxid,
d) Pyrimidinyl,
e) Pyrazinyl,
f) 3- oder 4-Cinnolinyl oder 2-Chinoxalinyl, oder
g) einem fünfgliedrigen heteroaromatischen Ring, der Sauerstoff Schwefel oder Stichstoff als Heteroatome enthält und der mit einem Benzolring verschmolzen sein kann oder als weiteres Heteroatom Stickstoff enthalten kann.
R steht für Cyano, Formyl, CX₁X₂X₃, -C(O)R'', worin R'' C₁-C₈-Alkyl, C₁₋₈-Halogenalkyl, C₁-C₈-AlkoxyC₁-C₈-alkyl, C₂-C₈-Alkenyl, C₂-C₈-Alkinyl, Aryl oder Aryl-C₁-C₈-alkyl ist, eine Carboxylgruppe, die in Form der freien Säure oder eines Esters oder Salzes hiervon vorliegen kann, eine Thiocarboxylgruppe, die in Form der freien Säure oder in Form eines Esters vorliegen kann, eine Carbamoylgruppe oder eine Gruppe -CONR₇R₈, Hydroxy-C₁-C₈-alkyl, Hydroxybenzyl, -CH=NOH, -CH=NO-C₁-C₈-Alkyl oder einen Ring C 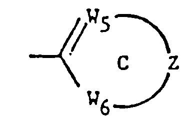
Y₁, Y₂ und Y₃ an Kohlenstoffatome gebunden sind und unabhängig voneinander stehen für Wasserstoff. Halogen, Hydroxy, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈-Alkylsulfonyloxy, Di-(C₁-C₈-alkyl)-sulfamoyloxy, C₁-C₈-Alkylsulfonyl, C₁-C₈-Alkylsulfinyl, Di-(C₁-C₈-alkyl)-carbamoyloxy, C₁-C₈-Alkylthio, C₂-C₈-Alkenylthio oder C₂-C₈-Alkinylthio, wobei jede dieser Gruppen wiederum substituiert sein kann durch 1 bis 6 Halogenatome, Di-(C₁-C₈-alkoxy)-methyl, konjugiertes C₁-C₈-Alkoxy, Hydroxy, C₁-C₈-Alkyl, C₂-C₈-Aryl, C₂-C₈-Acyloxy, Tri-(C₁-C₈-alkyl)-silyloxy, Tri-(C₁-C₈-alkyl)-silyl, Cyano, Nitro, Amino, Aryl, Aryl-C₁-C₈-alkyl, Aryloxy, ArylC₁-C₈-alkoxy, Arylsulfonyl, Arylsulfinyl, Arylthio oder Aryl-C₁-C₈-alkylthio, wobei diese Gruppen jeweils durch ein bis drei Substituenten substituiert sein können, die ausgewählt sind aus Halogen, C₁-C₈-Alkyl, C₁-C₈-Halogenalkyl, C₁-C₈-Alkoxy, C₁-C₈-Halogenalkoxy, Nitro, Cyano, C₁-C₈-Alkylthio, C₂-C₈-Acyl, Amino oder eine Gruppe -C(O)-R', worin R' Wasserstoff, C₁-C₈-Alkyl oder C₁-C₈-Alkoxy ist, oder
Y₁ und R zusammengenommen an benachbarten Kohlenstoffatomen eine Brücke der Formel -C(S)-O-, -C(O)-O-E-oder -C(O)-N(R₂)-E- bilden, worin E eine direkte Bindung oder eine ein- bis dreigliedrige Verknüpfungsgruppe ist, deren Elemente ausgewählt sind aus Methylen, -N(R₂)- und Sauerstoff, oder
Y₁ und Y₂ zusammengenommen an benachbarten Kohlenstoffatomen eine drei- bis fünfgliedrige Brücke bilden, die Elemente umfaßt, welche ausgewählt sind aus Methylen. -CH=, -C(R₄)=, -NH-, Sauerstoff und -S(O)ₙ-,
jede der Gruppen W₁, W₂, W₃, W₄ und W₅ unabhängig CH, CR₃ oder Stickstoff ist,
W₆ für NH, Sauerstoff, Schwefel, -CR₄=, -CH= oder -C(O)- steht,
Z eine zwei- oder dreigliedrige Brücke ist, welche Elemente umfaßt, die ausgewählt sind aus Methylen, -CH=, -C(R₄)=, -C(O)-, -NH-, -N=, Sauerstoff und -S(O)ₙ-,
R₁ und R₃ jeweils unabhängig stehen für Wasserstoff, Halogen, C₁-C₈-Alkyl, C₂-C₈-Alkenyl, C₂-C₈-Alkinyl, C₁-C₈-Alkoxy, C₂-C₈-Alkenyloxy, C₂-C₈-Alkinyloxy, C₁-C₈-Alkylthio, C₂-C₈-Alkenylthio oder C₂-C₈-Alkinylthio, wobei jeder dieser Reste wiederum durch 1 bis 6 Halogenatome substituiert sein kann, C₃-C₆-Cycloalkyl, fünf- oder sechsgliedriges heterocyclisches C₁-C₈-Alkoxy, Aryloxy, Aryl-C₁-C₈-alkoxy oder Aryl-C₁-C₈-alkylthio, wovon jeder dieser Reste durch ein bis drei Substituenten substituiert sein kann, die ausgewählt sind aus Halogen. C₁-C₈-Alkyl, C₁-C₈-Halogenalkyl, C₁-C₈-Alkoxy, C₁-C₈-Halogenalkoxy, Nitro, Cyano, C₁-C₈-Alkylthio, C₂-C₈-Acyl oder Amino, Aminoxy, Iminoxy, Amido, C₁-C₈-Alkylsulfonylmethyl, Cyano, Nitro oder -C(O)-Y₄, worin Y Wasserstoff, C₁-C₈-Alkyl, C₁-C₈-Alkoxy, Hydroxy oder Phenyl ist,
R₂ für Wasserstoff, C₁-C₈-Alkyl, C₁-C₈-Halogenalkyl, C₁-C₈-Alkoxyalkyl, Aryl oder Aryl-C₁-C₈-alkyl steht,
R₄ der Definition des Restes Y₁ entspricht, jedoch nicht Wasserstoffbedeuten kann,
Y und X jeweils unabhängig stehen für Wasserstoff, Hydroxy, Halogen, Cyano, C₁-C₈-Alkyl, C₁-C₈-Alkoxy, C₁-C₈-Alkoxycarbonyl, C₁-C₈-Alkoxycarbonyloxy, Hydroxy-C₁-C₈alkyl, C₁-C₈-Halogenalkyl, C₂-C₈-Acyl, C₂-C₈-Acyloxy, Carbamoyl, Carbamoyloxy, C₁-C₈-Alkylthio, C₁-C₈-Alkylsulfinyl, C₁-C₈-Alkylsulfonyl oder C₁-C₈-Alkylsulfonyloxy, Aryl, Aryloxy, Aryl-S(O)ₚ, Arylsulfonyloxy, wovon jeder dieser Reste wiederum durch ein bis drei Substituenten substituiert sein kann, die ausgewählt sind aus Halogen, C₁-C₈-Alkyl, C₁-C₈-Halogenalkyl, C₁-C₈-Alkoxy, C₁-C₈-Halogenalkoxy, Nitro, Cyano, C₁-C₈-Alkylthio oder C₂-C₈-Aryl, oder Amino oder zusammengenommen stehen für =O, =S, =NH, =NOR₁₂ oder =CR₁₃R_{14,} oder
X und R zusammen eine Brücke der Formeln -C(O)-O-, -C(O)-S- oder -C(O)-NR₂- bilden, worin das Carbonyl an das Ringsystem A gebunden ist.
p für 0, 1 oder 2 steht.
X₁, X₂ und X₃ unabhängig Wasserstoff, Hydroxy, C₁-C₈-Alkoxy, C₁-C₈-Alkylthio, Hydroxy-C₁-C₈-alkyl oder Hydroxybenzyl sind, wobei wenigstens einer der Substituenten X₁, X₂ und X₃ etwas anderes als Wasserstoffbedeutet, oder
X₃ für Wasserstoff steht und X₁ und X₂ zusammen eine viergliedrige oder fünfgliedrige Brücke aus Elementen bilden, die ausgewählt sind aus -O(CH₂)ₙ, -O-, -OC(O)(CH₂)ₘO- und -S(CH₂)_{n'}S-,
R₇ und R₈ jeweils unabhängig stehen für (a) Wasserstoff oder Halogen, (b) C₁-C₂₄-Alkyl, C₂-C₈-Alkenyl, C₂-C₈-Alkinyl, C₁-C₈-Alkoxy, C₁-C₈-Alkoxy-C₁-C₈-alkoxy, C₂-C₈-Alkenyloxy, C₂-C₈-Alkinyloxy, C₁-C₈-Alkylthio, C₂-C₈-Alkenylthio oder C₂-C₈-Alkinylthio, wobei jeder dieser Reste wiederum durch 1 bis 6 Halogenatome substituiert sein kann, (c) C₃-C₆-Cycloalkyl, C₃-C₆-Cycloalkyl-C₁-C₈-alkyl, Heterocyclyl, Heterocyclo-C₁-C₈-alkyl, Heterocyclo-C₁-C₈-alkoxy, Aryloxy, Aryl-C₁-C₈-alkoxy oder Aryl-C₁-C₈-alkylthio, wobei jeder dieser Reste unsubstituiert oder durch ein bis drei Substituenten substituiert sein kann, die ausgewählt sind aus (i) Halogen, (ii) C₁-C₈-Alkyl, C₁-C₈-Alkoxy, C₁-C₈-Halogenalkoxy, C₁-C₈-Halogenalkyl, C₁-C₈-Alkylthio, C₁-C₈-Alkylsulfonyl oder C₁-C₈-Alkylsulfonylmethyl und (iii) Nitro, Cyano, Acyl oder Amino oder (d) Amino, Amido. Aminosulfonyl, Cyano, Nitro oder -(CHR₄')_{n'''}-C(O)Y₄', worin Y₄' Wasserstoff, C₁-C₈-Alkyl, C₁-C₈-Alkoxy oder Hydroxy ist und n''' für 0, 1, 2 oder 3 steht und R₄' die Definition von Y₁ hat,
R₁₂ Wasserstoff oder C₁-C₈-Alkyl bedeutet.
R₁₃ und R₁₄ unabhängig Wasserstoff, C₁-C₈-Alkyl oder Halogen sind.
m für 1 oder 2 steht,
n für 0, 1 oder 2 steht und
n' für 2 oder 3 steht,
mit der Maßgabe, daß, falls R Carboxyl in freier Esterform oder Salzform ist und X sowie Y zusammen für =O stehen, einer der Ringe A und B dann ein Heteroatom enthält.

2. Verbindung der Formel 1 nach Ansprich 1, worin A für Pyridyl, Chinoly, Pyridyl-N-oxid, Pyrimidinyl, Pyrazinyl, Thienyl oder Furyl steht.

3. Verbindung der Formel I nach Anspruch 1, worin
das Ringsstem A ausgewählt ist aus Phenyl, Pyridyl oder Pyridyl-N-oxid,
R eine Carboxylgruppe, die in Form der freien Säure oder in Form eines Esters oder Salzes hiervon vorliegen kann, eine Thiocarboxylgruppe, die in Form der freien Säure oder in Form eines Esters hiervon vorliegen kann, eine Carbamoylgruppe oder eine Gruppe der Formel -CONR₇R₈ ist,
Y₁, Y₂ und Y₃ an Kohlenstoffatome gebunden sind und unabhängig voneinander Wasserstoff, Halogen, C₁-C₈-Alkyl oder C₁-C₈-Alkoxy bededeuten.
W₁, W₂, W₃ und W₄ jeweils unabhängig CH, CR₃ oder Stickstoff sind,
R₁ und R₃ jeweils unabhängig Wasserstoff, Halogen, C₁-C₈-Alkyl, C₁-C₈-Alkoxy, Aryloxy oder Aryl-C₁-C₈-alkoxy darstellen.
X und Y jeweils unabhängig Wasserstoff, Hydroxy, Cyano, C₁-C₈-Alkoxy oder C₂-C₈-Acyloxy sind oder zusammen für =O stehen, oder
X und R zusammen eine Brücke der Formeln -C(O)-O- oder -C(O)NR₂- bilden, worin die Carbonylgruppe an das Ringsystem A gebunden ist.

4. Verbindung der Formel I nach Anspruch 1, worin
das Ringsystem A Phenyl, Pyridyl oder Thienyl bedeutet,
der Ring B Pyrimidinyl oder Triazinyl ist,
R steht für einen Ring C, insbesondere Oxazol, Oxazolon, Oxazolidin oder Oxazolidinon, Carboxyl in Form der freien Säure oder in Form eines Esters oder Salzes hiervon, -CONR₇R₈ oder Cyano oder R zusammen mit X für -C(O)-O- oder -C(O)NR₂-,
Y₁, Y₂ und Y₃ jeweils unabhängig Wasserstoff, Halogen, C₁-C₈-Alkyl, C₁-C₈-Alkoxy, C₁-C₈-Alkylthio oder Arylthio sind.
X und Y jeweils unabhängig Halogen, Hydroxy, C₁-C₈-Alkoxy, C₁-C₈-Acyloxy, einen Ring B, Halogen, C₁-C₈-Alkylthio oder Arylthio bedeuten oder zusammen für, =O oder =NH stehen, und
R₁ und R₃ jeweils unabhängig Halogen, C₁-C₈-Alkoxy, C₁-C₈-Alkyl, C₁-C₈-Halogenalkoxy, Aryloxy, Aryl-C₁-C₈-alkoxy, C₂-C₈-Alkinyloxy oder C₂-C₈-Alkenyloxy bedeuten.

5. Verbindung nach Anspruch 4, worin
Y₁, Y₂ und Y₃ zusätzlich jeweils unabhängig Aryl-C₁-C₈-alkoxy, C₂-C₈-Alkenyloxy oder C₂-C₈-Alkinyloxy sein können,
der Ring B insbsondere Pyrimidinyl, vor allem 4,6-Dimethoxy-2-pyrimidyl ist,
der Ring A insbesondere Phenyl oder Pyridyl mit der oben definierten Substitution bedeutet, und
X und Y vorzugsweise Wasserstoff, Halogen, Cyano, Hydroxy oder Alkoxy sind oder zusammen für =O stehen, und insbesondere Wasserstoff oder Hydroxy sind oder zusammen für =O stehen.

6. Verbindung nach Anspruch 1, worin X und Y zusammen für =O stehen.

7. Verbindung nach Anspruch 1, nämlich Natrium-3,6-dichlor-2-[(4,6-dimethoxypyrimidin-2-yl)carbonyl]benzoat.

8. Herbizide Zusammensetzung aus einer herbizid wirksamen Menge einer Verbindung der Formel I nach Anspruch 1 bis 7.

9. Verfahren zur Bekämpfung von Unkräutern, dadurch gekennzeichnet, daß solche Unkräuter oder solche Unkräuter enthaltende Stellen mit einer herbizid wirksamen Menge einer Verbindung der Formel I nach Anspruch 1 bis 7 behandelt werden.

10. Verfahren zur Herstellung einer Verbindung der Formel I gemaß Definition von Anspruch 1,
gekennzeichnet durch
a) falls X und R zusammen eine Brückengruppe gemäß obiger Definition bilden und X Wasserstoff, Cyano, Arylthio, Arylsulfinyl oder Arylsulfonyl ist, Umsetzung einer Verbindung der Formel II 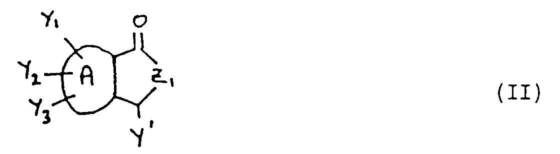 worin der Ring A, Y₁, Y₂ und Y₃ wie in Anspruch 1 definiert sind, Y' Wasserstoff, Cyano, Arylthio, Arylsulfinyl oder Arylsulfonyl ist und Z₁ Sauerstoff, Schwefel oder NR₂ bedeutet, wobei R₂ wie in Anspruch 1 definiert ist, mit einer Verbindung der Formel III 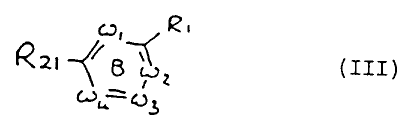 worin W₁, W₂, W₃, W₄ und R₁ wie in Anspruch 1 definiert sind und R₂₁ Methylsulfonyl oder Halogen bedeutet unter Bildung der entsprechenden Verbindung der Formel Ip 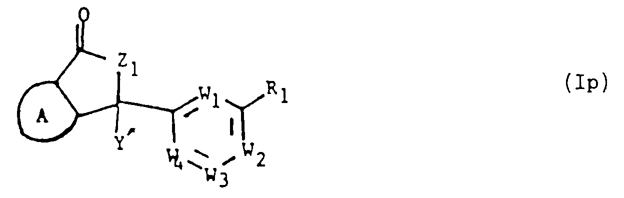
b) Behandlung einer Verbindung der Formel Ip, worin Y' Cyano oder Arylsulfonyl ist und Z₁ Sauerstoff bedeutet und die anderen Symbole wie in Anspruch 1 definiert sind, durch
(i) Hydrolyse unter Bildung einer entsprechenden Verbindung der Formel I, worin R und X eine Brücke bilden und Y Hydroxy ist oder eine Verbindung der Formel I, worin X und Y zusmmen für =O stehen,
(ii) Umsetzung mit einem Amin unter Bildung einer entsprechenden Verbindung der Formel I, worin R eine gegebenenfalls substituierte Carbamoylgruppe ist und X und Y zusammen für =O stehen, oder
(iii) Umsetzung mit einer Gruppe der Formel
MOR₂₂
worin M ein Alkalimetall ist und R₂₂ für Wasserstoff oder C₁-C₈-Alkyl steht, unter Bildung einer entsprechenden Verbindung, worin R und X eine Brücke bilden und Y Hydroxy oder C₁-C₈-Alkoxy ist,
c) Hydrolyse einer Verbindung der Formel Ip, worin Y' Hydroxy ist und Z₁ Sauerstoff bedeutet und die anderen Symbole 'wie in Anspruch 1 definiert sind, unter Bildung einer Verbindung der Formel I, worin R eine Carboxylgruppe ist, die gegebenenfalls in Salzform vorliegt, X für Wasserstoff steht und Y Hydroxy ist,
d) Ringöffnung einer Verbindung der Formel Ip, worin Y' Wasserstoff ist und Z₁ Sauerstoff bedeutet und die anderen Symbole wie in Anspruch 1 definiert sind, unter Bildung einer Verbindung der Formel I, worin R eine Carboxylgruppe ist, die gegebenenfalls in Salzform vorliegt, und X und Y zusammen für =O stehen,
e) Veresterung einer Verbindung der Formel I, worin X eine Carboxylgruppe ist, die gegebenenfalls in Salzform vorliegt, X und Y für =O stehen und die anderen Symbole wie in Anspruch 1 definiert sind, unter Bildung der entsprechenden Verbindung, worin R eine Carboxylgruppe in Esterform ist.
f) Halogenierung einer Verbindung der Formel Ip, worin Y' Hydroxy ist, Z₁ wie im Teil a) definiert ist und die anderen Symbole wie in Anspruch 1 definiert sind, unter Bildung einer Verbindung der Formel I, worin X und R zusammen eine Brücke bilden und Y' Halogen ist,
g) Umsetzung einer Verbindung der Formel Ip, worin Z₁ Sauerstoff ist, Y' Halogen bedeutet und die anderen Symbole wie in Anspruch 1 definiert sind mit einer Gruppe R₂NH₂ oder einer Gruppe HOR₂₃, worin R²³ C₁-C₈-Alkyl, C₂-C₈-Acyl oder Aryl bedeutet und R₂ wie in Anspruch 1 definiert ist, unter Bildung der entsprechenden Verbindung, worin Z₁ für NR₂ steht und Y' C₁-C₈-Alkoxy, Aryloxy oder C₂-C₈-Acyloxy ist.
h) Oxidation einer Verbindung der Formel Ip, worin Y' Wasserstoff ist, Z₁ wie in Teil a) definiert ist und die anderen Symbole wie in Anspruch 1 definiert sind, unter Bildung der entsprechenden Verbindung, worin Y' Hydroxy bedeutet,
i) Umsetzung einer Verbindung der Formel IV 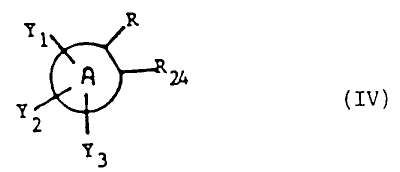 mit einer Verbindung der Formel V 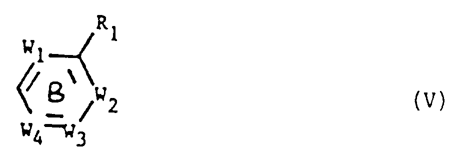 unter Bildung einer Verbindung der Formel Iq 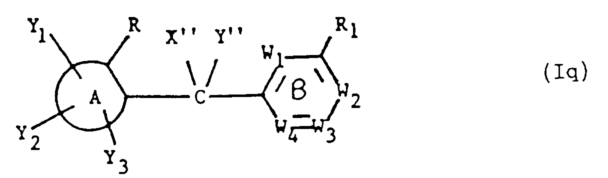 worin A, R, R₁, W₁, W₂, W₃, W₄, Y₁, Y₂ und Y₃ wie in Anspruch 1 definiert sind X'' und Y'' Wasserstoff bedeuten und R₂₄ für C₁-C₈-Alkyl steht,
j) Mono- oder Dihalogenierung einer Verbindung der Formel Iq, worin X'' und Y'' Wasserstoff sind und die anderen Symbole wie in Teil i) definiert sind, unter Bildung der entsprechenden Verbindung der Formel Iq, worin einer oder beide der Substituenten X'' und Y'' Halogen sind,
k) Oxidation einer Verbindung der Formel Iq, worin X'' und Y'' beide Wasserstoff sind oder X'' Halogen ist und Y'' Wasserstoff bedeutet, und die anderen Symbole wie in Anspruch 1 definiert sind, unter Bildung der entsprechenden Verbindung, worin X'' und Y'' zusammen für =O stehen oder einer dieser Reste Wasserstoff ist und der andere Hydroxy bedeutet,
l) Alkylierung einer Verbindung der Formel Iq, worin X'' Wasserstoff ist und Y'' Wasserstoff bedeutet, und die anderen Symbole wie in Anspruch 1 definiert sind, unter Bildung der entsprechenden Verbindung, worin X'' C₁-C₈-Alkyl ist und Y'' Wasserstoff darstellt,
m) Einführung einer C₁-C₈-Alkoxygruppe oder C₁-C₈-Alkylthiogruppe in eine Verbindung der Formel Iq, worin X'' Halogen ist und Y'' Wasserstoff bedeutet, und die anderen Symbole wie in Anspruch 1 definiert sind, unter Bildung der entsprechenden Verbindung, worin X'' C₁-C₈-Alkoxy oder C₁-C₈-Alkylthio ist und Y'' für Wasserstoff steht,
n) Acylierung einer Verbindung der Formel Iq, worin X'' Hydroxy ist und Y'' Wasserstoff darstellt, und die anderen Symbole wie in Anspruch 1 definiert sind, unter Bildung der entsprechenden Verbindung, worin X'' Acyloxy ist und Y'' Wasserstoff bedeutet,
o) Umsetzung einer Verbindung der Formel Ip, worin Z₁ Sauerstoff ist und Y' für Wasserstoff steht, und die anderen Symbole wie in Anspruch 1 definiert sind, mit einer Gruppe der Formel R₇NH₂, worin R₇ steht für (a) Wasserstoff oder Halogen, (b) C₁-C₂₄-Alkyl, C₂-C₈-Alkenyl, C₂-C₈-Alkinyl, C₁-C₈-Alkoxy, C₁-C₈-Alkoxy-C₁-C₈-alkoxy, C₂-C₈-Alkenyloxy, C₂-C₈-Alkinyloxy, C₁-C₈-Alkylthio, C₂-C₈-Alkenylthio oder C₂-C₈-Alkinylthio, wobei jeder dieser Reste wiederum durch 1 bis 6 Halogenatome substituiert sein kann, (c) C₃-C₆-Cycloalkyl, C₃-C₆-Cycloalkyl-C₁-C₈-alkyl, Heterocyclyl, Heterocyclo-C₁-C₈-alkyl, Heterocyclo-C₁-C₈-alkoxy, Aryloxy, Aryl-C₁-C₈-alkoxy oder Aryl-C₁-C₈-alkylthio, wobei jeder dieser Reste unsubstituiert oder durch ein bis drei Substituenten substituiert sein kann, die ausgewählt sind aus (i) Halogen, (ii) C₁-C₈-Alkyl, C₁-C₈-Alkoxy, C₁-C₈-Halogenalkoxy, C₁-C₈-Halogenalkyl, C₁-C₈-Alkylthio, C₁-C₈-Alkylsulfonyl oder C₁-C₈-Alkylsulfonylmethyl und (iii) Nitro, Cyano, Acyl oder Amino oder (d) Amino, Amido, Aminosulfonyl, Cyano, Nitro oder -(CHR₄')_{n'''}-C(O)Y₄', worin Y₄' Wasserstoff, C₁-C₈-Alkyl, C₁-C₈-Alkoxy oder Hydroxy ist und n''' für 0, 1, 2 oder 3 steht und R₄' die Definition von Y₁ hat unter Bildung einer Verbindung der Formel I, worin R für monosubstituiertes Carbamoyl steht, X Wasserstoff ist und Y Hydroxy darstellt,
p) Sulfonylierung, Carbamoylierung, Acylierung oder Carbalkoxylierung einer Verbindung der Formel Ip, worin Z₁ Sauerstoff ist und Y' Hydroxy bedeutet, und die anderen Symbole wie in Anspruch 1 definiert sind, unter Bildung der entsprechenden Verbindung der Formel I, worin R und X die Brücke -C(O)-O bilden und Y Sulfonyloxy, Carbamoyloxy, C₂-C₈-Acyloxy oder C₁-C₈-Alkoxycarbonyloxy sind,
q) Umsetzung einer Verbindung der Formel Ip, worin Z₁ Sauerstoff ist und Y' für Halogen steht, und die anderen Symbole wie in Anspruch 1 definiert sind, mit einer Gruppe der Formel R₇R₈NH, worin R₇ wie in Teil o) definiert ist und R₈ wie für den Rest R₇ definiert ist, unter Bildung einer Verbindung der Formel I, worin R disubstituiertes Carbamoyl bedeutet und X und Y zusammen für =O stehen,
und Gewinnung irgendeiner Verbindung, worin R eine Carboxylgruppe oder Thiocarboxylgruppe in freier Form oder in Esterform ist, und irgendeiner Verbindung, worin R Carboxyl in freier Form oder in Salzform darstellt.

## Revendications

1. Un composé de formule I 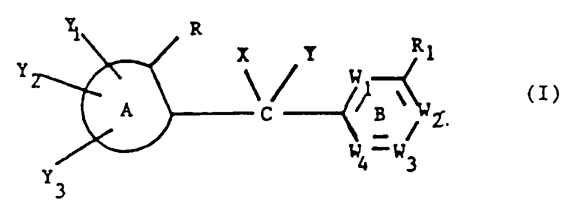 dans laquelle
le cycle A est choisi parmi les restes
a) phényle ou naphtyle,
b) pyridyle qui peut être condensé par son côté (b) ou (c) au benzène,
c) pyridyl-N-oxyde ou pyrazinyl-N-oxyde,
d) pyrimidinyle,
e) pyrazinyle,
f) 3- ou 4-cinnolynyle ou 2-quinoxalinyle, et
g) hétérocyclique à 5 chaînons comprenant de l'oxygène, du soufre ou de l'azote comme hétéroatome, ledit reste pouvant être condensé à un cycle benzénique ou pouvant comprendre de l'azote comme hétéroatome supplémentaire;
R signifie un groupe cyano, formyle, CX₁X₂X₃, -C(O)R'' où R'' signifie un groupe alkyle en C₁-C₈, haloalkyle en C₁-C₈, (alcoxy en C₁-C₈)alkyle en C₁-C₈, alcényle en C₂-C₈, alcynyle en C₂-C₈, aryle ou aryl-(alkyle en C₁-C₈); un groupe carboxy qui peut se présenter sous forme d'acide libre ou sous la forme d'un ester ou d'un sel, un groupe thiocarboxy qui peut se présenter sous forme d'acide libre ou sous la forme d'un ester, un groupe carbamoyle, ou un groupe -CONR₇R₈, hydroxyalkyle en C₁-C₈, hydroxybenzyle, -CH=NOH, -CH=NO(alkyle en C₁-C₈) ou un cycle C 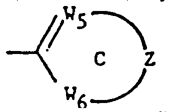
Y₁, Y₂ et Y₃ sont fixés aux atomes de carbone et signifient indépendamment l'hydrogène, un halogène ou un groupe hydroxy, alkyle en C₁-C₈, alcényle en C₂-C₈, alcynyle en C₂-C₈, alcoxy en C₁-C₈, alcényloxy en C₂-C₈, alcynyloxy en C₂-C₈, (alkyl en C₁-C₈)sulfonyloxy, di(alkyl en C₁-C₈)sulfamoyloxy, alkylsulfonyle en C₁-C₈, alkylsulfinyle en C₁-C₈, di(alkyl en C₁-C₈)-carbamoyloxy, alkylthio en C₁-C₈, alcénylthio en C₂-C₈ ou alcynylthio en C₂-C₈, chacun d'entre eux pouvant être substitué à son tour par 1 à 6 atomes d'halogène;
un groupe di(alcoxy en C₁-C₈)-méthyle, un groupe alcoxy en C₁-C₈ conjugué, hydroxyalkyle en C₁-C₈,
acyle en C₂-C₈, acyloxy en C₂-C₈, tri(alkyl en C₁-C₈)-silyloxy, tri(alkyl en C₁-C₈)silyle, cyano, nitro, amino, aryle, aryl-(alkyle en C₁-C₈), aryloxy, aryl-(alcoxy en C₁-C₈), arylsulfonyle, arylsulfinyle, arylthio ou aryl-(alkylthio en C₁-C₈), chacun d'entre eux pouvant être substitué par 1 à 3 substituants choisis parmi un halogène ou un groupe alkyle en C₁-C₈, haloalkyle en C₁-C₈, alcoxy en C₁-C₈, haloalcoxy en C₁-C₈, nitro, cyano, alkylthio en C₁-C₈, acyle en C₂-C₈, amino, un groupe -C(O)-R' où R' signifie l'hydrogène ou un groupe alkyle en C₁-C₈ ou alcoxy en C₁-C₈; ou bien
Y₁ et R pris ensemble sur les atomes de carbone adjacents forment un pont ayant la formule -C(S)-O-, -C(O)-O-E- ou -C(O)-N(R₂)-E- où E signifie une liaison directe ou un groupe de liaison ayant 1 à 3 chaînons avec des éléments choisis parmi le méthylène, -N(R₂)- et l'oxygène; ou bien
Y₁ et Y₂ pris ensemble sur les atomes de carbone adjacents forment un pont de 3 à 5 chaînons comprenant les éléments choisis parmi le méthylène, -CH=, -C(R₄)=, -NH-, l'oxygène et -S(O)ₙ-;
chaque symbole W₁, W₂, W₃, W₄ et W₅ signifie indépendamment CH, CR₃ ou l'azote;
W₆ signifie NH, l'oxygène, le soufre, -CR₄=, -CH= ou -C(O)-;
Z signifie un pont à 2 ou 3 chaînons comprenant les éléments choisis parmi le méthylène, CH=, C(R₄)=, -C(O)-, -NH-, -N=, l'oxygène et -S(O)ₙ-;
R₁ et R₃ signifient chacun indépendamment l'hydrogène, un halogène ou un groupe alkyle en C₁-C₈, alcényle en C₂-C₈, alcynyle en C₂-C₈, alcoxy en C₁-C₈, alcényloxy en C₂-C₈, alcynyloxy en C₂-C₈, alkylthio en C₁-C₈, alcénylthio en C₂-C₈ ou alcynylthio en C₂-C₈, chacun d'entre eux pouvant à son tour être substitué par 1 à 6 atomes d'halogène; cycloalkyle en C₃-C₆, hétérocyclo(alcoxy en C₁-C₈) à 5 ou 6 chaînons, aryloxy, aryl-(alcoxy en C₁-C₈)ou aryl-(alkylthio C₁-C₈), chacun d'entre eux pouvant être substitué par 1 à 3 substituants choisis parmi un halogène ou un groupe alkyle en C₁-C₈, haloalkyle en C₁-C₈, alcoxy en C₁-C₈, haloalcoxy en C₁-C₈, nitro, cyano, alkylthio en C₁-C₈, acyle en C₂-C₈, amino; aminoxy, iminoxy, amido, (alkyl en C₁-C₈)-sulfonylméthyle, cyano, nitro; ou bien -C(O)-Y₄, où Y₄ signifie l'hydrogène ou un groupe alkyle en C₁-C₈, alcoxy en C₁-C₈, hydroxy ou phényle;
R₂ signifie l'hydrogène ou un groupe alkyle en C₁-C₈, haloalkyle en C₁-C₈, alcoxyalkyle en C₁-C₈, aryle ou aryl-(alkyle en C₁-C₈);
R₄ a l'une des significations définies pour Y₁, sauf l'hydrogène;
X et Y signifient chacun indépendamment l'hydrogène ou un groupe hydroxy, un halogène, un groupe cyano, alkyle en C₁-C₈, alcoxy en C₁-C₈, (alcoxy en C₁-C₈)carbonyle, (alcoxy en C₁-C₈)-carbonyloxy, hydroxyalkyle en C₁-C₈, haloalkyle en C₁-C₈, acyle en C₂-C₈, alcyloxy en C₂-C₈, carbamoyle, carbamoyloxy, alkylthio en C₁-C₈, alkylsulfinyle en C₁-C₈, alkylsulfonyle en C₁-C₈ ou (alkyl en C₁-C₈)-sulfonyloxy; aryle, aryloxy, arylS(O)ₚ, arylsulfonyloxy, chacun d'entre eux pouvant à son tour être substitué par 1 à 3 substituants choisis parmi un halogène ou un groupe alkyle en C₁-C₈, haloalkyle en C₁-C₈, alcoxy en C₁-C₈, haloalcoxy en C₁-C₈, nitro, cyano, alkylthio en C₁-C₈, acyle en C₂-C₈; amino ou signifient ensemble =O, =S, =NH, =NOR₁₂ ou =CR₁₃R₁₄; ou bien
X et R peuvent former ensemble un pont ayant la formule -C(O)-O-, -C(O)-S ou -C(O)-NR₂- où le groupe carbonyle est fixé à A;
p signifie 0, 1 ou 2;
X₁, X₂ et X₃ signifient indépendamment l'hydrogène ou un groupe hydroxy, alcoxy en C₁-C₈, alkylthio en C₁-C₈, hydroxyalkyle en C₁-C₈, ou hydroxybenzyle, au moins l'un des symboles X₁, X₂ et X₃ devant avoir une signification autre que l'hydrogène; ou bien
X₃ signifie l'hydrogène et X₁ et X₂ forment ensemble un pont à 4 ou 5 chaînons comprenant des éléments choisis parmi -O(CH₂)ₙ, -O-, -OC(O)(CH₂)ₘO- et -S(CH₂)ₙ, S-;
R₇ et R₈ signifient chacun indépendamment (a) l'hydrogène ou un halogène; (b) un groupe alkyle en C₁-C₂₄, alcényle en C₂-C₈, alcynyle en C₂-C₈, alcoxy en C₁-C₈, (alcoxy en C₁-C₈)-alcoxy en C₁-C₈, alcényloxy en C₂-C₈, alcynyloxy en C₂-C₈, alkylthio en C₁-C₈, alcénylthio en C₂-C₈ ou alcynylthio en C₂-C₈, chacun d'entre eux pouvant à son tour être substitué par 1 à 6 atomes d'halogène; (c) cycloalkyle en C₃-C₆, (cycloalkyl en C₃-C₆)alkyle en C₁-C₈, hétérocyclique, hétérocyclo(alkyle en C₁-C₈), hétérocyclo(alcoxy en C₁-C₈), aryloxy, aryl-(alcoxy en C₁-C₈) ou aryl-(alkylthio en C₁-C₈), chacun d'entre eux étant non substitué ou pouvant être substitué par 1 à 3 substituants choisis parmi (i) un halogène; (ii) un groupe alkyle en C₁-C₈, alcoxy en C₁-C₈, haloalcoxy en C₁-C₈, haloalkyle en C₁-C₈, alkylthio en C₁-C₈, alkylsulfonyle en C₁-C₈, (alkyl en C₁-C₈)-sulfonylméthyle; et (iii) nitro, cyano, acyle, amino; (d) amino, amido, aminosulfonyle, cyano, nitro ou -(CHR₄')n'''-C(O)Y₄', où Y₄' signifie l'hydrogène ou un groupe alkyle en C₁-C₈, alcoxy en C₁-C₈ ou hydroxy et n''' signifie 0, 1, 2 ou 3; R₄' est tel que défini pour Y₁;
R₁₂ signifie l'hydrogène ou un groupe alkyle en C₁-C₈,
R₁₃ et R₁₄ signifient indépendamment l'hydrogène ou un groupe alkyle en C₁-C₈ ou un halogène;
m signifie 1 ou 2;
n signifie 0, 1 ou 2; et
n' signifie 2 ou 3;
lorsque R signifie un groupe carboxy sous forme d'un ester libre ou d'un sel et X et Y signifient ensemble =O, l'un des cycles A et B devant contenir un hétéroatome.

2. Un composé de formule (I) selon la revendication 1, dans lequel A signifie un reste pyridyle, quinolyle, pyridyl-N-oxyde, pyrimidinyle, pyrazinyle, thiényle ou furyle.

3. Un composé de formule I selon la revendication 1, dans lequel le système cyclique A est choisi parmi un reste phényle, pyridyle ou pyridyl-N-oxyde,
R signifie un groupe carboxy qui peut être sous forme d'un acide libre ou sous la forme d'un ester ou d'un sel, un groupe thiocarboxy qui peut être sous forme d'un acide libre ou sous la forme d'un ester, un groupe carbamoyle ou un groupe -CONR₇R₈,
Y₁, Y₂ et Y sont fixés à des atomes de carbone et signifient indépendamment l'hydrogène, un halogène ou un groupe alkyle en C₁-C₈, alcoxy en C₁-C₈; chacun des symboles W₁, W₂, W₃ et W₄ signifie indépendamment CH, CR₃ ou l'azote,
R₁ et R₃ signifient chacun indépendamment l'hydrogène, un halogène ou un groupe alkyle en C₁-C₈, alcoxy en C₁-C₈, aryloxy ou aryl-(alcoxy en C₁-C₈),
X et Y signifient chacun indépendamment l'hydrogène ou un groupe hydroxy, cyano, alcoxy en C₁-C₈, acyloxy en C₂-C₈ ou signifient ensemble =O; ou bien
X et R forment ensemble un pont ayant la formule -C(O)-O- ou -C(O)NR₂-, où le groupe carbonyle est fixé à A.

4. Un composé de formule I selon la revendication 1, dans lequel le système cyclique A signifie un reste phényle, pyridyle ou thiényle; B signifie un reste pyrimidinyle ou triazinyle; R signifie un cycle C, en particulier un cycle oxazole, oxazolone, oxazolidine ou oxazolidinone; un groupe carboxy sous forme d'un acide libre ou sous la forme d'un ester ou d'un sel; -CONR₇R₈, cyano ou signifient ensemble avec X -C(O)-O- ou -C(O)NR₂-,
Y₁, Y₂ et Y₃ signifient chacun indépendamment l'hydrogène, un halogène ou un groupe alkyle en C₁-C₈, alcoxy en C₁-C₈, alkylthio en C₁-C₈, ou arylthio,
X, Y signifient chacun indépendamment un halogène ou un groupe hydroxy, alcoxy en C₁-C₈, acyloxy en C₁-C₈, un cycle B, un halogène, un groupe alkylthio en C₁-C₈ ou arylthio ou signifient ensemble =O ou =NH, et
R₁ et R₃ signifient chacun indépendamment un halogène ou un groupe alcoxy en C₁-C₈, alkyle en C₁-C₈, haloalcoxy en C₁-C₈, aryloxy, aryl-(alcoxy en C₁-C₈), alcynyloxy en C₂-C₈, alcényloxy en C₂-C₈.

5. Un composé selon la revendication 4, dans lequel Y₁, Y₂ et Y₃ peuvent chacun également signifier indépendamment un groupe aryl-(alcoxy en C₁-C₈), alcényloxy en C₂-C₈ ou alcynyloxy en C₂-C₈,
B signifie spécialement un reste pyrimidinyle, en particulier un groupe 4,6-diméthoxy-2-pyrimidinyle,
A signifie spécialement phényle ou pyridyle substitués comme défini plus haut,
X et Y signifient de préférence l'hydrogène, un halogène, un groupe cyano, hydroxy, alcoxy ou signifient ensemble =O, spécialement l'hydrogène ou un groupe hydroxy ou signifient ensemble =O.

6. Un composé selon la revendication 1, dans lequel X et Y signifient ensemble =O.

7. Le composé selon la revendication 1 qui est le 3,6-dichloro-2-[(4,6-diméthoxypyrimidin-2-yl)carbonyl]benzoate de sodium.

8. Une composition herbicide comprenant une quantité efficace du point de vue herbicide d'un composé de formule (I) selon les revendications 1 à 7.

9. Une méthode de lutte contre les mauvaises herbes qui comprend l'application sur les mauvaises herbes ou sur leur zone de croissance d'une quantité efficace du point de vue herbicide d'un composé de formule (I) selon les revendications 1 à 7.

10. Un procédé de préparation d'un composé de formule (I) selon la revendication 1, procédé selon lequel
a) lorsque X et R forment ensemble un groupe formant pont tel que défini à la revendication 1 et Y signifie l'hydrogène ou un groupe cyano, arylthio, arylsulfinyle ou arylsulfonyle, on fait réagir un composé de formule II 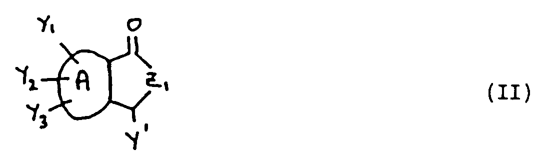 où le cycle A, Y₁, Y₂ et Y₃ sont tels que définis à la revendication 1, Y' signifie l'hydrogène ou un groupe cyano, arylthio, arylsulfinyle ou arylsulfonyle et Z₁ signifie l'oxygène, le soufre ou NR₂ où R₂ est tel que défini à la revendication 1,
avec un composé de formule III 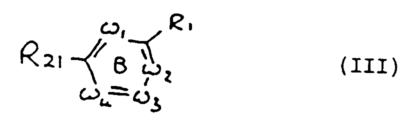 où W₁, W₂, W₃, W₄ et R₁ sont tels que définis à la revendication 1 et R₂₁ signifie un groupe méthylsulfonyle ou un halogène, pour obtenir le composé correspondant de formule Ip 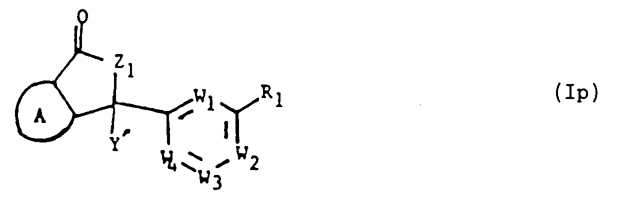
b) on traite un composé de formule Ip où Y' signifie un groupe cyano ou arylsulfonyle et Z₁ signifie l'oxygène et les autres symboles sont tels que définis à la revendication 1;
(i) par hydrolyse, ce qui donne un composé correspondant de formule I où R et X forment un pont et Y signifie un groupe hydroxy ou un composé de formule I où X et Y forment ensemble =O;
(ii) avec un groupe amino, ce qui donne un composé correspondant de formule I où R signifie un groupe carbamoyle éventuellement substitué et X et Y forment ensemble =O;
(iii) avec un groupe
MOR₂₂
où M signifie un métal alcalin et R₂₂ signifie l'hydrogène ou un groupe alkyle en C₁-C₈, ce qui donne un composé correspondant où R et X forment un pont et Y signifie un groupe hydroxy ou alcoxy en C₁-C₈;
c) on hydrolyse un composé de formule Ip où Y' signifie l'hydrogène, Z₁ signifie l'oxygène et les autres symboles sont tels que définis à la revendication 1, ce qui donne un composé de formule I où R signifie un groupe carboxy éventuellement sous forme d'un sel, X signifie l'hydrogène et Y signifie un groupe hydroxy;
d) on décyclise un composé de formule Ip où Y' signifie un groupe hydroxy, Z₁ signifie l'oxygène et les autres symboles sont tels que définis à la revendication 1, ce qui donne un composé de formule I où R signifie un groupe carboxy éventuellement sous forme d'un sel et X et Y signifient ensemble =O;
e) on estérifie un composé de formule I où R signifie un groupe carboxy éventuellement sous forme d'un sel et X et Y signifient =O et les autres symboles sont tels que définis à la revendication 1, ce qui donne le composé correspondant où R signifie un groupe carboxy sous la forme d'un ester;
f) on halogène un composé de formule Ip où Y' signifie un groupe hydroxy, Z₁ est tel que défini sous a) et les autres symboles sont tels que définis à la revendication 1, ce qui donne un composé de formule I où X et R forment ensemble un groupe formant pont et Y' signifie un halogène;
g) on fait réagir un composé de formule Ip où Z₁ signifie l'oxygène, Y' signifie un halogène et les autres symboles sont tels que définis à la revendication 1, avec un composé R₂NH₂ et un composé HOR₂₃ où R₂₃ signifie un groupe alkyle en C₁-C₈, acyle en C₂-C₈ ou aryle et R₂ est tel que défini à la revendication 1, ce qui donne le composé correspondant où Z₁ signifie NR₂ et Y' signifie un groupe alcoxy en C₁-C₈, aryloxy ou acyloxy en C₂-C₈;
h) on oxyde un composé de formule Ip où Y' signifie l'hydrogène, Z₁ est tel que défini sous a) et les autres symboles sont tels que définis à la revendication 1, ce qui donne le composé correspondant où Y' signifie un groupe hydroxy;
i) on fait réagir un composé de formule IV 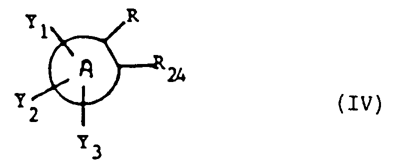 avec un composé de formule V 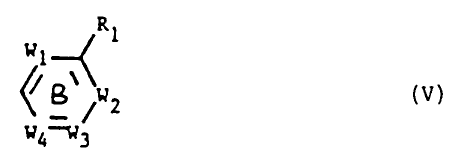 pour produire un composé de formule Iq 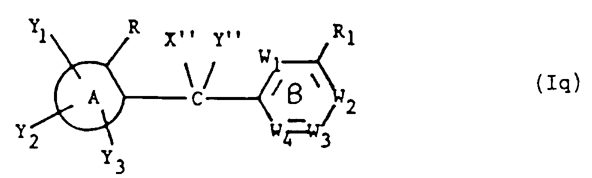 où le cycle A, R, R₁, W₁, W₂, W₃, W₄, Y₁, Y₂ et Y₃ sont tels que définis à la revendication 1 et X'' et Y'' signifient l'hydrogène et R₂₄ signifie un groupe alkyle en C₁-C₈;
j) on introduit un ou deux halogènes dans un composé de formule Iq où X'' et Y'' signifient l'hydrogène et les autres symboles sont tels que définis sous i), pour produire le composé correspondant de formule Iq où l'un ou les deux symboles X'' et Y'' signifient un halogène;
k) on oxyde un composé de formule Iq où X'' et Y'' signifient tous les deux l'hydrogène ou bien X'' signifie un halogène et Y'' signifie l'hydrogène et les autres symboles sont tels que définis à la revendication 1, pour produire le composé correspondant où X'' et Y'' signifient ensemble =O ou l'un des deux signifie l'hydrogène et l'autre signifie un groupe hydroxy;
l) on alkyle un composé de formule Iq où X'' signifie l' hydrogène et Y'' signifie l'hydrogène et les autres symboles sont tels que définis à la revendication 1, pour produire le composé correspondant où X'' signifie un groupe alkyle en C₁-C₈, et Y'' signifie l'hydrogène;
m) on introduit un groupe alcoxy en C₁-C₈ ou un groupe alkylthio en C₁-C₈, dans un composé de formule Iq où X'' signifie un halogène, Y'' signifie l'hydrogène et les autres symboles sont tels que définis à la revendication 1, pour produire le composé correspondant où X'' signifie un groupe alcoxy en C₁-C₈, ou alkylthio en C₁-C₈ et Y'' signifie l'hydrogène;
n) on acyle un composé de formule Iq où X'' signifie un groupe hydroxy, Y signifie l'hydrogène et les autres symboles sont tels que définis à la revendication 1, pour produire le composé correspondant où X'' signifie un groupe acyloxy et Y'' signifie l'hydrogène;
o) on fait réagir un composé de formule Ip où Z₁ signifie l'oxygène, Y' signifie l'hydrogène et les autres symboles sont tels que définis à la revendication 1 avec un composé R₇NH₂ où R₇ signifie (a) l'hydrogène, un halogène; (b) un groupe alkyle en C₁-C₂₄, alcényle en C₂-C₈, alcynyle en C₂-C₈, alcoxy en C₁-C₈, (alcoxy en C₁-C₈)-alcoxy en C₁-C₈, alcényloxy en C₂-C₈, alcynyloxy en C₂-C₈, alkylthio en C₁-C₈, alcénylthio en C₂-C₈ ou alcynylthio en C₂-C₈, chacun d'entre eux pouvant à son tour être substitué par 1 à 6 atomes d'halogène; (c) un groupe cycloalkyle en C₃-C₆, (cycloalkyl en C₃-C₆)alkyle en C₁-C₈, hétérocyclique, hétérocyclo(alkyle en C₁-C₈), hétérocyclo(alcoxy en C₁-C₈), aryloxy, aryl-(alcoxy en C₁-C₈), ou aryl-(alkylthio en C₁-C₈), chacun d'entre eux pouvant être non substitué ou être substitué par 1 à 3 substituants choisis parmi (i) un halogène; (ii) un groupe alkyle en C₁-C₈, alcoxy en C₁-C₈, haloalcoxy en C₁-C₈, haloalkyle en C₁-C₈, alkylthio en C₁-C₈, alkylsulfonyle en C₁-C₈, (alkyl en C₁-C₈)-sulfonylméthyle; et (iii) un groupe nitro, cyano, acyle amino; (d) un groupe amino, amido, aminosulfonyle, cyano, nitro ou -(CHR₄')ₙ'''-C(O)Y₄',
où Y₄' signifie l'hydrogène ou un groupe alkyle en C₁-C₈, alcoxy en C₁-C₈, ou hydroxy et n''' signifie 0, 1, 2 ou 3, R₄' est tel que défini pour Y₁; ce qui donne un composé de formule I où R signifie un groupe carbamoyle monosubstitué, X signifie l'hydrogène et Y signifie un groupe hydroxy;
p) on introduit un groupe sulfonyle, carboxy, acyle ou carbalcoxy dans un composé de formule Ip où Z₁ signifie l'oxygène, Y' signifie un groupe hydroxy et les autres symboles sont tels que définis à la revendication 1, pour produire le composé correspondant de formule I où R et X forment un pont -C(O)-O- et Y signifie un groupe sulfonyloxy, carbamoyloxy, acyloxy en C₂-C₈ ou (alcoxy en C₁-C₈)-carbonyloxy;
q) on fait réagir un composé de formule Ip où Z₁ signifie l'oxygène, Y' signifie un halogène et les autres symboles sont tels que définis à la revendication 1 avec un composé R₇R₈NH où R₇ est tel que défini sous o) et R₈ est tel que défini pour R₇, ce qui donne un composé de formule I où R signifie un groupe carbamoyle disubstitué et X et Z signifient ensemble =O;
et on récupère un composé où R signifie un groupe carboxy ou thiocarboxy sous forme libre ou sous forme d'un ester et un composé où R signifie un groupe carboxy sous forme libre ou sous forme d'un sel.
